# EUROPEAN PATENT APPLICATION

(11) **EP 4 781 980 A1**
(43) Date of publication of application: **29.07.2026**
(21) Application number: 24868291.6
(22) Date of filing: 18.09.2024
(51) Int. Cl.: A61K 9/51, A61K 9/10, A61K 31/7088, A61K 47/18, A61K 47/24, A61K 47/28, A61K 47/34, A61K 48/00

(54) **METHOD FOR PRODUCING LIGAND-MODIFIED LIPID NANOPARTICLES ENCAPSULATING NUCLEIC ACID**

(30) Priority: 22.09.2023 JP 2023159114
(71) Applicant: Tohoku University, Sendai-shi, Miyagi 980-8577 (JP); NOF Corporation, Shibuya-ku Tokyo 150-6019 (JP)
(72) Inventor: AKITA, Hidetaka, Sendai-shi, Miyagi 980-8577 (JP); TANAKA, Hiroki, Sendai-shi, Miyagi 980-8577 (JP); SAKURAI, Yu, Sendai-shi, Miyagi 980-8577 (JP); WATANABE, Himeka, Sendai-shi, Miyagi 980-8577 (JP); RI, Yoshie, Sendai-shi, Miyagi 980-8577 (JP); SUGAWARA, Takumi, Sendai-shi, Miyagi 980-8577 (JP); TANAKA, Akari, Sendai-shi, Miyagi 980-8577 (JP); OSHIMA, Shion, Sendai-shi, Miyagi 980-8577 (JP); ITO, Kasumi, Kawasaki-shi, Kanagawa 210-0865 (JP); NAKAI, Yuta, Kawasaki-shi, Kanagawa 210-0865 (JP); TANGE, Kota, Kawasaki-shi, Kanagawa 210-0865 (JP)
(74) Representative: Vossius & Partner Patentanwälte Rechtsanwälte mbB
(86) International application number: PCT/JP2024/033349
(87) International publication number: WO 2025/063214

(57) **Abstract**

The present invention provides a method for producing a ligand-modified lipid nanoparticle encapsulating a nucleic acid, including the following Steps a) to c) and the like
Step a) of mixing an alcohol solution comprising an ionic lipid, a sterol, and a PEG lipid with an acidic buffer having a pH of 1 to 6.5 to obtain a suspension of a lipid nanoparticle free of nucleic acids,
Step b) of mixing the lipid nanoparticle free of nucleic acids and a nucleic acid solution to obtain a suspension of a lipid nanoparticle encapsulating the nucleic acid, and
Step c) of mixing the suspension of the lipid nanoparticle encapsulating the nucleic acid and a ligand-bound lipid to obtain a suspension of a ligand-modified lipid nanoparticle encapsulating the nucleic acid.

## Description

### [Technical Field]

The present invention relates to a method for producing ligand-modified lipid nanoparticles encapsulating nucleic acids

### [Background Art]

For practicalization of nucleic acid therapy using oligonucleic acids such as siRNA, and gene therapy using mRNA, pDNA, and the like, an effective and safe nucleic acid delivery carrier is demanded. While vectors are nucleic acid delivery carriers with good expression efficiency, the development of non-viral nucleic acid delivery carriers that can be used more safely is ongoing. Among them, lipid nanoparticles that are carriers using ionic lipids are non-viral nucleic acid delivery carriers most generally used at present.

Ionic lipids are largely constituted of amine moiety and lipid moiety. The amine moiety, which is protonated under acidic conditions, interacts electrostatically with nucleic acids, which are polyanions, to form lipid nanoparticles, which promotes uptake into cells and delivers nucleic acids into cells.

A known ionic lipid that is generally widely used is, for example, 1,2-dioleoyl-3-dimethylammonium propane (DODAP). It is known that by combining known ionic lipids with phospholipids, cholesterol, and PEG lipids, lipid nanoparticles can be formed and nucleic acids can be delivered into cells (see, for example, Non Patent Literature 1).

Patent Literature 1 describes an ionic lipid having a structure in which compounds consisting of one or two amine moieties and one lipid moiety are connected by a biodegradable disulfide bond. This literature states that the ionic lipid can improve pharmacokinetics such as blood stability and tumor targeting, and that by changing the structure around the amine moiety, the pKa of a lipid membrane structure can be adjusted to a value advantageous for endosomal escape in cells, and further that it has the effect of dissociating nucleic acids from lipid membrane structures by utilizing the cleavage of disulfide bonds within cells. In fact, since it shows higher nucleic acid delivery efficiency compared to a known ionic lipid DODAP, it is clear that this ionic lipid can achieve improvement of the intracellular dynamics such as improvement of the delivery efficiency of nucleic acids into the cytoplasm and the like.

Furthermore, in Patent Literature 2, a lipid membrane structure is shown that has enhanced ability to fuse with endosomal membrane and has further improved efficiency of nucleic acid introduction into the cytoplasm, by using an ionic lipid having, in addition to a tertiary amine moiety and disulfide bond, an aromatic ring introduced near the lipid moiety.

As described above, ionic lipids with improved intracellular dynamics have been developed by increasing endosomal escape efficiency and membrane fusion ability. On the other hand, in order for lipid nanoparticles made of ionic lipids to exhibit more practical effects as nucleic acid delivery carriers in vivo, directivity to target organs and cells is demanded.

As a method for improving directivity toward target organ or cell, a method for modifying a lipid nanoparticle with a ligand is a known.

For example, Non Patent Literature 2 discloses a lipid nanoparticle modified with GalNAc. In this Literature, a ligand-modified lipid nanoparticle encapsulating a nucleic acid is produced by mixing a ligand-bound PEG lipid that affords cell directivity and a nucleic acid, in addition to an ionic lipid, a phospholipid, and cholesterol. This Literature shows that gene-silencing efficiency is improved by intravenously injecting the thus-produced ligand-modified lipid nanoparticle encapsulating a nucleic acid.

In addition, Non Patent Literature 3 discloses lipid nanoparticles modified with RGD peptide. In this Literature, a ligand-modified lipid nanoparticle encapsulating a nucleic acid is produced by binding a PEG lipid and a ligand in advance to synthesize a ligand-bound PEG lipid, producing a lipid nanoparticle encapsulating a nucleic acid composed of other components, and incubating the obtained lipid nanoparticle encapsulating the nucleic acid and the ligand-bound PEG lipid. This Literature shows that nucleic acid delivery efficiency to tumors is improved by intravenously injecting the thus-produced ligand-modified lipid nanoparticle encapsulating a nucleic acid.

Furthermore, Patent Literature 3 discloses a lipid nanoparticle modified with an anti-CD3 IgG antibody. In this Literature, a ligand-modified lipid nanoparticle encapsulating a nucleic acid is produced by adding a ligand to a lipid nanoparticle encapsulating a nucleic acid produced using an activated PEG lipid. This Literature shows that nucleic acid delivery efficiency to T cells in the spleen is improved by intravenously injecting the thus-produced ligand-modified lipid nanoparticle encapsulating a nucleic acid.

### [Citation List]

### [Patent Literature]

[Patent Literature 1]
   WO 2013/073480
[Patent Literature 2]
   WO 2019/188867
[Patent Literature 3]
   WO 2023/054243

### [Non Patent Literature]

[Non Patent Literature 1]
   Biomaterials 29 (2008) 3477-3496
[Non Patent Literature 2]
   Molecular Therapy vol. 18 no. 7, 1357-1364 july 2010
[Non Patent Literature 3]
   Journal of Controlled Release 173 (2014) 110-118

### [Summary of Invention]

### [Technical Problem]

The method of the aforementioned Non Patent Literature 2 is a method for obtaining a ligand-modified lipid nanoparticle encapsulating a nucleic acid by mixing the constituent components of the lipid nanoparticle, a nucleic acid, and a ligand-bound PEG lipid all at once. The methods of Non Patent Literature 3 and Patent Literature 3 include modification with ligand after production of lipid nanoparticles encapsulating nucleic acids. Both of these methods include simultaneously performing formation of lipid nanoparticles and encapsulation of nucleic acid thereinto to produce lipid nanoparticles encapsulating nucleic acids, where encapsulation of nucleic acids into lipid nanoparticles has a room for improvement.

The present invention was made taking note of the above-mentioned circumstances, and aims to provide a method for producing a ligand-modified lipid nanoparticle encapsulating a nucleic acid, which is capable of encapsulating a nucleic acid in a lipid nanoparticle by a procedure different from that of conventional techniques.

In view of the above-mentioned problem, the present inventors have conducted intensive studies and found that, unlike conventional techniques, nucleic acids can be encapsulated in lipid nanoparticles by first preparing lipid nanoparticles free of nucleic acids, and then encapsulating nucleic acids in these lipid nanoparticles. The present invention based on this finding is as follows.

[1] A method for producing a ligand-modified lipid nanoparticle encapsulating a nucleic acid, comprising
   Step a) of mixing an alcohol solution comprising an ionic lipid, a sterol, and a PEG lipid with an acidic buffer having a pH of 1 to 6.5 to obtain a suspension of a lipid nanoparticle free of nucleic acids; and
   Step b) of mixing the lipid nanoparticle free of nucleic acids and a nucleic acid solution to obtain a suspension of a lipid nanoparticle encapsulating a nucleic acid and Step c) of mixing the suspension of the lipid nanoparticle encapsulating the nucleic acid and a ligand-bound lipid to obtain a suspension of a ligand-modified lipid nanoparticle encapsulating the nucleic acid, or
   Step c') of mixing the lipid nanoparticle free of nucleic acids, water, and a ligand-bound lipid to obtain a suspension of a ligand-modified lipid nanoparticle free of nucleic acids and Step b') of mixing the ligand-modified lipid nanoparticle free of nucleic acids and a nucleic acid solution to obtain a suspension of a ligand-modified lipid nanoparticle encapsulating the nucleic acid, or
   Step d) of mixing the lipid nanoparticle free of nucleic acids, a nucleic acid solution, and a ligand-bound lipid to obtain a suspension of a ligand-modified lipid nanoparticle encapsulating the nucleic acid.
[2] The method of the aforementioned [1], wherein, in Step b), a suspension of the lipid nanoparticle free of nucleic acids and a nucleic acid solution are mixed to obtain a suspension of a lipid nanoparticle encapsulating the nucleic acid, or
   in Step c'), a suspension of the lipid nanoparticle free of nucleic acids and a ligand-bound lipid are mixed to obtain a suspension of a ligand-modified lipid nanoparticle free of nucleic acids and in Step b'), the suspension of the ligand-modified lipid nanoparticle free of nucleic acids and a nucleic acid solution are mixed to obtain a suspension of a ligand-modified lipid nanoparticle encapsulating a nucleic acid, or
   in Step d), a suspension of the lipid nanoparticle free of nucleic acids, a nucleic acid solution, and a ligand-bound lipid are mixed to obtain a suspension of a ligand-modified lipid nanoparticle encapsulating the nucleic acid.
[3] The method of the aforementioned [1] or [2], wherein the method comprises Step a), Step b), and Step c), or Step a), Step c'), and Step b').
[4] The method of any one of the aforementioned [1] to [3], wherein a mixture of the lipid nanoparticle free of nucleic acids and the nucleic acid solution in Step b), or a mixture of the ligand-modified lipid nanoparticle free of nucleic acids and the nucleic acid solution in Step b') is maintained at 0 to 95°C for 1 min to 2 hr.
[5] The method of any one of the aforementioned [1] to [4], wherein a mixture of the ligand-bound lipid and the suspension of the lipid nanoparticle encapsulating the nucleic acid in Step c), or a mixture of the lipid nanoparticle free of nucleic acids, water, and the ligand-bound lipid in Step c') is maintained at 0 to 95°C for 1 min to 5 hr.
[6] The method of any one of the aforementioned [1], [2], and [4] to [5], wherein a mixture of the lipid nanoparticle free of nucleic acids, the nucleic acid solution, and the ligand-bound lipid in Step d) is maintained at 0 to 95°C for 1 min to 2 hr.
[7] The method of any one of the aforementioned [1] to [6], wherein
   in Step c), the suspension of the lipid nanoparticle encapsulating the nucleic acid, a ligand-bound lipid, and a ligand-unbound lipid are mixed to obtain a suspension of a ligand-modified lipid nanoparticle encapsulating the nucleic acid, or
   in Step c'), the lipid nanoparticle free of nucleic acids, water, a ligand-bound lipid, and a ligand-unbound lipid are mixed to obtain a suspension of a ligand-modified lipid nanoparticle free of nucleic acids, or
   in Step d), the lipid nanoparticle free of nucleic acids, a nucleic acid solution, a ligand-bound lipid, and a ligand-unbound lipid are mixed to obtain a suspension of a ligand-modified lipid nanoparticle encapsulating the nucleic acid.
[8] The method of any one of the aforementioned [1] to [7], wherein the alcohol solution in Step a) further comprises a phospholipid.
[9] The method of any one of the aforementioned [1] to [8], wherein the ionic lipid comprises at least one selected from the group consisting of a compound represented by the formula (1): 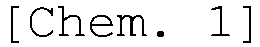 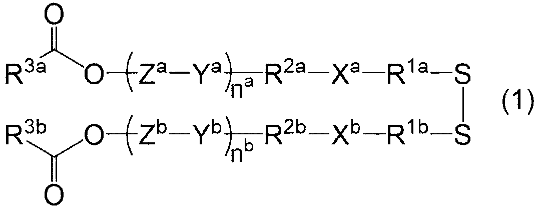 wherein, in the formula (1),
   R^{1a} and R^{1b} are each independently an alkylene group having 1 to 6 carbon atoms,
   X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
   R^{2a} and R^{2b} are each independently an alkylene group or an oxydialkylene group, each having 8 or less carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 to 16 carbon atoms and at least one aromatic ring, and optionally having a hetero atom,
   n^{a} and n^{b} are each independently 0 or 1,
   R^{3a} and R^{3b} are each independently
   a residue derived from a reaction product of a liposoluble vitamin having a hydroxyl group, and succinic anhydride or glutaric anhydride,
   a residue derived from a reaction product of a sterol derivative having a hydroxyl group, and succinic anhydride or glutaric anhydride,
   an aliphatic hydrocarbon group having 1 to 40 carbon atoms,
   an alkyl group having a cyclopropane ring and having 3 to 40 carbon atoms,
   a group represented by the formula (3):

      R⁹-O-CO-(CH₂)ₐ-* (3)

      wherein, in the formula (3),
   * is a bonding position,
   R⁹ is an aliphatic hydrocarbon group having 2 to 20 carbon atoms,
   a is an integer of 2 to 10,
   a group having 50 or less carbon atoms and represented by the formula (4): 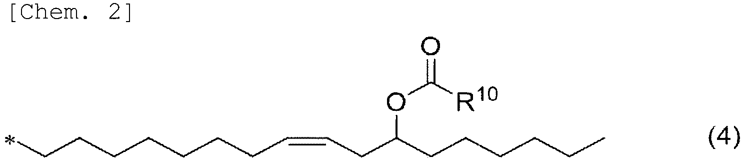 wherein, in the formula (4),
   * is a bonding position,
   R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms, or
   a group having 50 or less carbon atoms and represented by the following formula (5): 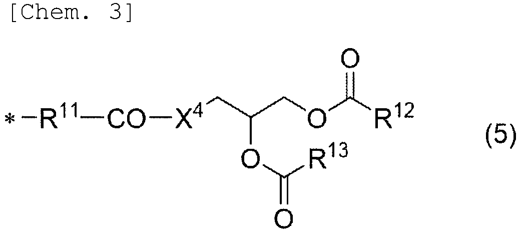 wherein, in the formula (5),
   * is a bonding position,
   R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxy group, and a hydrocarbon ring group having 3 to 12 carbon atoms, and
   R¹² and R¹³ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R¹² is optionally replaced by at least one bond selected from the group consisting of an ester bond, an amide bond, a carbamate bond, and a carbonate bond, at least one ethylene group or at least one trimethylene group in R¹³ is optionally replaced by at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxy group, and a hydrocarbon ring group having 3 to 12 carbon atoms, and
   X⁴ is an oxygen atom, NH, or a sulfur atom, [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, and (6Z,9Z,28Z,31Z)-heptatriaconta-6,9,28,31-tetraen-19-yl 4-(dimethylamino)butanoate.
[10] The method of the aforementioned [9], wherein the ionic lipid comprises a compound represented by the formula (1).
[11] The method of any one of the aforementioned [1] to [10], wherein the ligand in the ligand-bound lipid is a ligand having brain cell directivity or a ligand having T cell directivity.
[12] A method for introducing a nucleic acid into a cell, comprising a step of contacting the ligand-modified lipid nanoparticle encapsulating the nucleic acid obtained by the method of any one of the aforementioned [1] to [11] and the cell in vitro.
[13] A method for introducing a nucleic acid into a target cell in a living body, comprising a step of administering the ligand-modified lipid nanoparticle encapsulating the nucleic acid obtained by the method of any one of the aforementioned [1] to [11] to the living body.
[14] A method for producing a pharmaceutical composition, comprising the method of any one of the aforementioned [1] to [11].

### [Advantageous Effects of Invention]

According to the method for producing ligand-modified lipid nanoparticles encapsulating nucleic acids of the present invention, nucleic acids can be encapsulated in lipid nanoparticles using a procedure different from conventional techniques.

### [Description of Embodiments]

While the embodiments of the present invention are explained in the following, the present invention is not limited thereto. The descriptions in the present specification can be combined with each other unless it is clear that they cannot be combined.

### <Production method of ligand-modified lipid nanoparticle encapsulating nucleic acid>

The present invention provides a method for producing a ligand-modified lipid nanoparticle encapsulating a nucleic acid (hereinafter sometimes abbreviated as "the lipid nanoparticle of interest"), comprising
Step a) of mixing an alcohol solution comprising an ionic lipid, a sterol, and a PEG lipid (hereinafter sometimes abbreviated as "lipid solution") with an acidic buffer having a pH of 1 to 6.5 (hereinafter sometimes abbreviated as "acidic buffer") to obtain a suspension of a lipid nanoparticle free of nucleic acids; and
Step b) of mixing the lipid nanoparticle free of nucleic acids and a nucleic acid solution (preferably mixing a suspension of the lipid nanoparticle free of nucleic acids and a nucleic acid solution) to obtain a suspension of a lipid nanoparticle encapsulating the nucleic acid and Step c) of mixing the suspension of the lipid nanoparticle encapsulating the nucleic acid and a ligand-bound lipid to obtain a suspension of a ligand-modified lipid nanoparticle encapsulating the nucleic acid, or
Step c') of mixing the lipid nanoparticle free of nucleic acids, water, and a ligand-bound lipid (preferably mixing a suspension of the lipid nanoparticle free of nucleic acids and a ligand-bound lipid) to obtain a suspension of a ligand-modified lipid nanoparticle free of nucleic acids and Step b') of mixing the ligand-modified lipid nanoparticle free of nucleic acids and a nucleic acid solution (preferably mixing the suspension of the ligand-modified lipid nanoparticle free of nucleic acids and a nucleic acid solution) to obtain a suspension of a ligand-modified lipid nanoparticle encapsulating the nucleic acid, or
Step d) of mixing the lipid nanoparticle free of nucleic acids, a nucleic acid solution, and a ligand-bound lipid (preferably mixing a suspension of the lipid nanoparticle free of nucleic acids, a nucleic acid solution, and a ligand-bound lipid) to obtain a suspension of a ligand-modified lipid nanoparticle encapsulating the nucleic acid (hereinafter sometimes abbreviated as "the production method of the present invention").

The production method of the present invention includes "Step a), Step b), and Step c)", "Step a), Step c'), and Step b')", or "Step a) and Step d)". Between these steps, other steps (e.g., exchange of dispersion medium, etc.) may be performed as necessary. The production method of the present invention preferably includes "Step a), Step b), and Step c)" or "Step a), Step c'), and Step b')", more preferably "Step a), Step b), and Step c)".

In the present specification, the "lipid nanoparticle" means a particle having a membrane structure wherein the hydrophilic groups of amphiphilic lipid are arranged in the interface, facing the aqueous phase side. The "amphiphilic lipid" means a lipid having both a hydrophilic group and a hydrophobic group. Examples of the amphiphilic lipid include ionic lipid, phospholipid, PEG lipid, and the like.

The lipid nanoparticle of interest obtained by the production method of the present invention contains an ionic lipid, a sterol, and a PEG lipid as the constituent substances of the membrane, and may further contain a phospholipid. The particle size of the lipid nanoparticle of interest is not particularly limited and is preferably 10 nm to 500 nm, more preferably 30 nm to 300 nm. The particle size can be measured by using a particle size distribution measuring device such as Zetasizer Nano (Malvern) or the like. In the present specification, the "particle size" means an average particle size (Zeta-average) measured by a dynamic light scattering method.

In the present specification, the "total lipids" means the total amount of lipids. Examples of the lipid include ionic lipid, sterol, PEG lipid, and phospholipid.

In the present specification, the "(ligand-modified) lipid nanoparticle free of nucleic acids" refers to a (ligand-modified) lipid nanoparticle whose nucleic acid content is below the detection limit.

In the present specification, the "(ligand-modified) lipid nanoparticle encapsulating a nucleic acid" refers to a (ligand-modified) lipid nanoparticle containing a nucleic acid encapsulated into the inside thereof".

In the present specification, the "ligand" refers to a substance that has specific affinity for a molecule expressed on the surface of a target cell to which the lipid nanoparticle is to be delivered (cells (e.g., cancer cells) where the introduction of a drug (preferably a nucleic acid) encapsulated in the lipid nanoparticle affords a therapeutic effect against a disease.)

In the present specification, the "ligand-modified lipid nanoparticle" refers to a lipid nanoparticle having a ligand, which is presented on the surface of the lipid nanoparticle so that the ligand will interact with a molecule expressed on the target cell and having a specific affinity, and be delivered to the target cell.

### <Step a)>

In Step a), a suspension of lipid nanoparticles free of nucleic acids is obtained by mixing a lipid solution and an acidic buffer. The acidic buffer used in Step a) contains water as a solvent, and the suspension of lipid nanoparticles free of nucleic acids obtained in Step a) contains water as a dispersion medium.

Examples of the alcohol in the lipid solution include ethanol, tert-butanol, and the like. Only one kind of the alcohol may be used, or two or more kinds thereof may be used in combination. Alcohol is, preferably ethanol.

The mixing of the lipid solution and the acidic buffer in Step a) is preferably performed using a device including a microfluidic path or a vortex mixer, more preferably performed using a device including a microfluidic path. Examples of the device including a microfluidic path include "NanoAssemblr Ignite" (manufactured by Precision NanoSystems) and nanoparticles production device "iLiNP" (manufactured by Lilac pharma Inc.).

When mixing the lipid solution and acidic buffer in Step a) using a device including a microfluidic path, the flow rates and flow rate ratio of the lipid solution and acidic buffer are not particularly limited as long as they are capable of forming lipid nanoparticles, and can be selected appropriately according to the properties of the microfluidic path and device to be used. The flow rates of the lipid solution and acidic buffer are each preferably 100 to 200,000 µL/min, more preferably 1,000 to 200,000 µL/min. The ratio of the flow rate of the lipid solution to the flow rate of the acidic buffer (flow rate of lipid solution/flow rate of acidic buffer) is preferably 1/10 to 1/1, more preferably 1/8 to 1/2. The "ratio of the flow rate of the lipid solution to the flow rate of the acidic buffer (flow rate of lipid solution/flow rate of acidic buffer)" is equal to the "ratio of the volume of the lipid solution to the volume of the acidic buffer (volume of lipid solution/volume of acidic buffer)".

The temperature at which the lipid solution and acidic buffer are mixed in Step a) is not particularly limited as long as it is a temperature at which lipid nanoparticles can be formed. From the viewpoint of ease of lipid nanoparticle formation, it is preferably 4 to 50°C, more preferably 20 to 30°C.

The alcohol solution (i.e., lipid solution) in Step a) contains an ionic lipid.

In one embodiment of the present invention, ionic lipid preferably contains at least one selected from the group consisting of a compound represented by the following formula (1) (sometimes to be abbreviated as "ionic lipid (1)" in the present specification), [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, and (6Z,9Z,28Z,31Z)-heptatriaconta-6,9,28,31-tetraen-19-yl 4-(dimethylamino)butanoate, more preferably ionic lipid (1).

In another embodiment of the present invention, the ionic lipid is preferably at least one selected from the group consisting of ionic lipid (1), [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, and (6Z,9Z,28Z,31Z)-heptatriaconta-6,9,28,31-tetraen-19-yl 4-(dimethylamino)butanoate,
more preferably ionic lipid (1), [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, or (6Z,9Z,28Z,31Z)-heptatriaconta-6,9,28,31-tetraen-19-yl 4-(dimethylamino)butanoate,
further preferably ionic lipid (1).

Only one kind of ionic lipid (1) may be used, or two or more kinds thereof may be used in combination.

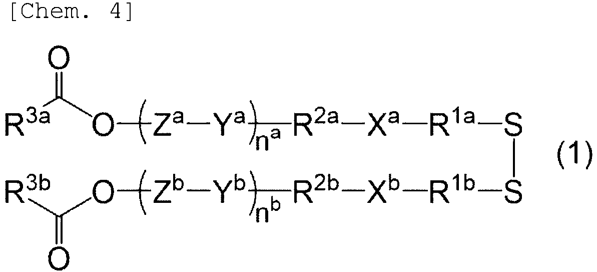

wherein, in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group, each having 8 or less carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1,
R^{3a} and R^{3b} are each independently
a residue derived from a reaction product of a liposoluble vitamin having a hydroxyl group, and succinic anhydride or glutaric anhydride,
a residue derived from a reaction product of a sterol derivative having a hydroxyl group, and succinic anhydride or glutaric anhydride,
an aliphatic hydrocarbon group having 1 to 40 carbon atoms,
an alkyl group having a cyclopropane ring and having 3 to 40 carbon atoms,
a group represented by the formula (3):

   R⁹-O-CO-(CH₂)ₐ-* (3)

   wherein, in the formula (3),
* is a bonding position,
R⁹ is an aliphatic hydrocarbon group having 2 to 20 carbon atoms,
a is an integer of 2 to 10,
a group having 50 or less carbon atoms and represented by the formula (4): 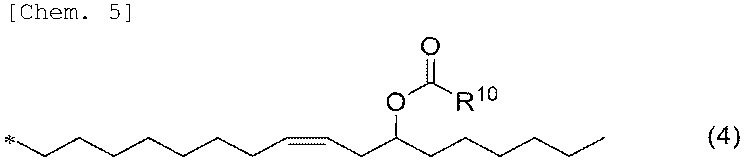 wherein, in the formula (4),
* is a bonding position,
R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms, or
a group having 50 or less carbon atoms and represented by the following formula (5): 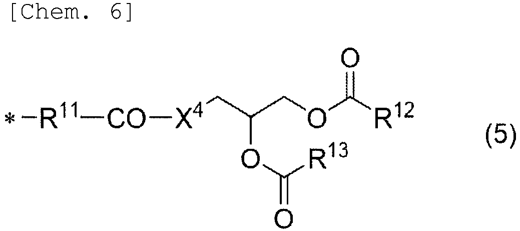 wherein, in the formula (5),
* is a bonding position,
R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxy group, and a hydrocarbon ring group having 3 to 12 carbon atoms, and
R¹² and R¹³ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R¹² is optionally replaced by at least one bond selected from the group consisting of an ester bond, an amide bond, a carbamate bond, and a carbonate bond, at least one ethylene group or at least one trimethylene group in R¹³ is optionally replaced by at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxy group, and a hydrocarbon ring group having 3 to 12 carbon atoms, and
X⁴ is an oxygen atom, NH, or a sulfur atom.

The definition of each symbol in the formula (1) is described in order below.

R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group, and the like. Preferably, R^{1a} and R^{1b} are each independently a methylene group, an ethylene group, a trimethylene group, an isopropylene group, or a tetramethylene group, most preferably an ethylene group.

R^{1a} may be the same as or different from R^{1b}, and R^{1a} is preferably the same group as R^{1b}.

X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

A preferred specific structure of the acyclic alkyl tertiary amino group having 1 to 6 carbon atoms and one tertiary amino group is represented by X¹. The both ends of X¹ in the following formula do not show carbon atoms but show bonding positions.

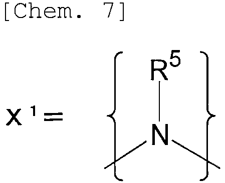

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like, preferably methyl group, ethyl group, propyl group, or isopropyl group, most preferably methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridinediyl group, an azetidinediyl group, a pyrrolidinediyl group, a piperidinediyl group, an imidazolidinediyl group, a piperazinediyl group, preferably a pyrrolidinediyl group, a piperidinediyl group, or a piperazinediyl group, most preferably a piperidinediyl group. In the present specification, "compound name + diyl group (e.g., aziridinediyl group)" refers to a divalent group having a structure obtained by removing two hydrogen atoms from the aforementioned compound.

A preferred specific structure of the cyclic alkylene tertiary amino group having 2 to 5 carbon atoms and one tertiary amino group is represented by X². The both ends of X² in the following formula do not show carbon atoms but show bonding positions.

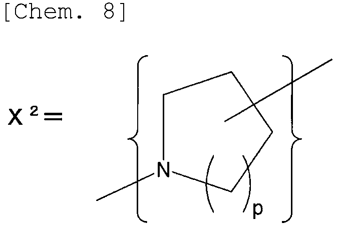

The p in X² is 1 or 2. When p is 1, X² is a pyrrolidinediyl group, and when p is 2, X² is a piperidinediyl group. Preferably, p is 2.

A preferred specific structure of the cyclic alkylene tertiary amino group having 2 to 5 carbon atoms and two tertiary amino groups is represented by X³. The both ends of X³ in the following formula do not show carbon atoms but show bonding positions.

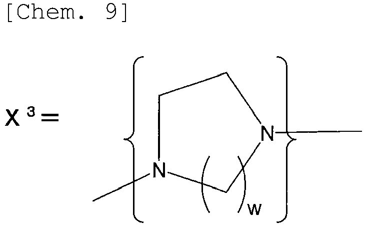

The w in X³ is 1 or 2. When w is 1, X³ is an imidazolidinediyl group, and when w is 2, X³ is a piperazinediyl group.

X^{a} may be the same as or different from X^{b}, and X^{a} is preferably the same group as X^{b}.

R^{2a} and R^{2b} are each independently an alkylene group or an oxydialkylene group, each having 8 or less carbon atoms, preferably each independently an alkylene group having 8 or less carbon atoms.

The alkylene group having 8 or less carbon atoms may be linear or branched, preferably linear. The number of carbons contained in the alkylene group is preferably 6 or less, most preferably 4 or less. Specific examples of the alkylene group having 8 or less carbon atoms include methylene group, ethylene group, trimethylene group, isopropylene group, tetramethylene group, isobutylene group, pentamethylene group, hexamethylene group, heptamethylene group, octamethylene group, and the like, preferably methylene group, ethylene group, trimethylene group, or tetramethylene group, most preferably ethylene group.

In the present specification, the "oxydialkylene group having 8 or less carbon atoms" means alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having 8 or less carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. Preferably, it is an oxydimethylene group, an oxydiethylene group, or an oxydi(tetramethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furan thiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. The both ends of Z¹ in the following formula do not show carbon atoms but show bonding positions. 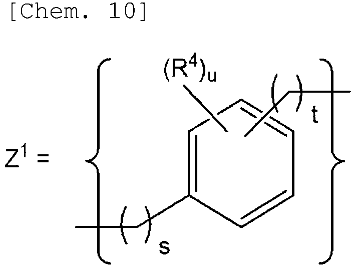 wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of an ionic lipid. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently a residue derived from a reaction product of a liposoluble vitamin having a hydroxyl group, and succinic anhydride or glutaric anhydride, or a residue derived from a reaction product of a sterol derivative having a hydroxyl group, and succinic anhydride or glutaric anhydride, or an aliphatic hydrocarbon group having 1 to 40 carbon atoms, an alkyl group having a cyclopropane ring and having 3 to 40 carbon atoms, a group represented by the aforementioned formula (3), a group having 50 or less carbon atoms and represented by the aforementioned formula (4), or a group having 50 or less carbon atoms and represented by the aforementioned formula (5).

In one embodiment of the present invention, R^{3a} and R^{3b} are
preferably each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having a cyclopropane ring and having 3 to 40 carbon atoms, or a group represented by the aforementioned formula (3),
more preferably, each independently a residue derived from a reaction product of a liposoluble vitamin having a hydroxyl group, and succinic anhydride or glutaric anhydride, or an aliphatic hydrocarbon group having 12 to 22 carbon atoms,
further preferably, each independently an aliphatic hydrocarbon group having 12 to 22 carbon atoms.

In another embodiment of the present invention, R^{3a} and R^{3b} are preferably each independently a residue derived from a reaction product of a liposoluble vitamin having a hydroxyl group, and succinic anhydride or glutaric anhydride, an aliphatic hydrocarbon group having 12 to 22 carbon atoms, a group having 50 or less carbon atoms and represented by the aforementioned formula (4), or a group having 50 or less carbon atoms and represented by the aforementioned formula (5).

In another embodiment of the present invention, it is more preferable that
R^{3a} and R^{3b} are each independently a residue derived from a reaction product of a liposoluble vitamin having a hydroxyl group, and succinic anhydride or glutaric anhydride,
R^{3a} and R^{3b} are each independently an aliphatic hydrocarbon group having 12 to 22 carbon atoms,
R^{3a} and R^{3b} are each independently a group having 50 or less carbon atoms and represented by the aforementioned formula (4),
one of R^{3a} and R^{3b} is an aliphatic hydrocarbon group having 12 to 22 carbon atoms, and the other is a group having 50 or less carbon atoms and represented by the aforementioned formula (4), or
one of R^{3a} and R^{3b} is an aliphatic hydrocarbon group having 12 to 22 carbon atoms, and the other is a group having 50 or less carbon atoms and represented by the aforementioned formula (5).

In another embodiment of the present invention, it is more preferable that
R^{3a} and R^{3b} are each independently a residue derived from a reaction product of a liposoluble vitamin having a hydroxyl group, and succinic anhydride or glutaric anhydride,
R^{3a} and R^{3b} are each independently an aliphatic hydrocarbon group having 12 to 22 carbon atoms,
one of R^{3a} and R^{3b} is an aliphatic hydrocarbon group having 12 to 22 carbon atoms, and the other is a group having 50 or less carbon atoms and represented by the aforementioned formula (4), or
one of R^{3a} and R^{3b} is an aliphatic hydrocarbon group having 12 to 22 carbon atoms, and the other is a group having 50 or less carbon atoms and represented by the aforementioned formula (5).

In another embodiment of the present invention, it is particularly preferable that
R^{3a} and R^{3b} are each independently a residue derived from a reaction product of a liposoluble vitamin having a hydroxyl group, and succinic anhydride or glutaric anhydride, or
R^{3a} and R^{3b} are each independently an aliphatic hydrocarbon group having 12 to 22 carbon atoms.

In still another embodiment of the present invention, it is more preferable that
R^{3a} and R^{3b} are each independently an aliphatic hydrocarbon group having 12 to 22 carbon atoms,
R^{3a} and R^{3b} are each independently a group having 50 or less carbon atoms and represented by the aforementioned formula (4), or
one of R^{3a} and R^{3b} is an aliphatic hydrocarbon group having 12 to 22 carbon atoms, and the other is a group having 50 or less carbon atoms and represented by the aforementioned formula (4).

In still another embodiment of the present invention, R^{3a} and R^{3b} are more preferably each independently an aliphatic hydrocarbon group having 12 to 22 carbon atoms.

The "residue derived from a reaction product of a liposoluble vitamin having a hydroxyl group, and succinic anhydride or glutaric anhydride" is a group having a structure in which the hydroxyl group of a liposoluble vitamin having a hydroxyl group is replaced by *-O-CO-CH₂-CH₂- or *-O-CO-CH₂-CH₂-CH₂-. * is the bonding position with the liposoluble vitamin.

The "residue derived from a reaction product of a sterol derivative having a hydroxyl group, and succinic anhydride or glutaric anhydride" is a group having a structure in which the hydroxyl group of a sterol derivative having a hydroxyl group is replaced by *-O-CO-CH₂-CH₂- or *-O-CO-CH₂-CH₂-CH₂-. * is the bonding position with the sterol derivative.

Examples of the liposoluble vitamin having a hydroxyl group include retinol, ergosterol, 7-dehydrocholesterol, calciferol, cholecalciferol, dihydroergocalciferol, dihydrotachysterol, tocopherol, tocotrienol, and the like. The liposoluble vitamin having a hydroxyl group is preferably tocopherol.

Examples of the sterol derivative having a hydroxyl group include cholesterol, cholestanol, stigmasterol, β-sitosterol, lanosterol, ergosterol, and the like, preferably cholesterol or cholestanol.

The aliphatic hydrocarbon group having 1 to 40 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 to 22, more preferably 13 to 19, most preferably 13 to 17. While the aliphatic hydrocarbon group includes an alkyl group, an alkenyl group, an alkynyl group, and the like, it is preferably an alkyl group or an alkenyl group. Specific examples of the aliphatic hydrocarbon group having 1 to 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, decadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, and the like. The aliphatic hydrocarbon group having 1 to 40 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In one embodiment of the present invention, the aliphatic hydrocarbon group having 1 to 40 (preferably 12 to 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, or a heptadecenyl group when oleic acid is used as the fatty acid.

The "alkyl group having a cyclopropane ring and having 3 to 40 carbon atoms" for R^{3a} or R^{3b} means an alkyl group having at least one cyclopropane ring in the alkyl chain and having 3 to 40 carbon atoms. The number of carbon atoms in the alkyl group is 3 to 40, and does not include the number of carbon atoms in the cyclopropane ring. The number of cyclopropane rings in the alkyl group is preferably one. The alkyl group having a cyclopropane ring and having 3 to 40 carbon atoms for R^{3a} or R^{3b} is preferably a group represented by the formula (6): 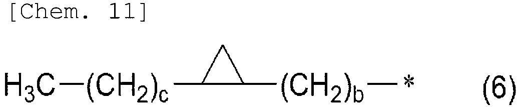 wherein, in the formula (6), * is a bonding position, b and c are each independently an integer, and the total of b and c is 2 to 39. Preferably, b is an integer of 1 to 20, c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (6) include 7-(2-octylcyclopropyl)heptyl and the like.

In the formula (3), * does not show a carbon atom but shows a bonding position as mentioned above.

In the formula (3), the aliphatic hydrocarbon group having 2 to 20 carbon atoms for R⁹ may be linear or branched.

The aforementioned aliphatic hydrocarbon group may be saturated or unsaturated. When the aforementioned aliphatic hydrocarbon group is an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aforementioned aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably alkyl group. Specific examples of the aliphatic hydrocarbon group having 2 to 20 carbon atoms include ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 to 20 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In the formula (3), a is preferably an integer of 3 to 9, more preferably an integer of 3 to 7, further preferably an integer of 5 to 7, and most preferably 5 or 7.

In the formula (4), * does not show a carbon atom but shows a bonding position as mentioned above.

In the formula (4), R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ may be substituted by a substituent selected from the group consisting of a 3- to 14-membered heterocyclic group and a hydrocarbon ring group having 3 to 12 carbon atoms.

In the present specification, "R¹⁰ may be substituted by a substituent selected from the group consisting of a 3- to 14-membered heterocyclic group and a hydrocarbon ring group having 3 to 12 carbon atoms" (hereinafter abbreviated as "the aforementioned expression") means that the aforementioned alkyl group, the aforementioned alkenyl group, the aforementioned alkynyl group, and the aforementioned hydrocarbon ring group for R¹⁰ are each independently optionally substituted by a substituent selected from the group consisting of a 3- to 14-membered heterocyclic group and a hydrocarbon ring group having 3 to 12 carbon atoms. Other expressions similar to the aforementioned expression have the same meaning as the aforementioned expression. In the present specification, unless specifically described as to a substituent, the "alkyl group", "alkenyl group", "alkynyl group", "alkylene group", "alkenediyl group", and "alkynediyl group" are unsubstituted.

In the present specification, the alkyl group may be linear or branched. Examples of the alkyl group include methyl group, ethyl group, propyl group, isopropyl group, butyl group, isobutyl group, sec-butyl group, tert-butyl group, a pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, hentriacontyl group, dotriacontyl group, tritriacontyl group, tetratriacontyl group, pentatriacontyl group, hexatriacontyl group, tetracontyl group, hentetracontyl group, dotetracontyl group, tritetracontyl group, and tetratetracontyl group.

In the present specification, the alkenyl group may be linear or branched. In the present specification, the number of the olefinic carbon-carbon double bond in the alkenyl group may be only one, or two or more. Examples of the alkenyl group include ethenyl group, propenyl group, butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, tricosenyl group, tetracosenyl group, pentacosenyl group, hexacosenyl group, heptacosenyl group, octacosenyl group, nonacosenyl group, triacontenyl group, hentriacontenyl group, and dotriacontenyl group.

In the present specification, the alkynyl group may be linear or branched. In the present specification, the number of the carbon-carbon triple bond of the alkynyl group may be only one, or two or more. Examples of the alkynyl group include ethynyl group, propynyl group, butynyl group, pentynyl group, hexynyl group, heptynyl group, octynyl group, nonynyl group, decynyl group, undecynyl group, dodecynyl group, tridecynyl group, tetradecynyl group, pentadecynyl group, hexadecynyl group, heptadecynyl group, octadecynyl group, nonadecynyl group, icosynyl group, henicosynyl group, docosynyl group, tricosynyl group, tetracosinyl group, pentacosinyl group, hexacosinyl group, heptacosinyl group, octacosinyl group, nonacosinyl group, triacontinyl group, hentriacontinyl group, and dotriacontinyl group.

In the present specification, the "hydrocarbon ring group having 3 to 12 carbon atoms" means a cyclic group wherein the ring thereof is composed of 3 to 12 carbon atoms. Examples of the hydrocarbon ring group having 3 to 12 carbon atoms include cycloalkyl group having 3 to 8 carbon atoms, phenyl group, naphthyl group, and adamantyl group. Examples of the cycloalkyl group having 3 to 8 carbon atoms include cyclopropyl group, cyclobutyl group, cyclopentyl group, cyclohexyl group, cycloheptyl group, and cyclooctyl group. The hydrocarbon ring group having 3 to 12 carbon atoms is preferably a non-aromatic hydrocarbon ring group having 3 to 12 carbon atoms, more preferably a cycloalkyl group having 3 to 8 carbon atoms or an adamantyl group, further preferably a cyclohexyl group or an adamantyl group.

In the present specification, the "3- to 14-membered heterocyclic group" means a heterocyclic group wherein the ring thereof is composed of 3 to 14 atoms. Examples of the 3- to 14-membered heterocyclic group include 5- to 14-membered aromatic heterocyclic group, and 3- to 14-membered non-aromatic heterocyclic group. The 3- to 14-membered heterocyclic group is preferably a 3- to 14-membered non-aromatic heterocyclic group.

In the present specification, examples of the 5- to 14-membered aromatic heterocyclic group include the following:
(i) 5- to 6-membered monocyclic aromatic heterocyclic groups such as thienyl group, furyl group, pyrrolyl group, imidazolyl group, pyrazolyl group, thiazolyl group, isothiazolyl group, oxazolyl group, isoxazolyl group, pyridyl group, pyrazinyl group, pyrimidinyl group, pyridazinyl group, 1,2,4-oxadiazolyl group, 1,3,4-oxadiazolyl group, 1,2,4-thiadiazolyl group, 1,3,4-thiadiazolyl group, triazolyl group, tetrazolyl group, triazinyl group, and the like;
(ii) 8- to 14-membered fused polycyclic aromatic heterocyclic groups such as benzothiophenyl group, benzofuranyl group, benzoimidazolyl group, benzoxazolyl group, benzoisoxazolyl group, benzothiazolyl group, benzoisothiazolyl group, benzotriazolyl group, imidazopyridinyl group, thienopyridinyl group, furopyridinyl group, pyrrolopyridinyl group, pyrazolopyridinyl group, oxazolopyridinyl group, thiazolopyridinyl group, imidazopyrazinyl group, imidazopyrimidinyl group, thienopyrimidinyl group, furopyrimidinyl group, pyrrolopyrimidinyl group, pyrazolopyrimidinyl group, oxazolopyrimidinyl group, thiazolopyrimidinyl group, pyrazolotriazinyl group, naphtho[2,3-b]thienyl group, phenoxathiinyl group, indolyl group, isoindolyl group, 1H-indazolyl group, purinyl group, isoquinolyl group, quinolyl group, phthalazinyl group, naphthyridinyl group, quinoxalinyl group, quinazolinyl group, cinnolinyl group, carbazolyl group, β-carbolinyl group, phenanthridinyl group, acrydinyl group, phenazinyl group, phenothiazinyl group, phenoxazinyl group, and the like.

In the present specification, examples of the 3- to 14-membered non-aromatic heterocyclic group include the following:
(i) 3- to 8-membered monocyclic non-aromatic heterocyclic groups such as aziridinyl group, oxiranyl group, thiiranyl group, azetidinyl group, oxetanyl group, thietanyl group, tetrahydrothienyl group, tetrahydrofuranyl group, pyrrolinyl group, pyrrolidinyl group, imidazolinyl group, imidazolidinyl group, oxazolinyl group, oxazolidinyl group, pyrazolinyl group, pyrazolidinyl group, thiazolinyl group, thiazolidinyl group, tetrahydroisothiazolyl group, dithioranyl group (e.g., 1,2-dithiolan-3-yl group), tetrahydroxazolyl group, tetrahydroisoxazolyl group, piperidinyl group, piperazinyl group, tetrahydropyridinyl group, dihydropyridinyl group, dihydrothiopyranyl group, tetrahydropyrimidinyl group, tetrahydropyridazinyl group, dihydropyranyl group, tetrahydropyranyl group, tetrahydrothiopyranyl group, morpholinyl group, thiomorpholinyl group, azepanyl group, diazepanyl group, azepinyl group, oxepanyl group, azocanyl group, diazocanyl group, and the like;
(ii) 9- to 14-membered fused polycyclic non-aromatic heterocyclic groups such as dihydrobenzofuranyl group, dihydrobenzoimidazolyl group, dihydrobenzoxazolyl group, dihydrobenzothiazolyl group, dihydrobenzoisothiazolyl group, dihydronaphto[2,3-b]thienyl group, tetrahydroisoquinolyl group, tetrahydroquinolyl group, 4H-quinolizinyl group, indolinyl group, isoindolinyl group, tetrahydrothieno[2,3-c]pyridinyl group, tetrahydrobenzoazepinyl group, tetrahydroquinoxalinyl group, tetrahydrophenanthridinyl group, hexahydrophenothiazinyl group, hexahydrophenoxazinyl group, tetrahydrophthalazinyl group, tetrahydronaphthyridinyl group, tetrahydroquinazolinyl group, tetrahydrocinnolinyl group, tetrahydrocarbazolyl group, tetrahydro-β-carbolinyl group, tetrahydroacrydinyl group, tetrahydrophenazinyl group, tetrahydrothioxanthenyl group, octahydroisoquinolyl group, and the like.

In one embodiment of the present invention, R¹⁰ is preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms.

In one embodiment of the present invention, R¹⁰ is more preferably an alkyl group having 1 to 20 carbon atoms, an alkenyl group having 2 to 20 carbon atoms, an alkynyl group having 2 to 20 carbon atoms, or a cyclohexyl group, and the aforementioned alkyl group is optionally substituted by an adamantyl group.

In one embodiment of the present invention, R¹⁰ is further preferably an alkyl group having 1 to 20 carbon atoms, particularly preferably an alkyl group having 1 to 10 carbon atoms, most preferably a heptyl group.

In another embodiment of the present invention, R¹⁰ is preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a 3- to 14-membered non-aromatic heterocyclic group (e.g., dithioranyl group).

In another embodiment of the present invention, R¹⁰ is more preferably an alkyl group having 1 to 20 carbon atoms or an alkynyl group having 2 to 20 carbon atoms, and the aforementioned alkyl group is optionally substituted by a 3- to 14-membered non-aromatic heterocyclic group (e.g., dithioranyl group).

In one embodiment of the present invention, R¹⁰ is further preferably an alkyl group having 1 to 10 carbon atoms or an alkynyl group having 1 to 10 carbon atoms, and the aforementioned alkyl group is optionally substituted by a 3- to 14-membered non-aromatic heterocyclic group (e.g., dithioranyl group).

In the formula (5), * does not show a carbon atom but shows a bonding position as mentioned above.

In the formula (5), R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxy group, and a hydrocarbon ring group having 3 to 12 carbon atoms.

In the present specification, the alkylene group may be linear or branched. Examples of the alkylene group include methylene group, ethylene group, trimethylene group (-(CH₂)₃-), propylene group (-CH(CH₃)CH₂-, -CH₂CH(CH₃)-), tetramethylene group (-(CH₂)₄-), butylene group (-CH(C₂H₅)CH₂-, -CH₂CH(C₂H₅)-), pentamethylene group (-(CH₂)₅-), hexamethylene group (-(CH₂)₆-), heptamethylene group (-(CH₂)₇-), octamethylene group (-(CH₂)₈-), nonamethylene group (-(CH₂)₉-), and decamethylene group (-(CH₂)₁₀-) (in the aforementioned formulas, "-" is a single bond).

In the present specification, the "alkenediyl group" refers to a divalent group having a structure obtained by removing two hydrogen atoms from an alkene. In the present specification, the alkenediyl group may be linear or branched. The number of olefinic carbon-carbon double bonds in the alkene or alkenediyl group may be one or two or more. Examples of the alkenediyl group include ethenediyl group, propenediyl group, butenediyl group, pentenediyl group, hexenediyl group, heptenediyl group, octenediyl group, nonenediyl group, and decenediyl group. In the present specification, "compound name + diyl group (e.g., ethenediyl group)" refers to a divalent group having a structure obtained by removing two hydrogen atoms from the aforementioned compound.

In the present specification, the "alkynediyl group" means a divalent group having a structure obtained by removing two hydrogen atoms from an alkyne. In the present specification, the alkynediyl group may be linear or branched. In the present specification, the number of carbon-carbon triple bonds in the alkyne or alkynediyl group may be one or two or more. Examples of the alkynediyl group include ethynediyl group, propynediyl group, butynediyl group, pentynediyl group, hexynediyl group, heptynediyl group, octynediyl group, noninediyl group, and decynediyl group.

In the formula (5), R¹¹ is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms or an alkenediyl group having 2 to 9 carbon atoms, further preferably an alkylene group having 2 to 9 carbon atoms, particularly preferably an alkylene group having 2 or 3 carbon atoms, most preferably a trimethylene group.

In the formula (5), R¹² and R¹³ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R¹² may be substituted by at least one bond selected from the group consisting of an ester bond, an amide bond, a carbamate bond, and a carbonate bond, and at least one ethylene group or at least one trimethylene group in R¹³ may be substituted by at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxy group, and a hydrocarbon ring group having 3 to 12 carbon atoms.

R¹² and R¹³ are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms, further preferably an alkyl group having 1 to 10 carbon atoms, particularly preferably a hexyl group.

In the formula (5), X⁴ is an oxygen atom, NH, or a sulfur atom. X⁴ is preferably an oxygen atom or NH, more preferably an oxygen atom.

In the formula (5), it is most preferable that R¹¹ is a trimethylene group, R¹² and R¹³ are both hexyl groups, and X⁴ is an oxygen atom.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of ionic lipid (1) include the following ionic lipids.

### [Ionic lipid (1-1a)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 8 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, an aliphatic hydrocarbon group having 12 to 22 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 20 carbon atoms or an alkynyl group having 2 to 20 carbon atoms, and the aforementioned alkyl group is optionally substituted by a 3- to 14-membered non-aromatic heterocyclic group (e.g., dithioranyl group)), or a group having 50 or less carbon atoms and represented by the aforementioned formula (5) (in the formula (5), R¹¹ is an alkylene group having 2 to 9 carbon atoms, R¹² and R¹³ are each independently an alkyl group having 1 to 17 carbon atoms, and X⁴ is an oxygen atom or NH).

### [Ionic lipid (1-1b)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 8 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, an aliphatic hydrocarbon group having 12 to 22 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 20 carbon atoms), or a group having 50 or less carbon atoms and represented by the aforementioned formula (5) (in the formula (5), R¹¹ is an alkylene group having 2 to 9 carbon atoms, R¹² and R¹³ are each independently an alkyl group having 1 to 17 carbon atoms, and X⁴ is an oxygen atom or NH).

### [Ionic lipid (1-1c)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 8 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 to 22 carbon atoms (e.g., heptadecenyl group, heptadecadienyl group, 1-hexylnonyl group).

### [Ionic lipid (1-1d)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidinediyl group);
R^{2a} and R^{2b} are each independently an alkylene group having 8 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently an aliphatic hydrocarbon group having 12 to 22 carbon atoms (e.g., heptadecenyl group, heptadecadienyl group, 1-hexylnonyl group), or a group having 50 or less carbon atoms and represented by the aforementioned formula (4) (in the formula (4), R¹⁰ is an alkyl group having 1 to 20 carbon atoms or an alkynyl group having 2 to 20 carbon atoms, and the aforementioned alkyl group is optionally substituted by a 3- to 14-membered non-aromatic heterocyclic group (e.g., dithioranyl group)).

### [Ionic lipid (1-2a)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidinediyl group);
R^{2a} and R^{2b} are each independently an alkylene group having 6 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, an aliphatic hydrocarbon group having 13 to 19 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms or an alkynyl group having 1 to 10 carbon atoms, the aforementioned alkyl group is optionally substituted by a 3- to 14-membered non-aromatic heterocyclic group (e.g., dithioranyl group)), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-2b)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidinediyl group);
R^{2a} and R^{2b} are each independently an alkylene group having 6 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, an aliphatic hydrocarbon group having 13 to 19 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-2c)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidinediyl group);
R^{2a} and R^{2b} are each independently an alkylene group having 6 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride, or an aliphatic hydrocarbon group having 13 to 19 carbon atoms (e.g., heptadecenyl group, heptadecadienyl group, 1-hexylnonyl group).

### [Ionic lipid (1-2d)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidinediyl group);
R^{2a} and R^{2b} are each independently an alkylene group having 6 or less carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently an aliphatic hydrocarbon group having 13 to 19 carbon atoms (e.g., heptadecenyl group, heptadecadienyl group, 1-hexylnonyl group) or a group having 50 or less carbon atoms and represented by the aforementioned formula (4) (in the formula (4), R¹⁰ is an alkyl group having 1 to 10 carbon atoms or an alkynyl group having 1 to 10 carbon atoms, and the aforementioned alkyl group is optionally substituted by a 3- to 14-membered non-aromatic heterocyclic group (e.g., dithioranyl group)).

### [Ionic lipid (1-3a)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 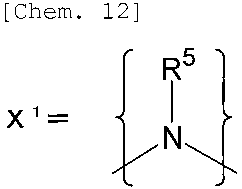 wherein R⁵ is an alkyl group having 1 to 3 carbon atoms (e.g., a methyl group), or X²: 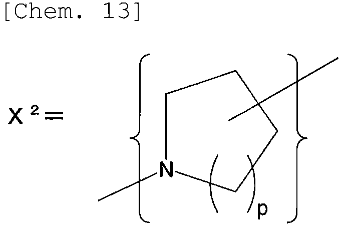 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 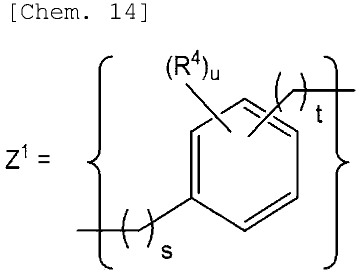 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, an aliphatic hydrocarbon group having 13 to 17 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms or an alkynyl group having 2 to 10 carbon atoms, the aforementioned alkyl group is optionally substituted by a 3- to 14-membered non-aromatic heterocyclic group (e.g., dithioranyl group)), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-3b)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 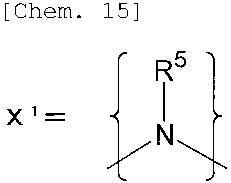 wherein R⁵ is an alkyl group having 1 to 3 carbon atoms (e.g., a methyl group), or X²: 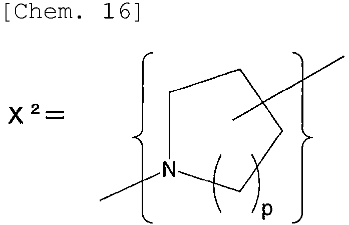 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 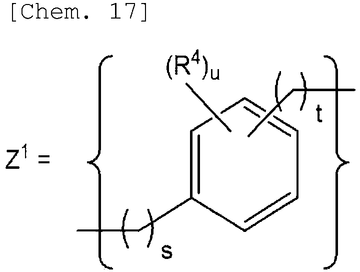 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, an aliphatic hydrocarbon group having 13 to 17 carbon atoms (e.g., heptadecenyl group, heptadecadienyl group, 1-hexylnonyl group), a group having 50 or less carbon atoms and represented by the aforementioned formula (4) (in the formula (4), R¹⁰ is an alkyl group having 1 to 10 carbon atoms), or a group having 50 or less carbon atoms and represented by the aforementioned formula (5) (in the formula (5), R¹¹ is an alkylene group having 2 or 3 carbon atoms, R¹² and R¹³ are each independently an alkyl group having 1 to 10 carbon atoms, and X⁴ is an oxygen atom).

### [Ionic lipid (1-3c)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 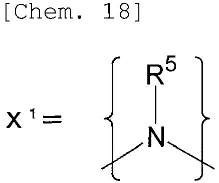 wherein R⁵ is an alkyl group having 1 to 3 carbon atoms (e.g., a methyl group), or X²: 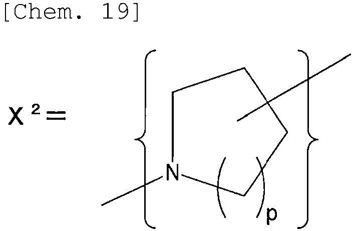 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 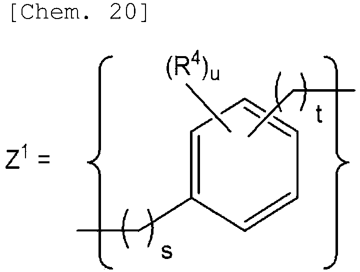 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a residue derived from a reaction product of a liposoluble vitamin having a hydroxyl group (e.g., tocopherol) and succinic anhydride, or an aliphatic hydrocarbon group having 13 to 17 carbon atoms (e.g., heptadecenyl group, heptadecadienyl group, 1-hexylnonyl group).

### [Ionic lipid (1-3d)]

Ionic lipid (1) wherein
R^{1a} and R^{1b} are each independently an alkylene group having 1 to 2 carbon atoms (e.g., methylene group, ethylene group);
X^{a} and X^{b} are each independently X¹: 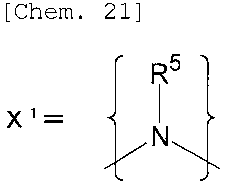 wherein R⁵ is an alkyl group having 1 to 3 carbon atoms (e.g., a methyl group), or X²: 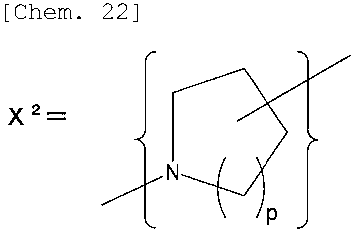 wherein p is 1 or 2;
R^{2a} and R^{2b} are each independently alkylene group having 4 or less carbon atoms (e.g., methylene group, ethylene group, trimethylene group);
Y^{a} and Y^{b} are each independently an ester bond or an amide bond;
Z^{a} and Z^{b} are each independently Z¹: 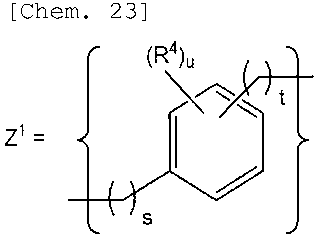 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently an aliphatic hydrocarbon group having 13 to 17 carbon atoms (e.g., heptadecenyl group, heptadecadienyl group, 1-hexylnonyl group) or a group having 50 or less carbon atoms and represented by the aforementioned formula (4) (in the formula (4), R¹⁰ is an alkyl group having 1 to 10 carbon atoms or an alkynyl group having 2 to 10 carbon atoms, and the aforementioned alkyl group is optionally substituted by a 3- to 14-membered non-aromatic heterocyclic group (e.g., dithioranyl group)).

Specific examples of ionic lipid (1) include compounds described in the following Table 1-1 to Table 1-5.

**[Table 1-1]**

| name of ionic lipid | structure |
|---|---|
| O-Ph-P3C1 | 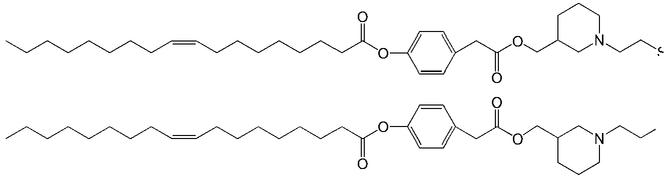 |
| O-Ph-P4C1 | 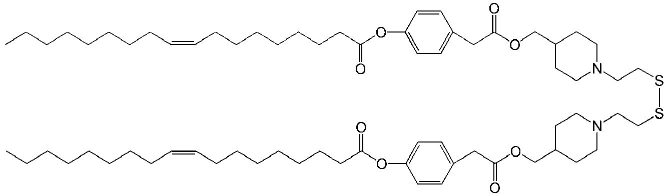 |
| O-Ph-P4C2 (or SS-OP) | 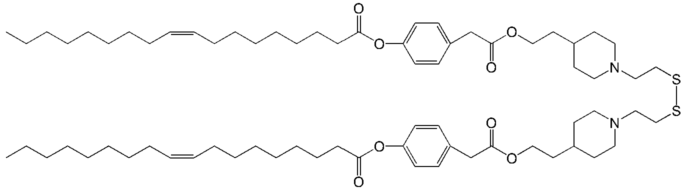 |
| O-Bn-P4C2 | 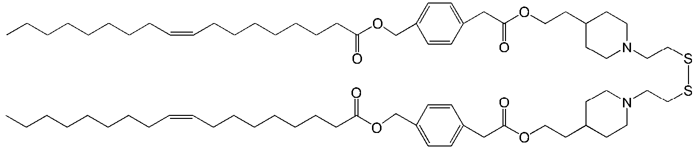 |
| E-Ph-P4C2 (or SS-EP) | 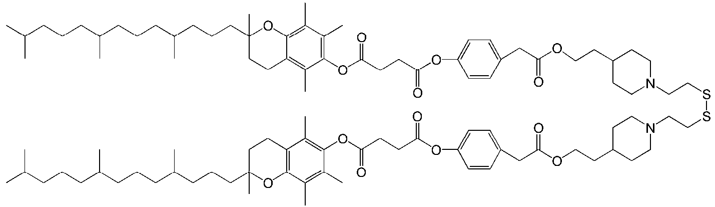 |

**[Table 1-2]**

| name of ionic lipid | structure |
|---|---|
| L-Ph-P4C2 | 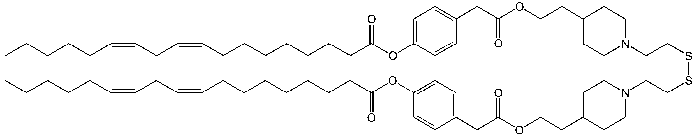 |
| HD-Ph-P4C2 | 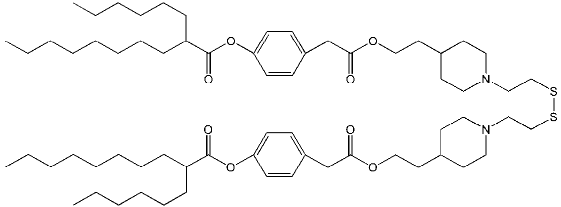 |
| O-Ph-amide-P4C2 | 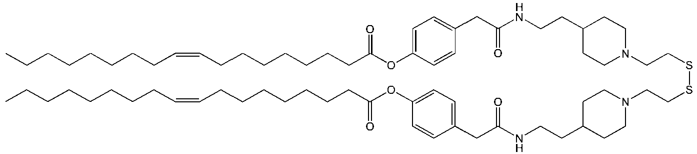 |
| O-Ph-C3M | 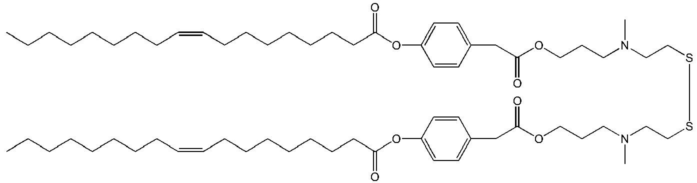 |

**[Table 1-3]**

| name of ionic lipid | structure |
|---|---|
| B-2 | 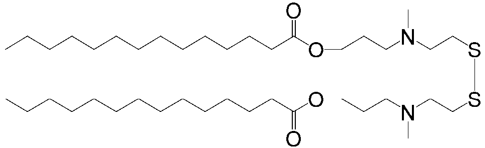 |
| B-2-5 | 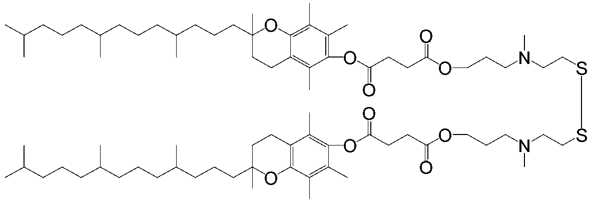 |
| TS-P4C2 (or SS-EC) | 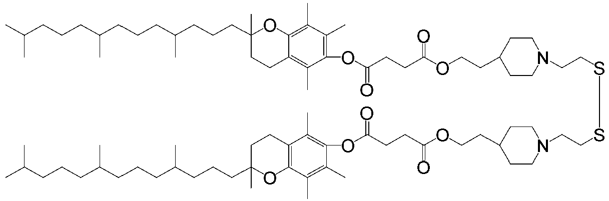 |
| L-P4C2 | 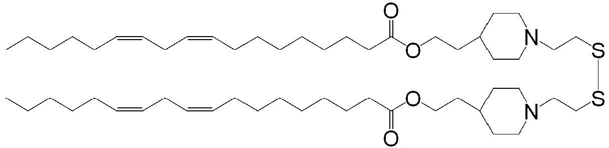 |
| O-P4C2 (or SS-OC) | 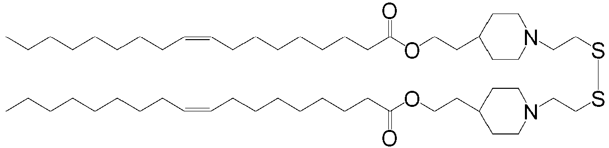 |

**[Table 1-4]**

| name of ionic lipid | structure |
|---|---|
| compound 1 | 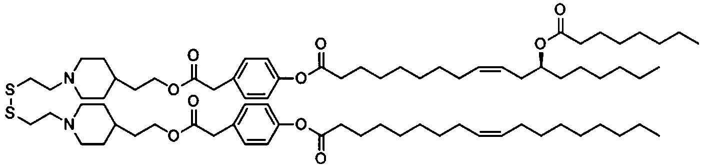 |
| compound 2 | 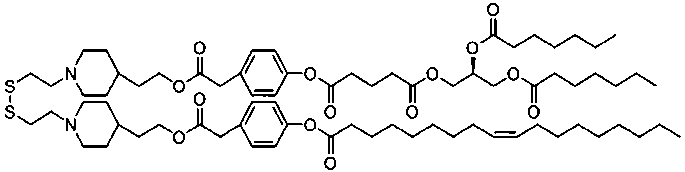 |

**[Table 1-5]**

| name of ionic lipid | structure |
|---|---|
| compound 3 | 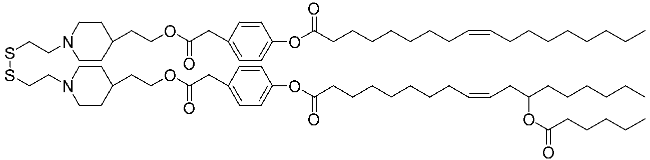 |
| compound 4 | 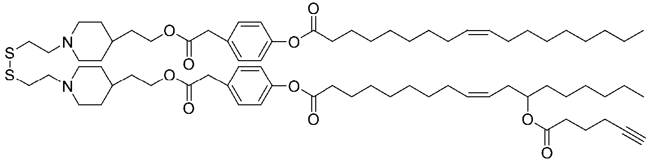 |
| compound 5 | 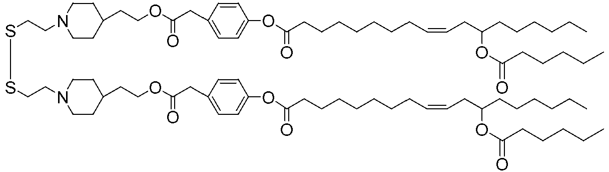 |
| compound 6 | 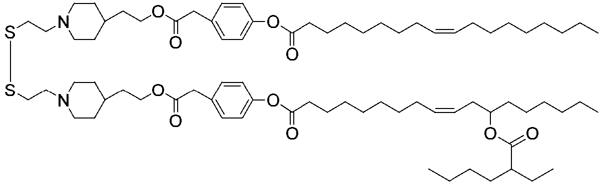 |

In one embodiment of the present invention, the ionic lipid (1) (i.e., the compound represented by the formula (1)) is
preferably at least one selected from the group consisting of the compounds described in the aforementioned Table 1-1 to Table 1-5,
more preferably at least one selected from the group consisting of SS-OP, SS-EC, compound 1, compound 2, compound 3, compound 4, compound 5, and compound 6.

In another embodiment of the present invention, the ionic lipid (1) (i.e., a compound represented by the formula (1)) is
preferably at least one selected from the group consisting of the compounds described in the aforementioned Table 1 -1 to Table 1 -4,
more preferably at least one selected from the group consisting of SS-OP, SS-EC, compound 1, and compound 2,
further preferably SS-OP.

In one embodiment of the present invention, the ionic lipid is
preferably at least one selected from the group consisting of SS-OP, compound 3, compound 4, compound 5, compound 6, [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, and (6Z,9Z,28Z,31Z)-heptatriaconta-6,9,28,31-tetraen-19-yl 4-(dimethylamino)butanoate,
more preferably SS-OP, compound 3, compound 4, compound 5, compound 6, [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, or (6Z,9Z,28Z,31Z)-heptatriaconta-6,9,28,31-tetraen-19-yl 4-(dimethylamino)butanoate,
further preferably SS-OP, compound 3, compound 4, compound 5, or compound 6,
particularly preferably SS-OP.

The ionic lipid may be a commercially available product (e.g., "ALC-0315" manufactured by Jenkem Technology USA; "SM-102" and "DLin-MC3-DMA" manufactured by Cayman Chemical). In addition, ionic lipid (1) (i.e., a compound represented by the formula (1)) can be produced by a known method (e.g., methods described in WO2019/188867A1 (US2021/0023008A1), US9708628B2, WO2021/195529A2). In addition, compound 1 and compound 2 can be produced by the following synthesis methods. Ionic lipid (1) other than Compound 1 or Compound 2 can also be produced in the same manner as in the synthesis of Compound 1 or Compound 2.

### (I) Synthesis method of compound 1

### <Synthesis of intermediate 1>

Intermediate 1 was synthesized by the following synthesis pathway.

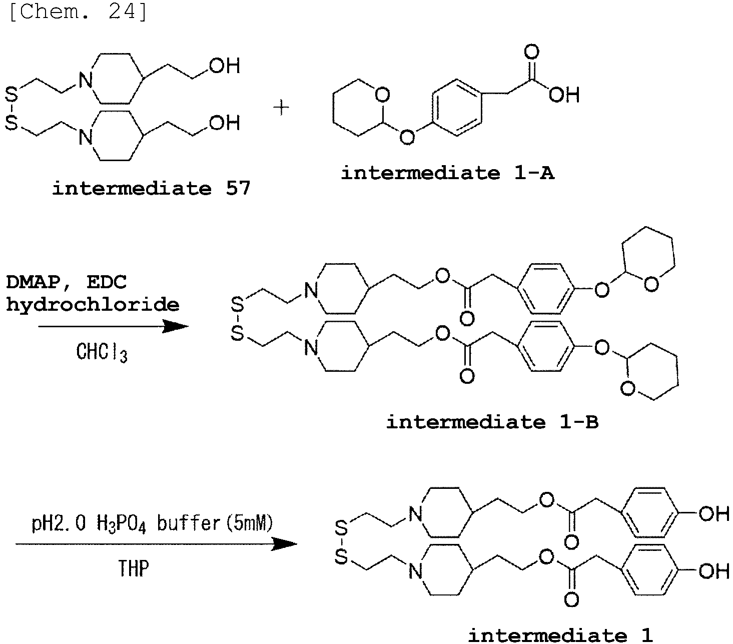

### <Synthesis of intermediate 1-A>

4-Hydroxyphenylacetic acid (30.0 g, 197 mmol), and pyridinium p-toluenesulfonate (5.00 g, 19.9 mmol) were dissolved in dichloromethane (120 mL) at room temperature. To the obtained mixture was added dropwise a mixed solution of 3,4-dihydro-2H-pyran (83.0 g, 987 mmol) and dichloromethane (31.1 mL) at 20°C or below and the mixture was reacted at room temperature for 2 hr. Thereafter, the reaction mixture was neutralized with DMAP (12.0 g, 98.2 mmol). To the obtained mixture were added 2-propanol (301 mL), 100 g/L NaOH aqueous solution (160 g), and the mixture was reacted at room temperature for 1 hr. The mixed solution was concentrated by an evaporator, the concentrate was washed with chloroform and neutralized with 6 M hydrochloric acid. The obtained mixture was extracted with chloroform, and the organic layer was dehydrated by adding sodium sulfate. Sodium sulfate was removed by filtration, and the filtrate was concentrated by an evaporator to give 47.0 g of intermediate 1-A.

### <Synthesis of intermediate 1-B>

Intermediate 57 (36.0 g, 95.6 mmol) synthesized according to the method described in WO 2016/121942, intermediate 1-A (49.7 g, 210 mmol), and DMAP (4.68 mg, 38.3 mmol) were dissolved in chloroform (240 mL) at room temperature. To the obtained mixture was added EDC hydrochloride (55.1 g, 287 mmol), and the mixture was reacted at room temperature for 2 hr. Thereafter, the reaction solution was washed with 5 wt% sodium dihydrogen phosphate aqueous solution, 9 wt% sodium hydrogen carbonate aqueous solution, and 20 wt% saline, and dehydrated by adding sodium sulfate. Sodium sulfate was removed by filtration, and the filtrate was concentrated by an evaporator to give 77.9 g of intermediate 1-B.

### <Synthesis of intermediate 1>

Intermediate 1-B (77.7 g, 95.6 mmol) was dissolved in THF (325 mL) at room temperature. To the obtained mixture were added 2-propanol (319 mL), p-toluenesulfonic acid monohydrate (38.2 g, 201 mmol) and the mixture was reacted at 25°C or below for 1 hr. Thereafter, to the reaction mixture was neutralized with DMAP (25.0 g, 205 mmol). DMAP was removed by filtration, and the filtrate was concentrated by an evaporator. The obtained residue was dissolved in chloroform (627 mL), washed with 0.5 M phosphate buffer (pH=6.5), 0.5 M glycinate buffer (pH=9.5) and dehydrated by adding sodium sulfate. Sodium sulfate was removed by filtration, and toluene (1.56 L) was added to allow for crystallization. The obtained crude product was washed with hexane and vacuum dried to give 40.3 g of intermediate 1.

### <Synthesis of intermediate 1-1>

Oleic acid (5.11 g, 18.1 mmol), intermediate 1 (16.6 g, 25.8 mmol), and DMAP (630 mg, 5.16 mmol) were dissolved in chloroform (166 mL) at room temperature. To the obtained mixture was added EDC hydrochloride (5.94 g, 31.0 mmol) and the mixture was reacted at room temperature for 2 hr. Thereafter, the reaction solution was washed with 5 wt% sodium hydrogen phosphate aqueous solution, 0.5 M phosphate buffer (pH=2.0), and 20 wt% saline, and dehydrated by adding sodium sulfate. Sodium sulfate was removed by filtration, and the filtrate was concentrated by an evaporator. The obtained residue was purified by silica gel column chromatography (chloroform/ethanol=88/12) to give 8.21 g of intermediate 1-1.

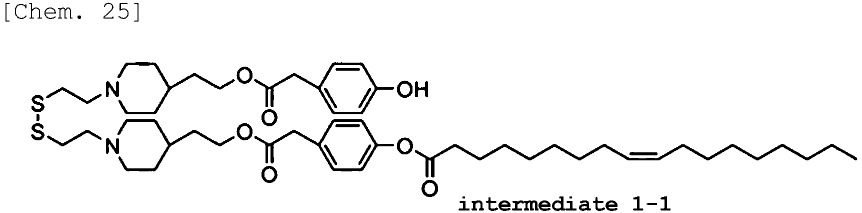

### <Synthesis of intermediate 5-1>

Methyl ricinoleate (5.00 g, 16.0 mmol), octanoic acid (2.54 g, 17.6 mmol), and DMAP (391 mg, 3.20 mmol) were dissolved in chloroform (50.0 g) at room temperature. To the obtained mixture was added EDC hydrochloride (4.60 g, 24.0 mmol) and the mixture was reacted at room temperature for 1 hr. Thereafter, the reaction solution was washed with 0.5 M phosphate buffer (pH 4.0), 7 wt% sodium bicarbonate water, and 20 wt% brine, and dehydrated with sodium sulfate. Sodium sulfate was removed by filtration, and the filtrate was concentrated by an evaporator to obtain 6.78 g of intermediate 5-1.

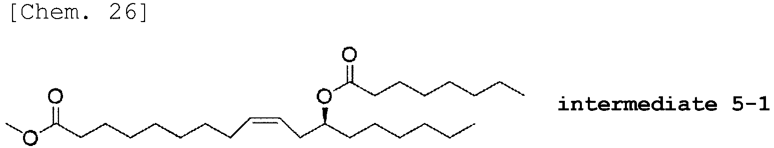

### <Synthesis of intermediate 5>

Intermediate 5-1 (6.40 g, 14.6 mmol) was dissolved in tert-butanol to 0.3 M at room temperature. To the obtained mixture was added 2 M NaOH aqueous solution (8.17 g) and the mixture was reacted at room temperature for 8 hr. Thereafter, 1 M hydrochloric acid was added, the obtained mixture was extracted with hexane, the organic layer was washed with 20 wt% brine and dehydrated with sodium sulfate. Sodium sulfate was removed by filtration, and the filtrate was concentrated by an evaporator. The obtained residue was purified by silica gel column chromatography (chloroform/ethanol =94/6 (volume ratio)) to obtain 5.93 g of intermediate 5.

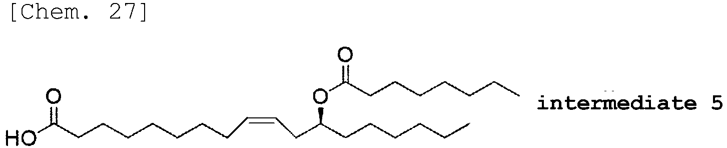

### <Synthesis of compound 1>

Intermediate 1-1 (492 mg, 0.541 mmol) was dissolved in chloroform (2.91 g), intermediate 5 (230 mg, 0.541 mmol), DMAP (13.2 mg, 0.108 mmol), and EDC hydrochloride (207 mg, 1.08 mmol) were added, and the mixture was reacted at room temperature for 2 hr. The solution after the reaction was washed with 5 wt% saline, and concentrated by an evaporator, and the concentrate was subjected to silica gel column purification using ethanol/chloroform to give compound 1 (532 mg).

### (II) Synthesis method of compound 2

### <Synthesis of intermediate 11-1>

(R)-(-)-2,2-dimethyl-1,3-dioxolane-4-methanol (25.0 g, 189 mmol), and imidazole (19.3 g, 284 mmol) were dissolve in dimethylformamide (251 g) at room temperature. To the obtained mixture was added TBDPS-Cl (57.3 g, 208 mmol) and the mixture was reacted at room temperature for 1 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 56.0 g of intermediate 11-1.

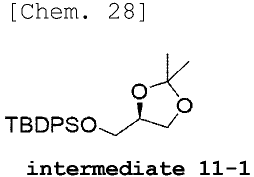

### <Synthesis of intermediate 11-2>

Intermediate 11-1 (55.9 g, 151 mmol), 0.5 M phosphate buffer (pH 1.0) (503 g) were dissolved in THF (530 g) at room temperature, and the mixture was reacted at 50°C for 12 hr. 1.0 M NaOH aqueous solution was added to pH 7.0. To the obtained mixture was added chloroform, and the mixture was washed with 20 wt% saline, and dehydrated by adding sodium sulfate. Sodium sulfate was removed by filtration, and the filtrate was concentrated by an evaporator to give 44.9 g of intermediate 11-2.

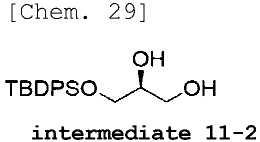

### <Synthesis of intermediate 14-1>

Intermediate 11-2 (6.90 g, 20.9 mmol), heptanoic acid (6.84 g, 46.0 mmol), and DMAP (0.511 g, 4.18 mmol) were dissolved in chloroform (69.5 g) at room temperature. To the obtained mixture was added EDC hydrochloride (12.0 g, 62.7 mmol) and the mixture was reacted at room temperature for 1 hr. Thereafter, the reaction solution was washed with 0.5 M phosphate buffer (pH 4.0), 7 wt% sodium bicarbonate water, and 20 wt% brine, and dehydrated with sodium sulfate. Sodium sulfate was removed by filtration, and the filtrate was concentrated by an evaporator to obtain 9.03 g of intermediate 14-1.

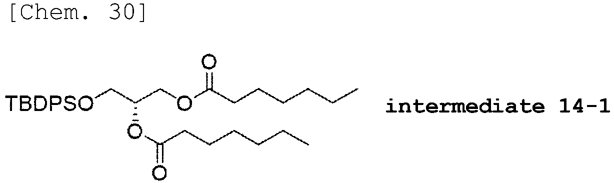

### <Synthesis of intermediate 14-2>

Intermediate 14-1 (9.41 g, 17.9 mmol) and acetic acid (3.92 g, 65.3 mmol) were dissolved in THF (27.2 g) at room temperature. To the obtained mixture was added 1 M TBAF solution (59.9 g, 65.2 mmol) in THF and the mixture was reacted at room temperature for 18 hr. Thereafter, to the reaction solution was added ethyl acetate (94.3 g). Thereafter, the reaction solution was washed with 0.5 M phosphate buffer (pH 4.0) and 20 wt% brine, and dehydrated with sodium sulfate. Sodium sulfate was removed by filtration, and the filtrate was concentrated by an evaporator to obtain 4.90 g of intermediate 14-2.

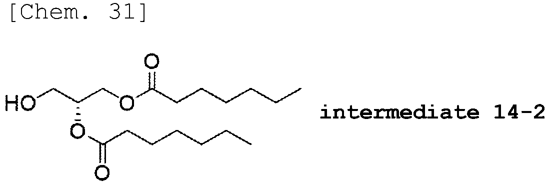

### <Synthesis of intermediate 14>

Intermediate 14-2 (4.90 g, 15.4 mmol), glutaric anhydride (3.73 g, 32.7 mmol), triethylamine (4.95 g, 48.9 mmol), and DMAP (0.398 g, 3.26 mmol) were dissolved in chloroform (51.6 g) at room temperature, and the mixture was reacted at room temperature for 1 hr. Thereafter, the reaction solution was washed with 0.5 M phosphate buffer (pH 4.0), 7 wt% sodium bicarbonate water, and 20 wt% brine, and dehydrated with sodium sulfate. Sodium sulfate was removed by filtration, and the filtrate was concentrated by an evaporator to obtain 3.16 g of intermediate 14.

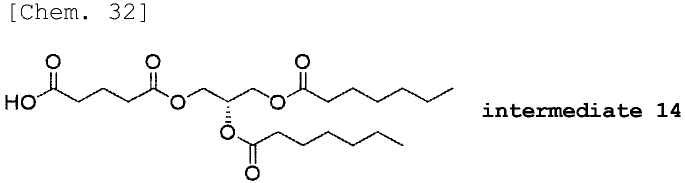

### <Synthesis of compound 2>

Using intermediate 14 and intermediate 1-1 and in the same manner as in the synthesis of compound 1 as mentioned above, 0.215 g of compound 2 was synthesized.

In one embodiment of the present invention, the concentration of ionic lipid (1) in the alcohol solution (i.e., lipid solution) in Step a) is preferably 0.05 to 20 mM, more preferably 0.1 to 15 mM, further preferably 1 to 10 mM, from the viewpoint of the solubility of the lipid.

In another embodiment of the present invention, the concentration of ionic lipid (1) in the alcohol solution (i.e., lipid solution) in Step a) is preferably 0.05 to 30 mM, more preferably 0.1 to 25 mM, further preferably 0.3 to 20 mM, from the viewpoint of the solubility of the lipid.

When two or more kinds of ionic lipid (1) are used, "the concentration of ionic lipid (1)" in the present specification refers to the total concentration of the two or more kinds of ionic lipid (1). The "concentration of other components" also means the same as the "concentration of ionic lipid (1)".

The content of ionic lipid (1) in the lipid nanoparticle of interest is preferably 20 to 75 mol%, more preferably 30 to 70 mol%, further preferably 35 to 60 mol%, with respect to the total lipids, from the aspects of efficiency of nucleic acid encapsulation, efficiency of intracellular release of nucleic acid, and stability of the lipid nanoparticles.

When two or more kinds of ionic lipid (1) are used, "the content of ionic lipid (1)" in the present specification refers to the total content of the two or more kinds of ionic lipid (1). The "content of other components" also means the same as the "content of ionic lipid (1)". In the present specification, the "content of B with respect to A (mol%)" or the "amount of B with respect to A (mol%)" means "100 x amount of B (mol)/amount of A (mol)".

The alcohol solution in Step a) preferably further contains a phospholipid. Only one kind of the phospholipid may be used, or two or more kinds thereof may be used in combination.

Examples of the phospholipid include 1,2-diacyl-sn-glycero-3-phosphocholine (PC), 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), 1,2-diacyl-sn-glycero-3-phosphatidic acid (PA), and lyso forms of these.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), and 1-stearoyl-2-oleoyl-sn-glycero-3-phosphocholine (SOPC).

The above-mentioned specific examples of 1,2-diacyl-sn-glycero-3-phosphocholine (PC) wherein the phosphocholine moiety is substituted by phosphoethanolamine, phosphoserine, phosphoglycerol, or phosphatidic acid, such as 1,2-diacyl-sn-glycero-3-phosphoethanolamine (PE), 1,2-diacyl-sn-glycero-3-phosphoserine (PS), 1,2-diacyl-sn-glycero-3-phosphoglycerol (PG), and 1,2-diacyl-sn-glycero-3-phosphatidic acid (PA), can also be used.

As the phospholipid, PC and PE are preferred, DOPC, DSPC, DEPC, POPC, DOPE (1,2-dioleoyl-sn-glycero-3-phosphoethanolamine) and POPE (1-palmitoyl-2-oleoyl-sn-glycero-3-phosphoethanolamine) are more preferred, DOPC, DSPC, and DEPC are further preferred, and DOPC is particularly preferred.

When phospholipid is used, from the viewpoint of the solubility of the lipid, the concentration of the phospholipid in the alcohol solution (i.e., lipid solution) in Step a) in one embodiment of the present invention is preferably 0.01 to 5 mM, more preferably 0.05 to 3 mM, further preferably 0.1 to 2 mM.

When phospholipid is used, from the viewpoint of the solubility of the lipid, the concentration of the phospholipid in the alcohol solution (i.e., lipid solution) in Step a) in another embodiment of the present invention is preferably 0.01 to 20 mM, more preferably 0.01 to 15 mM, further preferably 0.03 to 10 mM.

The content of the phospholipid in the lipid nanoparticle of interest is preferably 0 to 35 mol%, more preferably 2.5 to 30 mol%, further preferably 5 to 30 mol%, with respect to the total lipids, from the aspects of efficiency of nucleic acid encapsulation, efficiency of intracellular release of nucleic acid, and stability of the lipid nanoparticles.

The alcohol solution (i.e., lipid solution) in Step a) contains a sterol. Only one kind of the sterol may be used, or two or more kinds thereof may be used in combination.

Examples of the sterol include cholesterol, lanosterol, phytosterol, zymosterol, zymostenol, desmosterol, stigmastanol, dihydrolanosterol, and 7-dehydrocholesterol. As the sterol, cholesterol, lanosterol, and phytosterol are preferred, and cholesterol is more preferred,.

From the viewpoint of sterol solubility, the concentration of the sterol in the alcohol solution (i.e., lipid solution) in Step a) in one embodiment of the present invention is preferably 0.05 to 15 mM, more preferably 0.1 to 10 mM, further preferably 0.5 to 10 mM.

From the viewpoint of sterol solubility, the concentration of the sterol in the alcohol solution (i.e., lipid solution) in Step a) in another embodiment of the present invention is preferably 0.001 to 25 mM, more preferably 0.01 to 20 mM, further preferably 0.05 to 15 mM.

The content of the sterol in the lipid nanoparticle of interest is preferably 5 to 60 mol%, more preferably 7 to 55 mol%, further preferably 10 to 50 mol%, with respect to the total lipids, from the aspects of efficiency of nucleic acid encapsulation, efficiency of intracellular release of nucleic acid, and stability of the lipid nanoparticles.

The alcohol solution in step (a) (i.e., lipid solution) contains a PEG lipid. In the present specification, "PEG lipid" means a lipid having a polyethylene glycol (PEG) chain. Only one kind of the PEG lipid may be used or two or more kinds thereof may be used in combination.

PEG lipids are used to coat the surface of lipid nanoparticles with hydrophilic polyethylene glycol (PEG), to suppress the aggregation of lipid nanoparticles, and to suppress the interaction between biological components and particles when administered to a living body.

The PEG moiety in PEG lipid can have any molecular weight. In some embodiments, the number average molecular weight of this PEG moiety moiety is preferably 200 to 10,000, more preferably 1,000 to 10,000. This PEG moiety moiety may be linear or branched.

Examples of the PEG lipid include PEG-phospholipid (i.e., PEG-conjugated phospholipid), PEG-ceramide (i.e., PEG-conjugated ceramide), PEG-diacylglycerol (i.e., PEG-conjugated diacylglycerol), and PEG-cholesterol (i.e., PEG-conjugated cholesterol). Among these, PEG-diacylglycerol is preferred.

The PEG lipid is
preferably PEG-diacylglycerol with a number-average molecular weight of the PEG moiety of 1,000 to 10,000 (i.e., PEG-conjugated diacylglycerol),
more preferably PEG-dimyristoylglycerol with a number-average molecular weight of the PEG moiety of 1,000 to 10,000 (i.e., PEG-conjugated dimyristoylglycerol) and PEG-distearoylglycerol with a number-average molecular weight of the PEG moiety of 1,000 to 10,000 (i.e., PEG-conjugated distearoylglycerol),
further preferably PEG-dimyristoylglycerol with a number-average molecular weight of the PEG moiety of 1,000 to 10,000 (e.g., DMG-PEG2000 used in the below-mentioned Examples).

The concentration of the PEG lipid in the alcohol solution (i.e., lipid solution) in Step a) is preferably 0.001 to 1 mM, more preferably 0.005 to 0.8 mM, further preferably 0.01 to 0.5 mM, from the viewpoint of the solubility of the lipid.

The content of the PEG lipid in the lipid nanoparticle of interest is preferably 0.1 to 6.0 mol%, more preferably 0.5 to 5 mol%, further preferably 0.7 to 3.0 mol%, with respect to the total (mol) of other lipids, from the viewpoint of efficiency of nucleic acid encapsulation, efficiency of intracellular release of nucleic acid, and stability of the lipid nanoparticles. As used herein, the aforementioned "PEG lipid" refers to the PEG lipid used in Step a), and does not include ligand-bound PEG lipids (one type of ligand-bound lipid) that may be used in Step c), and the like. In addition, the aforementioned "other lipids" refers to lipids other than the PEG lipid used in Step a). For example, when ionic lipid (1), a sterol, and a PEG lipid are used as lipids in Step a), "the total of other lipids" means the total of the ionic lipid (1) and the sterol. Furthermore, for example, when ionic lipid (1), a phospholipid, a sterol, and a PEG lipid are used as lipids in Step a), "the total of other lipids" means the total of ionic the lipid (1), the phospholipid, and the sterol.

From the viewpoints of total lipid solubility and nucleic acid encapsulation, the concentration of total lipids is preferably 0.1 to 30 mM, more preferably 0.5 to 25 mM, further preferably 1 to 20 mM.

From the viewpoints of total lipid solubility and nucleic acid encapsulation, the concentration of total lipids in the alcohol solution (i.e., lipid solution) in Step a) is preferably 0.1 to 40 mM, more preferably 0.5 to 30 mM, further preferably 1 to 25 mM.

In Step a), an acidic buffer with pH 1 to 6.5 is used. Examples of the aforementioned acidic buffer include HCl/KCl buffer, p-toluenesulfonic acid/Na p-toluenesulfonate buffer, tartaric acid/NaOH buffer, citrate buffer, KH phthalate/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1M NaCl buffer, phenylacetic acid/Na phenylacetate buffer, acetic acid/Na acetate buffer, succinic acid/NaOH buffer, KH phthalate/NaOH buffer, Na cacodylate/HCl buffer, NaH maleate/NaOH buffer, maleic acid/trishydroxymethylaminomethane (Tris)/NaOH buffer, phosphate buffer, KH₂PO₄/NaOH buffer, imidazole/HCl buffer, s-collidine (2,4,6-trimethylpyridine)/HCl buffer, triethanolamine HCl/NaOH buffer, Na 5,5-diethylbarbiturate/HCl buffer, N-methylmorpholine/HCl buffer, Na pyrophosphate/HCl buffer, 2-morpholinoethanesulfonic acid (MES) buffer, malic acid buffer, N-(2-acetamido)iminodiacetic acid (ADA) buffer, 1,4-piperazinediethanesulfonic acid (PIPES) buffer, N-(2-acetamido)-2-aminoethanesulfonic acid (ACES) buffer, N-(2-hydroxyethyl)piperazine-N'-(2-ethanesulfonic acid) (HEPES) buffer, N,N-bis(2-hydroxyethyl)-2-aminoethanesulfonic acid (BES) buffer, bis-Tris buffer, bis-Tris propane buffer, anhydrous sodium carbonate buffer, glycylglycine buffer, 3-(N-morpholino)propanesulfonic acid (MOPS) buffer, 2-hydroxy-3-morpholinopropanesulfonic acid (MOPSO) buffer, and N-tris(hydroxymethyl)methyl-2-aminoethanesulfonic acid (TES) buffer.

The acidic buffer is preferably malic acid buffer, citrate buffer, acetic acid/Na acetate buffer, or 2-morpholinoethanesulfonic acid (MES) buffer, more preferably malic acid buffer or MES buffer, further preferably malic acid buffer. The pH of the aforementioned acidic buffer is 1 to 6.5, preferably 3 to 6.5.

From the viewpoint of lipid nanoparticle formation, the total concentration of the acid and a salt thereof in the acidic buffer (hereinafter sometimes abbreviated as "buffer concentration") is preferably 10 to 100 mM, more preferably 10 to 80 mM.

### <Exchange of dispersion medium>

The dispersion medium of a suspension of lipid nanoparticles free of nucleic acids, a suspension of lipid nanoparticles encapsulating nucleic acids, a suspension of ligand-modified lipid nanoparticles free of nucleic acids, or a suspension of ligand-modified lipid nanoparticles encapsulating nucleic acids (hereinafter sometimes collectively abbreviated as "lipid nanoparticle suspension") can be exchanged for other buffer different from the acidic buffer used in Step a) by an operation such as ultrafiltration, dialysis, dilution, and the like. Other buffer contains water as a solvent, and a suspension in which the dispersion medium has been exchanged contains water as the dispersion medium.

Examples of other buffer include tartaric acid/NaOH buffer, KH phthalate/HCl buffer, glycine/HCl buffer, trans-aconitic acid/NaOH buffer, formic acid/Na formate buffer, 3,3-dimethylglutaric acid/NaOH buffer, 3,3-dimethylglutaric acid/NaOH/0.1 M NaCl buffer, phenylacetic acid/Na phenylacetate buffer, acetic acid/Na acetate buffer, succinic acid/NaOH buffer, KH phthalate/NaOH buffer, Na cacodylate/HCl buffer, NaH maleate/NaOH buffer, maleic acid/trishydroxymethylaminomethane(Tris)/NaOH buffer, phosphate buffer, KH₂PO₄/NaOH buffer, 2-morpholinoethanesulfonic acid (MES) buffer, malic acid buffer, phthalic acid buffer, maleic acid buffer, succinic acid buffer, tartaric acid buffer, citrate buffer, bis-Tris buffer, glycyl glycine buffer, and the like.

Other buffer is preferably malic acid buffer, citrate buffer, acetic acid/Na acetate buffer, 2-morpholinoethanesulfonic acid (MES) buffer, or maleic acid/tris hydroxymethylaminomethane (Tris)/NaOH buffer, more preferably malic acid buffer or MES buffer, further preferably MES buffer. The pH of other buffer is preferably 4.5 to 6.9, more preferably 5.0 to 6.9, further preferably 5.5 to 6.9. The total concentration of the acid and a salt thereof in other buffer (hereinafter sometimes abbreviated as "buffer concentration") is preferably 10 to 100 mM, more preferably 20 to 50 mM, from the viewpoint of nucleic acid encapsulation and lipid nanoparticle formation.

It is preferable to exchange the dispersion medium such that the concentration of each component in the suspension after the exchange of the dispersion medium is approximately the same as that in the suspension before the exchange of the dispersion medium (i.e., suspension obtained in Step a)).

### <Mixing of other components>

The lipid nanoparticle suspension may be mixed with other components. Examples of other component include saccharides such as monosaccharide, sugar alcohol, disaccharide, oligosaccharide, polysaccharide, and the like. Saccharide is preferably disaccharide, more preferably sucrose. The sugar concentration in the lipid nanoparticle suspension is preferably 0 to 320 mg/mL, further preferably 0 to 160 mg/mL.

### <Storage of suspension of lipid nanoparticles free of nucleic acids>

The suspension of lipid nanoparticles free of nucleic acids obtained in Step a) may be frozen or lyophilized and stored before use in Step b), Step c'), or Step d), or it may be stored as a suspension. Furthermore, the lipid nanoparticle suspensions obtained in other steps may also be frozen or lyophilized and stored before use in the next step, or may be stored as a suspension. Storage may be performed under air or an inert atmosphere (e.g., under argon atmosphere).

The temperature for freezing the suspension of lipid nanoparticles free of nucleic acids is preferably -200°C to - 5°C, more preferably -85°C to -10°C, and the freezing time is preferably 1 to 72 hr, more preferably 3 to 24 hr.

The temperature for freeze-drying the suspension of lipid nanoparticles free of nucleic acids is preferably -80°C to 50°C, more preferably -60°C to 40°C, the freeze-drying pressure is preferably 0 to 300 mTorr, more preferably 50 to 250 mTorr, and the freezing time is preferably 12 to for 120 hr, more preferably 24 to for 100 hr. It is preferable to initially freeze-dry at a low temperature, and then gradually increase the temperature.

From the viewpoint of the stability of the constituent components of lipid nanoparticles, the storage temperature of a frozen or lyophilized suspension of lipid nanoparticles free of nucleic acids is preferably -80°C to 0°C, more preferably -80°C to -10°C, further preferably -80°C to -20°C.

From the viewpoint of the stability of the constituent components of lipid nanoparticles, the storage temperature for storing a suspension of lipid nanoparticles free of nucleic acids as is is preferably 1°C to 50°C, more preferably 1°C to 30°C, further preferably 1°C to 10°C, most preferably 1°C to 5°C.

When a frozen or lyophilized suspension of lipid nanoparticles free of nucleic acids has been stored, the obtained frozen product is thawed at 0 to 100°C to prepare a suspension before use in Step b) or Step c'), or water is added to the lyophilized product and the obtained mixture is maintained at 0 to 100°C to prepare a suspension. It is desirable to add water to the lyophilized product such that the concentrations of the components of the suspension obtained after the addition of water are of the same level as those of the suspension before lyophilization.

From the viewpoint of the stability of the constituent components of lipid nanoparticles, the the melting temperature of the aforementioned frozen product or the temperature at which the mixture of the aforementioned lyophilized product and water is maintained is preferably 0 to 95°C, more preferably 0 to 50°C, further preferably 0 to 30°C, most preferably 0 to 10°C. The thawing of the aforementioned frozen product, the addition of water to the aforementioned lyophilized product, and the maintenance of the obtained mixture are preferably performed under atmospheric pressure.

### <Step b)>

In one embodiment of the production method of the present invention, Step b) is performed after Step a) and other steps that are performed as necessary. In Step b), a suspension of lipid nanoparticles encapsulating nucleic acids is obtained by mixing the lipid nanoparticle free of nucleic acids and a nucleic acid solution. Examples of the materials to be mixed with the nucleic acid solution in Step b) include (i) a suspension of the lipid nanoparticle free of nucleic acids, and (ii) a lyophilized product of the lipid nanoparticle free of nucleic acids obtained by lyophilizing the aforementioned suspension. In Step b), a suspension of the lipid nanoparticle free of nucleic acids and a nucleic acid solution are preferably mixed. The nucleic acid solution used in Step b) contains water as a solvent, and the suspension of the lipid nanoparticle encapsulating the nucleic acid obtained in Step b) contains water as a dispersant. Furthermore, in a preferred embodiment of the present invention, the suspension of the lipid nanoparticle free of nucleic acids used in Step b) contains water as a dispersion medium.

The mixing in Step b) can be performed, for example, by pipetting or using a device including a microfluidic path or a mixer. Examples of the mixer include, but are not limited to, vortex mixer, T-junction mixer, and jet mixer.

Examples of nucleic acids include those described below. Preferably, the nucleic acid is mRNA.

In Step b), the molar ratio of the total amino groups of the ionic lipids in the lipid nanoparticle free of nucleic acids (e.g., a suspension of the lipid nanoparticle free of nucleic acids, or a lyophilized product of the lipid nanoparticle free of nucleic acids) to the total phosphate groups of the nucleic acid in the nucleic acid solution (total amount of amino groups of ionic lipids (mol)/total amount of phosphate groups of nucleic acid (mol)) (sometimes referred to as the "N/P ratio" in the present specification) is preferably 5 to 280, more preferably 10 to 140, and further preferably 15 to 70, from the viewpoint of nucleic acid encapsulation efficiency and toxicity.

The concentration of the nucleic acid in the nucleic acid solution (i.e., amount of nucleic acid (µg)/volume of solution (mL)) is not particularly limited as long as the desired target nucleic acid can be introduced into cells, and is preferably 0.25 to 2700 µg/mL, more preferably 0.25 to 1000 µg/mL, further preferably 10 to 450 µg/mL.

The nucleic acid solution is preferably an aqueous nucleic acid solution. In order to increase the encapsulation rate of nucleic acids in lipid nanoparticles, the aqueous nucleic acid solution may contain alcohol. Examples of the alcohol include methanol, ethanol, n-butanol, and tert-butanol. Only one alcohol may be used, or two or more alcohols may be used in combination. The alcohol is preferably ethanol. The concentration of the alcohol in the aqueous nucleic acid solution is preferably 0 to 50v/v%, more preferably 0 to 30v/v%, further preferably 0 to 25v/v%.

The aqueous nucleic acid solution is preferably an acidic buffer containing a nucleic acid. The pH of the acidic buffer is preferably 1 to 6.5, more preferably 3 to 6.5. Examples of the aforementioned acidic buffer are the same as those in Step a). The aforementioned acidic buffer is preferably an acetic acid/Na acetate buffer, a maleic acid/trishydroxymethylaminomethane (Tris)/NaOH buffer, a 2-morpholinoethanesulfonic acid (MES) buffer, a malic acid buffer, or a citrate buffer, more preferably an MES buffer. From the viewpoint of the stability of nucleic acids, the total concentration of the acid and a salt thereof in the aforementioned acidic buffer (i.e., buffer concentration) is preferably 10 to 100 mM, more preferably 10 to 80 mM.

The volume ratio of the suspension of the lipid nanoparticle free of nucleic acids to the nucleic acid solution in Step b) (volume of the suspension of the lipid nanoparticle free of nucleic acids:volume of the nucleic acid solution) is not particularly limited as long as it is a volume ratio that allows nucleic acid to be encapsulated. From the viewpoint of efficient encapsulation of nucleic acid, it is preferably 1:20 to 20:1, more preferably 1:12 to 12:1.

The concentration of nucleic acid in the suspension of the lipid nanoparticle encapsulating the nucleic acid obtained in Step b) (i.e., amount of nucleic acid (µg)/volume of suspension (mL)) is preferably 0.25 to 2700 µg/mL, more preferably 0.25 to 1000 µg/mL, further preferably 1 to 450 µg/mL. In a preferred embodiment of Step b) of the present invention, the lipid nanoparticle free of nucleic acids (e.g., a suspension of the lipid nanoparticle free of nucleic acids, or a lyophilized product of the lipid nanoparticle free of nucleic acids) is mixed with a nucleic acid solution in order to obtain a suspension of the aforementioned concentration.

To increase the efficiency of nucleic acid encapsulation, a mixture of the lipid nanoparticle free of nucleic acids (preferably, a suspension of the lipid nanoparticle free of nucleic acids) and the nucleic acid solution may be maintained at a predetermined temperature for a predetermined period of time. The aforementioned temperature is preferably 0 to 95°C, more preferably 0 to 60°C, further preferably 0 to 40°C, and the aforementioned time is preferably 1 min to 2 hr, more preferably 1 to 60 min, further preferably 1 to 30 min. The mixture in the aforementioned embodiment may also contain other components (e.g., ligand-unbound lipid).

### <Step c)>

In one embodiment of the production method of the present invention, Step c) is performed after Step b) and other steps that are performed as necessary. In Step c), a suspension of a lipid nanoparticle encapsulating the nucleic acid is obtained by mixing the suspension of the ligand-modified lipid nanoparticle encapsulating the nucleic acid and a ligand-bound lipid. The suspension of the lipid nanoparticle encapsulating the nucleic acid used in Step c) contains water as a dispersion medium, and the suspension of the ligand-modified lipid nanoparticle encapsulating the nucleic acid obtained in Step c) contains water.

The mixing in Step c) can be performed, for example, by pipetting or using a device including a microfluidic path or a mixer. Examples of the mixer include, but are not limited to, vortex mixer, T-junction mixer, and jet mixer.

Examples of the ligand include peptide (including proteins), antibody, nucleic acid (including oligonucleotide), aptamer, sugar, lipid, and the like. The ligand is preferably a peptide, antibody, or sugar, more preferably a peptide or antibody, further preferably an antibody, that has specific affinity for a molecule expressed on the surface of a target cell. Examples of the peptide, antibody, or sugar as a ligand include those described below.

Specific examples of the ligand include, transferrin, erythropoietin, EGF, VEGF, PDGF, IL-8, SDF1, thrombin, etanercept, romiplostim abatacept, HER2, transferrin receptor, glucose transporter, MDR1, Muromonab, Ibritumomab, Tositumomab, Rituximab, Basiliximab, Cetuximab, Brentuximab, Margetuximab, Daclizumab, Trastuzumab, Pertuzumab, Gemtuzumab, Alemtuzumab, Natalizumab, Tocilizumab, Mogamulizumab, Obinutuzumab, Vedolizumab, Pembrolimazab, Atezolizumab, Ocrelizumab, Inotuzumab, Benralizumab, Ibalizumab, Polatuzumab, Crizanlizumab, Sacituzumab, Inebilizumab, Tafasitamab, Satralizumab, Loncastuximab, Dostarlimab, Panitumumab, Ofatumumab, Ipirimumab, Ramsilmab, Nivolumab, Necitumumab, Daratumumab, Brodalumab, Olaratumab, Avelumab, Durvalumab, Dupilumab Sarilumab, Teprotunumab, Amivantamab, Anifrolumab, Tisotumab, Pavinafsp, PD-1, PD-L1, CTLA4, glutamine, leucine, isoleucine, valine, phenylalanine, tyrosine, tryptophan, methionine, histidine, Fc domain-binding peptide, RGD peptide, DNA aptamer, RNA aptamer, AS1411, A10, Sgc8, HeA21, HeA23, estrone, anisamide, methotrexate, adenosine, thiamine, riboflavin, biotin, glucose, mannose, lactose, maltose, N-acetylglucosamine, sucrose, galactose, Sialyl Lewis X, sialic acid, and hyaluronic acid.

The ligand is preferably a ligand having cell directivity. Here, the "ligand having cell directivity " refers to a ligand that has a higher affinity for one or more specific types of cells (including tissues and organs containing the cells) than for other cells. The ligand-modified lipid nanoparticle encapsulating a nucleic acid having cell directivity, obtained by the production method of the present invention, can be selectively delivered to specific cells.

Examples of the peptide having cell directivity include, but are not limited to, RGD peptides containing RGD motif (e.g., GRGDS pentapeptide) that has affinity for integrin αᵥβ₃, which is highly expressed in the vascular system and some cancer cells, laminin α2 chain-derived peptide having affinity for dystroglycan highly expressed in muscle cells, and the like. Alternatively, polypeptides that are natural ligands for cell surface receptors such as cytokine, hormone, and the like can also be mentioned.

Examples of the antibody having cell directivity include any antibodies against molecules expressed on the surface of target cells. Specific examples of the antibody include, but are not limited to, anti-transferrin receptor antibody, anti-glucose transporter antibody, anti-insulin receptor antibody, anti-LAT1 antibody, anti-MDR1 antibody, anti-BCRP antibody, anti-SMVT1 antibody, anti-Cluaudin 5 antibody, anti-LDL receptor antibody, anti-CD3 antibody, anti-CD4 antibody, anti-CD5 antibody, anti-CD7 antibody, anti-CD8 antibody, anti-CD19 antibody, anti-CD28 antibody, anti-CD147 antibody, and the like. Alternatively, antibodies used as existing antibody drugs or antibodies that recognize the same antigen as these can be used as ligands to target target cells expressing the antigen.

Antibodies used in the present invention may be complete antibody molecules, and, for example, any other antibody fragments such as single-stranded antibody (scFv), Fab, F(ab')₂, Fab', Fv, reduced antibody (rIgG), dsFv, sFv, diabody, triabody, and the like may be preferably used. In the present specification, unless otherwise specified, the "antibody" is used to include antibody fragments.

In addition, examples of the nucleic acid include ligands for Toll-like receptors, such as double-stranded RNA, CpG oligonucleotide, and the like, examples of the sugar include N-acetylgalactosamine having affinity for asialoglycoprotein receptor expressed on hepatocytes and the like, and examples of the lipid include various lipids (e.g., lysophosphatidylserine) which are natural ligands for G protein coupled receptor and the like.

The ligand is more preferably a ligand having brain cell directivity or a ligand having T cells directivity. Examples of the ligand having brain cell directivity include anti-transferrin receptor antibody, anti-glucose transporter antibody, anti-insulin receptor antibody, anti-LAT1 antibody, anti-CD147 antibody, anti-MDR1 antibody, anti-BCRP antibody, anti-SMVT1 antibody, anti-Cluaudin 5 antibody, and the like. Examples of the ligand having T cell directivity include anti-CD3 antibody, anti-CD5 antibody, anti-CD7 antibody, anti-CD28 antibody, anti-LDL receptor antibody, and the like.

From the viewpoint of achieving both the selectivity for target cells and preferred particle properties, the amount of the ligand in the lipid nanoparticle of interest is preferably 0.001 to 30 mol%, more preferably 0.001 to 10 mol%, and further preferably 0.01 to 5 mol%, with respect to the total amount of lipids in the lipid nanoparticles. It is preferable to mix the suspension of the lipid nanoparticle encapsulating the nucleic acids and a ligand-bound lipid so as to obtain such an amount of ligand.

In the present specification, the "ligand-bound lipid" refers to a lipid to which a ligand is covalently bound. The ligand-bound lipid is preferably a ligand-bound PEG lipid. In the present specification, the "ligand-bound PEG lipid" refers to a PEG lipid to which a ligand is covalently bound.

The production method of the ligand-bound PEG lipid is not particularly limited, and a ligand-bound PEG lipid can be produced by a known method. For example,
(i) a ligand-bound PEG lipid may be produced by reacting an activated PEG lipid (i.e., PEG lipid having a reactive group capable of binding to a ligand) as described in WO 2023/054243 with a ligand,
(ii) a ligand-bound PEG lipid may be produced by reacting a compound having a reactive group (e.g., dibenzocyclooctyne-N-hydroxysuccinimidyl ester) with a ligand to form a ligand having a reactive group, and then reacting the aforementioned PEG lipid having a group (e.g., azide group) capable of forming a covalent bond with the reactive group and the ligand. Of these, the latter production method (hereinafter abbreviated as "production method (ii)") is preferred.

The reaction between the compound having a reactive group and the ligand in production method (ii) is preferably performed in a solvent. Examples of the aforementioned solvent include water and buffer. The temperature of the aforementioned reaction is preferably 0 to 95°C, more preferably 0 to 40°C, further preferably 0 to 25°C and the time of the aforementioned reaction is preferably 10 min to 24 hr, more preferably 10 hr to 24 hr. The pH during the reaction is preferably 6 to 9, more preferably 7 to 9.

In production method (ii), the reaction between the aforementioned PEG lipid having a group capable of forming a covalent bond with the reactive group and the aforementioned ligand having the reactive group is preferably performed in a solvent. Examples of the aforementioned solvent include water and buffer. The temperature of the aforementioned reaction is preferably 0 to 95°C, more preferably 0 to 40°C, further preferably 0 to 25°C, and the time of the aforementioned reaction is preferably 1 to 24 hr, more preferably 10 to 24 hr, further preferably 16 to 20 hr.

The PEG moiety in the ligand-bound PEG lipid may have any molecular weight. In some embodiments, the number average molecular weight of the PEG moiety is preferably 200 to 10,000, more preferably 1,000 to 10,000. The PEG moiety may be linear or branched.

Examples of the PEG lipid in the ligand-bound PEG lipid include PEG-phospholipid (i.e., PEG-conjugated phospholipid), PEG-ceramide (i.e., PEG-conjugated ceramide), PEG-diacylglycerol (i.e., PEG-conjugated diacylglycerol), and PEG-cholesterol (i.e., PEG-conjugated cholesterol). Among these, PEG-phospholipid and PEG-diacylglycerol are preferred.

The PEG lipid in the ligand-bound PEG lipid is preferably PEG-phospholipid (i.e., PEG-conjugated phospholipid) or PEG-diacylglycerol (i.e., PEG-conjugated diacylglycerol), each having a number average molecular weight of the PEG moiety of 1,000 to 10,000, more preferably PEG-phospholipid having a number average molecular weight of the PEG moiety of 1,000 to 10,000, further preferably PEG-1,2-diacyl-sn-glycero-3-phosphoethanolamine (i.e., PEG-conjugated 1,2-diacyl-sn-glycero-3-phosphoethanolamine) having a number average molecular weight of the PEG moiety of 1,000 to 10,000, particularly preferably PEG-1,2-distearoyl-sn-glycero-3-phosphoethanolamine (i.e., PEG-conjugated 1,2-distearoyl-sn-glycero-3-phosphoethanolamine, for example, PEG-DSPE used in the below-mentioned Examples), having a number average molecular weight of the PEG moiety of 1,000 to 10,000.

The ligand-bound PEG lipid is preferably used in the form of an aqueous solution containing same. From the viewpoint of the efficiency of ligand insertion into lipid nanoparticles, the concentration of the ligand-bound PEG lipid in the aqueous solution is preferably 0.0001 to 0.12 mM, more preferably 0.001 to 0.06 mM.

Other components may be mixed during the mixing in Step c). Examples of other component include ligand-unbound lipid.

In Step c), the suspension of the lipid nanoparticle encapsulating the nucleic acid, a ligand-bound lipid, and a ligand-unbound lipid may be mixed. The order of mixing is not particularly limited. For example, (i) the suspension of the lipid nanoparticle encapsulating the nucleic acid, a ligand-bound lipid, and a ligand-unbound lipid may be mixed simultaneously, (ii) the suspension of the lipid nanoparticle encapsulating the nucleic acid and a ligand-bound lipid are first mixed, then the obtained mixture and a ligand-unbound lipid may be mixed, (iii) a ligand-bound lipid and a ligand-unbound lipid are mixed, and then the obtained mixture and the suspension of the lipid nanoparticle encapsulating the nucleic acid may be mixed, (iv) an aqueous solution or aqueous dispersion containing a ligand-bound lipid and a ligand-unbound lipid are first prepared, and then the obtained aqueous solution or aqueous dispersion and the suspension of the lipid nanoparticle encapsulating the nucleic acid may be mixed. Examples of the aqueous solution or aqueous dispersion in the aforementioned (iv) include an aqueous solution or aqueous dispersion (reaction mixture) obtained in the a synthesis reaction of a ligand-bound lipid, containing the ligand-bound lipid (product) and a ligand-unbound lipid (unreacted reactant).

When using a ligand-unbound lipid in Step c), in order to achieve both the preferred properties and target cell selectivity of the ligand-modified lipid nanoparticles, the amount of the ligand-unbound lipid used in Step c) is preferably 0.01 to 5.0 mol%, more preferably 0.1 to 3.0 mol%, with respect to the total lipids. The aforementioned "amount of the ligand-unbound lipid used in Step c)" does not include the amount of ligand-unbound lipid used in any step other than Step c') (e.g., ionic lipids used in Step a).

The "ligand-unbound lipid" used in Step c) is preferably a ligand-unbound PEG lipid. The description of the ligand-unbound PEG lipid is the same as the description of the PEG lipid in Step a).

As the ligand-unbound PEG lipid in Step c),
PEG-phospholipid (i.e., PEG-conjugated phospholipid) and PEG-diacylglycerol (i.e., PEG-conjugated diacylglycerol), each having a number average molecular weight of the PEG moiety of 1,000 to 10,000 are preferred,
PEG-phospholipid having a number average molecular weight of the PEG moiety of 1,000 to 10,000 is more preferred,
PEG-1,2-diacyl-sn-glycero-3-phosphoethanolamine (i.e., PEG-conjugated 1,2-diacyl-sn-glycero-3-phosphoethanolamine) having a number average molecular weight of the PEG moiety of 1,000 to 10,000 is further preferred,
PEG-1,2-distearoyl-sn-glycero-3-phosphoethanolamine (i.e., PEG-conjugated 1,2-distearoyl-sn-glycero-3-phosphoethanolamine, for example, PEG-DSPE used in the below-mentioned Examples) having a number average molecular weight of the PEG moiety of 1,000 to 10,000 is particularly preferred.

When using a ligand-unbound PEG lipid in Step c), in order to achieve both the preferred properties and target cell selectivity of the ligand-modified lipid nanoparticles, the amount of the ligand-unbound PEG lipid used in Step c) is preferably 0.01 to 5.0 mol%, more preferably 0.1 to 3.0 mol%, with respect to the total lipids. The aforementioned "amount of the ligand-unbound PEG lipid used in Step c)" does not include the amount of ligand-unbound PEG lipid used in any step other than Step c') (e.g., PEG lipids used in Step a).

When using a ligand-bound PEG lipid and a ligand-unbound PEG lipid in Step c), an aqueous solution containing these is preferably used. From the viewpoint of the efficiency of insertion into lipid nanoparticles, the concentration of the ligand-bound PEG lipid in the aqueous solution is preferably 0.0001 to 0.12 mM, more preferably 0.001 to 0.06 mM, and the concentration of the ligand-unbound PEG lipid in the aqueous solution is preferably 0.0001 to 0.12 mM, more preferably 0.001 to 0.06 mM.

For efficient modification of the lipid nanoparticle encapsulating the nucleic acid with a ligand-bound lipid, a mixture of the suspension of the lipid nanoparticle encapsulating the nucleic acid and a ligand-bound lipid may be maintained at a predetermined temperature for a predetermined period of time. The aforementioned maintenance temperature is preferably 0 to 95°C, more preferably 0 to 40°C, further preferably 0 to 25°C, and the aforementioned maintenance time is preferably 1 min to 5 hr, more preferably 1 to 2 hr, further preferably 1 to 60 min. The mixture in the aforementioned embodiment may also contain other components (e.g., ligand-unbound lipid).

### <Step c')>

In one embodiment of the production method of the present invention, Step c') is performed after Step a) and other steps that are performed as necessary. In Step c'), a suspension of a ligand-modified lipid nanoparticle free of nucleic acids is obtained by mixing the lipid nanoparticle free of nucleic acids, water, and a ligand-bound lipid. The suspension of the ligand-modified lipid nanoparticles free of nucleic acids obtained used in Step c') contains water as a dispersion medium.

Examples of the embodiment in which water and other components are mixed in Step c') include (i) an embodiment in which a suspension of the lipid nanoparticle free of nucleic acids (i.e., a suspension containing the lipid nanoparticle free of nucleic acids and water) is mixed with a ligand-bound lipid, (ii) an embodiment in which the lipid nanoparticle free of nucleic acids (e.g., a lyophilized product of the lipid nanoparticle free of nucleic acids), and an aqueous solution or aqueous dispersion containing a ligand-bound lipid (preferably an aqueous solution containing a ligand-bound lipid) are mixed, and (iii) an embodiment in which the lipid nanoparticle free of nucleic acids (e.g., a lyophilized product of the lipid nanoparticle free of nucleic acids), water, and a ligand-bound lipid are simultaneously mixed. Among these, the aforementioned embodiments (i) and (ii) are preferred, and the aforementioned embodiment (i) is more preferred,.

The mixing in Step c) can be performed, for example, by pipetting or using a device including a microfluidic path or a mixer. Examples of the mixer include, but are not limited to, vortex mixer, T-junction mixer, and jet mixer.

Other components may be mixed during the mixing in Step c). Examples of other component include ligand-unbound lipid.

In Step c'), the lipid nanoparticle free of nucleic acids, water, a ligand-bound lipid, and a ligand-unbound lipid may be mixed. The order of mixing is not particularly limited. For example, (i) the lipid nanoparticle free of nucleic acids, water, a ligand-bound lipid, and a ligand-unbound lipid may be mixed simultaneously, (ii) a suspension of the lipid nanoparticle free of nucleic acids (i.e., a suspension containing the lipid nanoparticle free of nucleic acids and water), a ligand-bound lipid, and a ligand-unbound lipid may be mixed simultaneously, (iii) a suspension of the lipid nanoparticle free of nucleic acids (i.e., a suspension containing the lipid nanoparticle free of nucleic acids and water) and a ligand-bound lipid are first mixed, and then the obtained mixture and a ligand-unbound lipid may be mixed, (iii) water and a ligand-bound lipid, and a ligand-unbound lipid are first mixed, and then the obtained aqueous solution or aqueous dispersion and the lipid nanoparticle free of nucleic acids may be mixed, (iv) an aqueous solution or aqueous dispersion containing a ligand-bound lipid and a ligand-unbound lipid are first prepared, then the obtained aqueous solution or aqueous dispersion and the lipid nanoparticle free of nucleic acids may be mixed. Examples of the aqueous solution or aqueous dispersion in the aforementioned (iv) include an aqueous solution or aqueous dispersion (reaction mixture) obtained in the a synthesis reaction of a ligand-bound lipid, containing the ligand-bound lipid (product) and a ligand-unbound lipid (unreacted reactant).

When using a ligand-unbound lipid in Step c'), in order to achieve both the preferred properties and target cell selectivity of the ligand-modified lipid nanoparticles, the amount of the ligand-unbound lipid used in Step c') is preferably 0.01 to 5.0 mol%, more preferably 0.1 to 3.0 mol%, with respect to the total lipids. The aforementioned "amount of the ligand-unbound lipid used in Step c')" does not include the amount of ligand-unbound lipid used in any step other than Step c') (e.g., ionic lipids used in Step a).

When using a ligand-unbound PEG lipid in Step c'), in order to achieve both the preferred properties and target cell selectivity of the ligand-modified lipid nanoparticles, the amount of the ligand-unbound PEG lipid used in Step c') is preferably 0.01 to 5.0 mol%, more preferably 0.1 to 3.0 mol%, with respect to the total lipids. The aforementioned "amount of the ligand-unbound PEG lipid used in Step c')" does not include the amount of ligand-unbound PEG lipid used in any step other than Step c') (e.g., PEG lipid used in Step a).

The descriptions of the ligand, ligand-bound lipid, and ligand-unbound lipid in Step c') are the same as the descriptions of these in Step c).

From the viewpoint of achieving both the selectivity for target cells and preferred particle properties, the amount of ligand in the lipid nanoparticle of interest is preferably 0.001 to 30 mol%, more preferably 0.001 to 10 mol%, and further preferably 0.01 to 5 mol%, with respect to the total amount of lipids in the lipid nanoparticles. It is preferable to mix the lipid nanoparticle free of nucleic acids, water, and a ligand-bound lipid so as to obtain such an amount of ligand.

For efficient modification of the lipid nanoparticle free of nucleic acids with a ligand-bound lipid, a mixture of the lipid nanoparticle free of nucleic acids, water, and a ligand-bound lipid (preferably a mixture of a ligand-bound lipid and a suspension of the lipid nanoparticle free of nucleic acids) may be maintained at a predetermined temperature for a predetermined period of time. The aforementioned maintenance temperature is preferably 0 to 95°C, more preferably 0 to 40°C, further preferably 0 to 25°C, and the aforementioned maintenance time is preferably 1 min to 5 hr, more preferably 1 to 2 hr, further preferably 1 to 60 min. The mixture in the aforementioned embodiment may also contain other components (e.g., ligand-unbound lipid).

### <Step b')>

In one embodiment of the production method of the present invention, Step b') is performed after Step c') and other steps that are performed as necessary. In Step b'), a suspension of a ligand-modified lipid nanoparticle encapsulating the nucleic acid is obtained by mixing the ligand-modified lipid nanoparticle free of nucleic acids and a nucleic acid solution. The nucleic acid solution used in Step b') contains water as a solvent, and the suspension of the ligand-modified lipid nanoparticle encapsulating the nucleic acid obtained in Step b') contains water as a dispersion medium.

Examples of the materials to be mixed with the nucleic acid solution in Step b') include (i) a suspension of the ligand-modified lipid nanoparticle free of nucleic acids, and (ii) a lyophilized product of the ligand-modified lipid nanoparticle free of nucleic acids obtained by lyophilizing the aforementioned suspension. In Step b'), a suspension of the ligand-modified lipid nanoparticle free of nucleic acids and a nucleic acid solution are preferably mixed. In a preferred embodiment of the present invention, the suspension of the ligand-modified lipid nanoparticle free of nucleic acids used in Step b') contains water as a dispersion medium.

The mixing in Step b') can be performed, for example, by pipetting or using a device including a microfluidic path or a mixer. Examples of the mixer include, but are not limited to, vortex mixer, T-junction mixer, and jet mixer.

Examples of nucleic acids include those described below. Preferably, the nucleic acid is mRNA. The description of the nucleic acid solution in Step b') (e.g., description of the concentration thereof, etc.) is the same as the description of the nucleic acid solution in Step b).

In Step b'), the molar ratio of the total amino groups of the ionic lipids in the ligand-modified lipid nanoparticle free of nucleic acids (e.g., a suspension of the ligand-modified lipid nanoparticle free of nucleic acids, or a lyophilized product of the ligand-modified lipid nanoparticle free of nucleic acids) to the total phosphate groups of the nucleic acid in the nucleic acid solution (total amount of amino groups of ionic lipids (mol)/total amount of phosphate groups of nucleic acid (mol)) (sometimes referred to as the "N/P ratio" in the present specification) is preferably 5 to 280, more preferably 10 to 140, and further preferably 15 to 70, from the viewpoint of nucleic acid encapsulation efficiency and toxicity.

The volume ratio of the suspension of the ligand-modified lipid nanoparticle free of nucleic acids to the nucleic acid solution in Step b') (volume of the suspension of the ligand-modified lipid nanoparticle free of nucleic acids:volume of the nucleic acid solution) is not particularly limited as long as it is a volume ratio that allows nucleic acid to be encapsulated. From the viewpoint of efficient encapsulation of nucleic acid, it is preferably 1:20 to 20:1, more preferably 1:12 to 12:1.

To increase the efficiency of nucleic acid encapsulation, a mixture of the ligand-modified lipid nanoparticle free of nucleic acids (preferably, a suspension of the ligand-modified lipid nanoparticle free of nucleic acids) and the nucleic acid solution may be maintained at a predetermined temperature for a predetermined period of time. The aforementioned temperature is preferably 0 to 95°C, more preferably 0 to 60°C, further preferably 0 to 40°C, and the aforementioned time is preferably 1 min to 2 hr, more preferably 1 to 60 min, further preferably 1 to 30 min. The mixture in the aforementioned embodiment may also contain other components (e.g., ligand-unbound lipid).

The concentration of nucleic acid in the suspension of the ligand-modified lipid nanoparticle encapsulating the nucleic acid obtained in Step b') (i.e., amount of nucleic acid (µg)/volume of suspension (mL)) is preferably 0.25 to 2700 µg/mL, more preferably 0.25 to 1000 µg/mL, further preferably 1 to 450 µg/mL. In a preferred embodiment of Step b') of the present invention, the ligand-modified lipid nanoparticle free of nucleic acids is mixed with a nucleic acid solution in order to achieve the aforementioned concentration.

### <Step d)>

In one embodiment of the production method of the present invention, Step d) is performed after Step a) and other steps that are performed as necessary. In Step d), a suspension of a ligand-modified lipid nanoparticle encapsulating the nucleic acid is obtained by mixing the lipid nanoparticle free of nucleic acids, a nucleic acid solution, and a ligand-bound lipid. Examples of the materials to be mixed with the nucleic acid solution and the ligand-bound lipid in Step d) include (i) a suspension of the lipid nanoparticle free of the nucleic acid, and (ii) a lyophilized product of the lipid nanoparticle free of nucleic acids obtained by lyophilizing the aforementioned suspension. In Step d), a suspension of the lipid nanoparticle free of nucleic acids, a nucleic acid solution, and a ligand-bound lipid are preferably mixed.

The mixing in Step d) can be performed, for example, by pipetting or using a device including a microfluidic path or a mixer. Examples of the mixer include, but are not limited to, vortex mixer, T-junction mixer, and jet mixer.

The order of mixing in Step d) is not particularly limited, and for example, (i) the lipid nanoparticle free of nucleic acids (preferably, a suspension of the lipid nanoparticle free of nucleic acids), a nucleic acid solution, and a ligand-bound lipid may be mixed simultaneously, (ii) the lipid nanoparticle free of nucleic acids (preferably, a suspension of the lipid nanoparticle free of nucleic acids) and a nucleic acid solution are first mixed, and then the obtained mixture and a ligand-bound lipid may be mixed simultaneously, (iii) a nucleic acid solution and a ligand-bound lipid are first mixed, and then the obtained solution containing the nucleic acid and a ligand-bound lipid and the lipid nanoparticle free of nucleic acids (preferably, a suspension of the lipid nanoparticle free of nucleic acids) may be mixed. Among the aforementioned embodiments, the aforementioned embodiment (iii) is preferred.

Other components may be mixed during the mixing in Step d). Examples of other component include ligand-unbound lipid.

In Step d), the lipid nanoparticle free of nucleic acids (preferably, a suspension of the lipid nanoparticle free of nucleic acids), a nucleic acid solution, a ligand-bound lipid, and a ligand-unbound lipid may be mixed. The order of mixing is not particularly limited. For example, (i) the lipid nanoparticle free of nucleic acids (preferably, a suspension of the lipid nanoparticle free of nucleic acids), a nucleic acid solution, a ligand-bound lipid, and a ligand-unbound lipid may be mixed simultaneously, (ii) an aqueous solution or aqueous dispersion containing a ligand-bound lipid and a ligand-unbound lipid are first prepared, and then the obtained aqueous solution or aqueous dispersion and a nucleic acid solution are mixed, and then the obtained mixture and the lipid nanoparticle free of nucleic acids (preferably, a suspension of the lipid nanoparticle free of nucleic acids) may be mixed. Examples of the aqueous solution or aqueous dispersion in the aforementioned (ii) include an aqueous solution or aqueous dispersion (reaction mixture) obtained in the a synthesis reaction of a ligand-bound lipid, containing the ligand-bound lipid (product) and a ligand-unbound lipid (unreacted reactant).

When using a ligand-unbound lipid in Step d), in order to achieve both the preferred properties and target cell selectivity of the ligand-modified lipid nanoparticles, the amount of the ligand-unbound lipid used in Step d) is preferably 0.01 to 5.0 mol%, more preferably 0.1 to 3.0 mol%, with respect to the total lipids. The aforementioned "amount of the ligand-unbound lipid used in Step d)" does not include the amount of ligand-unbound lipid used in any step other than Step d') (e.g., ionic lipids used in Step a).

When using a ligand-unbound PEG lipid in Step d), in order to achieve both the preferred properties and target cell selectivity of the ligand-modified lipid nanoparticles, the amount of the ligand-unbound PEG lipid used in Step d) is preferably 0.01 to 5.0 mol%, more preferably 0.1 to 3.0 mol%, with respect to the total lipids. The aforementioned "amount of the ligand-unbound PEG lipid used in Step d)" does not include the amount of ligand-unbound PEG lipid used in any step other than Step d) (e.g., PEG lipid used in Step a).

The descriptions of the ligand, ligand-bound lipid, and ligand-unbound lipid in Step d) are the same as the descriptions of these in Step c).

From the viewpoint of achieving both the selectivity for target cells and preferred particle properties, the amount of ligand in the lipid nanoparticle of interest is preferably 0.001 to 30 mol%, more preferably 0.001 to 10 mol%, and further preferably 0.01 to 5 mol%, with respect to the total amount of lipids in the lipid nanoparticles. It is preferable to mix the lipid nanoparticle free of nucleic acids, a nucleic acid solution, and a ligand-bound lipid so as to obtain such an amount of ligand.

Examples of nucleic acids include those described below. Preferably, the nucleic acid is mRNA. The description of the nucleic acid solution in Step d) (e.g., description of the concentration thereof, etc.) is the same as the description of the nucleic acid solution in Step b).

In Step d), the molar ratio of the total amino groups of the ionic lipids in the lipid nanoparticle free of nucleic acids (e.g., a suspension of the lipid nanoparticle free of nucleic acids, or a lyophilized product of the lipid nanoparticle free of nucleic acids) to the total phosphate groups of the nucleic acid in the nucleic acid solution (total amount of amino groups of ionic lipids (mol)/total amount of phosphate groups of nucleic acid (mol)) (sometimes referred to as the "N/P ratio" in the present specification) is preferably 5 to 280, more preferably 10 to 140, and further preferably 15 to 70, from the viewpoint of nucleic acid encapsulation efficiency and toxicity.

The volume ratio of the suspension of the lipid nanoparticle free of nucleic acids to the nucleic acid solution in Step d) (volume ratio of the suspension of the lipid nanoparticle free of nucleic acids:volume of the nucleic acid solution) is not particularly limited as long as it is a volume ratio that allows nucleic acid to be encapsulated. From the viewpoint of efficient encapsulation of nucleic acid, it is preferably 200:1 to 1:200, more preferably 20:1 to 1:20. The "nucleic acid solution" in the aforementioned volume ratio may contain components other than nucleic acid (e.g., ligand-bound lipid, ligand-unbound lipid).

The concentration of nucleic acid in the suspension of the ligand-modified lipid nanoparticle encapsulating the nucleic acid obtained in Step d) (i.e., amount of nucleic acid (µg)/volume of suspension (mL)) is preferably 0.25 to 2700 µg/mL, more preferably 0.25 to 1000 µg/mL, further preferably 1 to 450 µg/mL. In a preferred embodiment of Step d) of the present invention, the lipid nanoparticle free of nucleic acids, a nucleic acid solution, and a ligand-bound lipid are mixed to achieve the aforementioned concentration.

To increase the efficiency of nucleic acid encapsulation, a mixture of the lipid nanoparticle free of nucleic acids (preferably, a suspension of the lipid nanoparticles free of nucleic acids), the nucleic acid, and the ligand-bound lipid solution may be maintained at a predetermined temperature for a predetermined period of time. The aforementioned temperature is preferably 0 to 95°C, more preferably 0 to 60°C, further preferably 0 to 40°C, and the aforementioned time is preferably 1 min to 2 hr, more preferably 1 to 60 min, further preferably 1 to 30 min. The mixture in the aforementioned embodiment may also contain other components (e.g., ligand-unbound lipid).

### <Method for introducing nucleic acid into cell>

The present invention also provides
(i) a method for introducing a nucleic acid into a cell, including a step of contacting the ligand-modified lipid nanoparticle encapsulating the nucleic acid obtained by the method of the present invention and the cell in vitro, and
(ii) a method for introducing a nucleic acid into a target cell in a living body, comprising a step of administering the ligand-modified lipid nanoparticle encapsulating the nucleic acid obtained by the method of the present invention to the living body
(hereinafter these are sometimes collectively abbreviated as "the method of the present invention").

Any nucleic acid can be introduced into cells by the method of the present invention. The type of nucleic acid includes, but is not limited to, DNA, RNA, RNA chimeric nucleic acid, DNA/RNA hybrid, and the like. While any of single-stranded to triple-stranded nucleic acids can be used, single-stranded or double-strand one is preferred. The nucleic acid may also be other type of nucleotide that is N-glycoside of a purine or pyrimidine base, other oligomer having a non-nucleotide backbone (e.g., commercially available peptide nucleic acid (PNA) and the like), other oligomer containing a special bond (said oligomer containing a nucleotide having a configuration permitting base pairing or base attachment, which are found in DNA and RNA), or the like. Furthermore, the nucleic acid may also be, for example, a nucleic acid added with known modification, a nucleic acid with a label known in the pertinent field, a nucleic acid with a cap, a methylated nucleic acid, a nucleic acid in which one or more natural nucleotides substituted by an analog, a nucleic acid with an intramolecularly modified nucleotide, a nucleic acid with a non-charge bond (e.g., methyl sulfonate, phosphotriester, phosphoramidate, carbamate, etc.), a nucleic acid with a charged bond or sulfur-containing bond (e.g., phosphorothioate, phosphorodithioate, etc.), a nucleic acid with a side chain group such as protein (nuclease, nuclease inhibitor, toxin, antibody, signal peptide, poly-L-lysine, etc.), sugar (e.g., monosaccharide and the like), or the like, a nucleic acid containing an intercalating compound (e.g., acridine, psoralen, etc.), a nucleic acid containing a chelate compound (e.g., metal, radioactive metal, boron, oxidative metal, etc.), a nucleic acid containing an alkylating agent, a nucleic acid with a modified bond (e.g., α anomer-type nucleic acid and the like), or the like.

The type of the DNA that can be used in the present invention is not particularly limited, and can be selected as appropriate according to the purpose of use. Examples thereof include plasmid DNA, cDNA, antisense DNA, chromosomal DNA, PAC, BAC, CpG oligosaccharide, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples thereof include siRNA, miRNA, shRNA, antisense RNA, messenger RNA (mRNA), single-stranded RNA genome, double-stranded RNA genome, RNA replicon, transfer RNA, ribosomal RNA, and the like, with preference given to siRNA, miRNA, shRNA, mRNA, antisense RNA, and RNA replicon.

The nucleic acid used in the present invention is preferably purified by a method generally used by those of ordinary skill in the art.

The ligand-modified lipid nanoparticle encapsulating a nucleic acid, obtained by the production method of the present invention, can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

The particle size of the ligand-modified lipid nanoparticle encapsulating a nucleic acid is not particularly limited, and is preferably 10 nm to 500 nm, more preferably 30 nm to 300 nm. The particle size can be measured by using a particle size distribution measuring device such as Zetasizer Nano (Malvern) or the like.

The surface potential (zeta potential) of the ligand-modified lipid nanoparticle encapsulating a nucleic acid is not particularly limited and preferably -15 to +15 mV, more preferably -10 to +10 mV. In conventional gene transfer, particles electrically charged to have a plus surface potential have been mainly used. The particles electrically charged to have a plus surface potential are useful as a method for promoting electrostatic interactions with negatively-charged heparin sulfate on the cell surface to enhance uptake into cells. However, the positive surface charge may suppress release of nucleic acid from the carrier in the cell due to the interaction with a nucleic acid to be delivered, or protein synthesis due to the interaction between mRNA and a nucleic acid to be delivered. This problem can be solved by adjusting the surface charge to fall within the above-mentioned range. The surface charge can be measured using a zeta potential measuring device such as Zetasizer Nano and the like. The surface charge of the ligand-modified lipid nanoparticle encapsulating a nucleic acid can be adjusted by the composition thereof.

The step of contacting the ligand-modified lipid nanoparticle encapsulating a nucleic acid with a cell in vitro is specifically described below.

Cells are suspended in a suitable medium several days before contact with the ligand-modified lipid nanoparticle encapsulating a nucleic acid, and cultured under appropriate conditions. At the time of this contact, the cells may or may not be in a proliferative phase.

The culture medium at the time of the contact may be a serum-containing medium or a serum-free medium. The serum concentration of the medium is preferably 30 wt% or less, more preferably 20 wt% or less. When the medium contains excess proteins such as serum and the like, the contact between the ligand-modified lipid nanoparticle encapsulating a nucleic acid and the cell may be inhibited.

The cell density at the time of the contact is not particularly limited, and can be appropriately determined in consideration of the kind of the cell and the like. It is generally within the range of 1×10⁴ to 1×10⁷ cells/mL.

For example, a suspension of the above-mentioned ligand-modified lipid nanoparticle encapsulating a nucleic acid is added to the thus-prepared cells. The amount of the suspension to be added is not particularly limited, and can be appropriately determined in consideration of the cell number and the like. The concentration of the ligand-modified lipid nanoparticle encapsulating a nucleic acid when contacting cells is not particularly limited as long as the desired introduction of the nucleic acid into the cells can be achieved. The concentration of the total lipids is generally 0.0075 to 14 mM, preferably 0.05 to 10 mM, more preferably 0.075 to 7 mM, and the concentration of the nucleic acid is preferably 0.1 to 1000 µg/mL, more preferably 0.1 to 375 µg/mL, further preferably 4 to 170 µg/mL.

After the aforementioned suspension is added to cells, the cells are cultured. The temperature, humidity, CO₂ concentration, and the like during culturing are appropriately determined in consideration of the kind of the cell. When the cell is derived from a mammal, generally, the temperature is about 37°C, the humidity is about 95%, and the CO₂ concentration is about 5%. While the culture time can also be appropriately determined in consideration of the conditions such as the kind of the cell and the like, it is generally a range of 0.1 to 96 hr, preferably a range of 0.2 to 72 hr, more preferably a range of 0.5 to 48 hr. When the above-mentioned culture time is too short, the nucleic acid is not sufficiently introduced into the cells, and when the culture time is too long, the cells may become weak.

By the above-mentioned culture, a nucleic acid is introduced into cells. The culture is further continued preferably by exchanging the medium with a fresh medium, or adding a fresh medium to the medium. When the cell is derived from a mammal, the fresh medium preferably contains a serum or nutrition factor.

As mentioned above, a nucleic acid can be introduced into cells not only in vitro but also in vivo by using the ligand-modified lipid nanoparticle encapsulating a nucleic acid. That is, by administering the ligand-modified lipid nanoparticle encapsulating a nucleic acid to a subject, the lipid nanoparticles reach and contact the target cells, and the nucleic acid encapsulated in the lipid nanoparticle is delivered into the cells. The subject to which the lipid nanoparticles can be administered is not particularly limited and examples thereof include vertebrates such as mammals (e.g., human, monkey, mouse, rat, hamster, bovine, etc.), birds (e.g., chicken, ostrich, etc.), amphibia (e.g., frog and the like), fishes (e.g., zebrafish, rice-fish, etc.), and the like, invertebrates such as insects (e.g., silk moth, moth, Drosophila, etc.) and the like, plants, and the like. The subject of administration of the lipid nanoparticle encapsulating a nucleic acid is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue, lymph node, tumor, etc.) by using the ligand-modified lipid nanoparticle encapsulating a nucleic acid.

The administration method of the ligand-modified lipid nanoparticle encapsulating a nucleic acid to a subject (e.g., vertebrate, invertebrate, and the like) is not particularly limited as long as the lipid nanoparticle reaches and contacts with the target cells, and the compound introduced into the lipid nanoparticle can be introduced into the cell, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected in consideration of the kind of the compound to be introduced, the kind and the site of the target cell, and the like. The dose of the lipid nanoparticle is not particularly limited as long as the introduction of the compound into the cells can be achieved, and can be appropriately selected in consideration of the kind of the subject of administration, administration method, the kind of the compound to be introduced, the kind and the site of the target cell and the like.

When the ligand-modified lipid nanoparticle encapsulating a nucleic acid and obtained by the production method of the present invention is used as a nucleic acid-introducing agent, it can be formulated according to a conventional method.

When the nucleic acid-introducing agent is provided as a reagent for studies, the nucleic acid-introducing agent may be provided (i) as a ligand-modified lipid nanoparticle encapsulating a nucleic acid, or (ii) for example, as a mixture with water or another physiologically acceptable liquid (e.g., water-soluble solvent (e.g., malic acid buffer, etc.), organic solvent (e.g., ethanol, methanol, DMSO, tert-butanol, etc.), or a mixture of aqueous solvent and organic solvent, etc.). The nucleic acid-introducing agent of the present invention may appropriately contain physiologically acceptable additive (e.g., excipient, vehicle, preservative, stabilizer, binder, etc.), which are known per se.

Furthermore, when the nucleic acid-introducing agent is provided as a medicament, the nucleic acid-introducing agent can be provided as a ligand-modified lipid nanoparticle encapsulating a nucleic acid, or as a mixture with known pharmaceutically acceptable additives (e.g., carrier, flavor, excipient, vehicle, preservative, stabilizer, binder, etc.). The nucleic acid-introducing agent can be formulated as an oral preparation (e.g., tablet, capsule, etc.) or a parenteral preparation (more preferably injectable preparation). The nucleic acid-introducing agent can be formulated for adults as well as for children.

### <Method for producing pharmaceutical composition>

The ligand-modified lipid nanoparticle encapsulating a nucleic acid and obtained by the method of the present invention can be used as a drug delivery system for selectively delivering a nucleic acid and the like into a particular cell, and is useful for, for example, DNA vaccines by introducing antigen gene into dendritic cells, gene therapy drugs for tumor, nucleic acid pharmaceutical products that suppress expression of target genes by utilizing RNA interference, and the like. Therefore, the present invention also provides a production method of a pharmaceutical composition, including the production method of the present invention.

### [Example]

The present invention is described in more detail in the following by referring to Examples; however, the present invention is not limited to the following Examples and the like.

In the following Examples and the like, an ionic lipid represented by the formula (1) is shown by the name listed in the aforementioned Table. The abbreviations used in the following Examples and the like each mean the following.
ALC-0315: [(4-hydroxybutyl)azanediyl]bis(hexane-6,1-diyl)bis(2-hexyldecanoate) (manufactured by Jenkem Technology USA)
Azide-PEG-DSPE: 1,2-distearoyl-sn-glycero-3-phosphoethanolamine-N-[azido(polyethylene glycol)-2000] (manufactured by Avanti Polar Lipids)
cRGD: Cyclo[Arg-Gly-Asp-D-Phe-Lys(Cys)] (manufactured by PEPTIDE INSTITUTE, INC.)
DBCO-NHS: dibenzocyclooctyne-N-hydroxysuccinimidyl ester (manufactured by Merck-Millipore)
DSPE-PEG-Mannose: DSPE-PEG-Mannose, MW 2,000 (manufactured by Biopharma PEG Scientific)
DMG-PEG2000: 1,2-dimyristoyl-rac-glycero-3-methylpolyoxyethylene (number average molecular weight of PEG
chain: 2000) (manufactured by NOF CORPORATION)
DSPE-034GS: N-[N'-(succinimidyloxyglutaryl)aminopropylpolyoxyethyleneoxycarbonyl] -1,2-distearoyl-sn-glycero-3-phosphoethanolamine sodium salt (number average molecular weight of PEG chain: 3400) (manufactured by NOF CORPORATION)
DSPE-050GS: N-[N'-(succinimidyloxyglutaryl)aminopropylpolyoxyethyleneoxycarbonyl] -1,2-distearoyl-sn-glycero-3-phosphoethanolamine sodium salt (number average molecular weight of PEG chain: 5000) (manufactured by NOF CORPORATION)
EDTA: ethylenediaminetetraacetic acid
GS-020TS: 1,2-distearoyl-rac-glycero-3-(succinimidyloxycarbonyl)polyoxyethylene (number average molecular weight of PEG chain: 2000) (manufactured by NOF CORPORATION)
LNP: lipid nanoparticle
MC3: DLin-MC3-DMA (manufactured by Cayman Chemical)
Methoxy-PEG: Methyl-PEG2000-DSPE (manufactured by Tokyo Chemical Industry Co., Ltd.)
Nuclease free water: UltraPure^{TM} DNase/RNase-Free Distilled Water (manufactured by Thermo Fisher Scientific)
PBS: phosphate buffered saline
SM-102: heptadecan-9-yl 8-((2-hydroxyethyl) (6-oxo-6-(undecyloxy)hexyl)amino)octanoate (manufactured by Cayman Chemical)
Tris·HCl: tris(hydroxymethyl)aminomethane hydrochloride
Triton X-100: nonionic surfactant (manufactured by NACALAI TESQUE, INC.)

### [Preparation Example 1] Preparation of aqueous solution containing ligand-bound PEG lipid

Using Amicon Ultra-4 (MWCO: 50 kDa), the solvent of an anti-transferrin receptor antibody (manufactured by BioXCell) aqueous solution was substituted with a 200 mM NaHCO₃ aqueous solution (pH: 9.0). The antibody aqueous solution with the substituted buffer solvent was mixed with 2 equivalents of DBCO-NHS, and the obtained mixture was incubated at room temperature for 2 hr. The mixture was further incubated overnight at 4°C. Using Amicon Ultra-4 (3 kDa), the solvent of the mixture was substituted with ultrapure water to prepare a DBCO-anti-transferrin receptor antibody aqueous solution.

The 74 µM DBCO-anti-transferrin receptor antibody aqueous solution (10.1 µL) obtained as mentioned above, a 0.1 mM Azide-PEG-DSPE aqueous solution (7.47 µL), and a 2-morpholinoethanesulfonic acid buffer (pH: 6.0, 32.5 µL) were mixed, the obtained mixture was incubated overnight at 4°C to prepare an aqueous solution containing a ligand-bound PEG lipid (ligand: anti-transferrin receptor antibody).

### [Comparative Example 1] Preparation of LNP encapsulating mRNA but not modified with ligand

A 10 mM SS-OP ethanol solution, a 20 mM cholesterol ethanol solution, and a 10 mM 1,2-dioleoyl-sn-glycero-3-phosphocholine ethanol solution were mixed in a molar ratio of 52.5/40/7.5. The obtained mixture and a 1 mM DMG-PEG2000 ethanol solution were mixed to prepare a lipid solution (DMG-PEG2000 amount: 1.5 mol% with respect to the total of SS-OP, cholesterol, and 1,2-dioleoyl-sn-glycero-3-phosphocholine).

The concentration of each lipid and the concentration of the total lipids in the lipid solution obtained as described above were as follows.
SS-OP concentration: 4.2 mM
cholesterol concentration: 3.2 mM
1,2-dioleoyl-sn-glycero-3-phosphocholine concentration: 0.6 mM DMG-PEG2000 concentration: 0.12 mM
total lipids concentration: 8.12 mM

Using the nanoparticle production device "iLiNP" (manufactured by Lilac Pharma Co., Ltd.), a malic acid buffer (buffer concentration: 20 mM, pH: 3.0) was mixed at a flow rate of 875 µL/min and the lipid solution was mixed at a flow rate of 125 µL/min at 25°C for 2 min to obtain a suspension (2 mL) of an LNP free of nucleic acids (hereinafter referred to as "empty LNP suspension") (Step a)). A similar operation was performed to obtain 2 mL of an empty LNP suspension (Step a)).

To each of the two obtained empty LNP suspensions (2 mL) was quickly added 2.5 mL of a 2-morpholinoethanesulfonic acid buffer (buffer concentration: 20 mM, pH: 6.0) while stirring with a vortex mixer, and the obtained two 4.5 mL mixtures were combined. To 9.0 mL of the obtained mixture was added 4 mL of a 2-morpholinoethanesulfonic acid buffer (buffer concentration: 20 mM, pH: 6.0), and the obtained mixture was ultrafiltered using Amicon Ultra-15 (MWCO: 100 kDa). After concentration by ultrafiltration, the obtained concentrate was diluted with 14 mL of a 2-morpholinoethanesulfonic acid buffer (buffer concentration: 20 mM, pH: 6.0), and the obtained diluted product was ultrafiltered. To the obtained concentrate (100 µL) was added a 320 mg/mL sucrose solution (100 µL) to prepare an empty LNP suspension with the dispersion medium exchanged. The LNP suspension was stored at 4°C under an argon atmosphere until use.

An aqueous solution of mRNA encoding NanoLuc^{®} luciferase (2.5 µL, mRNA amount: 3 µg) was mixed with a 2-morpholinoethanesulfonic acid buffer (132.5 µL, buffer concentration: 20 mM, pH: 6.0) to prepare an mRNA solution. The entire amount of the obtained mRNA solution was mixed using a vortex mixer with 15 µL of the empty LNP suspension with the dispersion medium exchanged, and the obtained mixture was incubated at 37°C for 5 min. After incubation, a phosphate buffer (150 µL, buffer concentration: 9.6 mM, pH: 7.4) was added to the mixture to prepare an mRNA-encapsulating LNP suspension (N/P ratio: 36, mRNA concentration: 10 µg/mL) (Step b)).

Then, 7.47 µL of a 0.1 mM Azide-PEG-DSPE aqueous solution was mixed with 42.6 µL of a 2-morpholinoethanesulfonic acid buffer (buffer concentration: 20 mM, pH: 6.0), and the obtained solution was incubated overnight at 4°C. The entire amount of the incubated solution was mixed with the entire amount of the aforementioned mRNA-encapsulating LNP suspension. The obtained mixture was incubated at 4°C for 30 min to prepare an mRNA-encapsulating and Azide-PEG-DSPE-modified LNP suspension.

### [Comparative Example 2] Preparation of LNP encapsulating mRNA

A 10 mM SS-OP ethanol solution, a 10 mM cholesterol ethanol solution, and a 5 mM 1,2-dioleoyl-sn-glycero-3-phosphocholine ethanol solution were mixed in a molar ratio of 52.5/40/7.5. The obtained mixture, a 1 mM DMG-PEG2000 ethanol solution, and a 1 mM Azide-PEG-DSPE aqueous solution were mixed to prepare a lipid solution (DMG-PEG2000 amount: 1.5 mol% and Azide-PEG-DSPE amount: 0.8 mol%, each with respect to the total of SS-OP, cholesterol, and 1,2-dioleoyl-sn-glycero-3-phosphocholine).

The concentration of each lipid and the concentration of the total lipids in the lipid solution obtained as described above were as follows.
SS-OP concentration: 2.1 mM
cholesterol concentration: 1.6 mM
1,2-dioleoyl-sn-glycero-3-phosphocholine concentration: 0.3 mM
DMG-PEG2000 concentration: 0.06 mM
Azide-PEG-DSPE concentration: 0.0032 mM
total lipids concentration: 4.092 mM

Using the nanoparticle production device "iLiNP" (manufactured by Lilac Pharma Co., Ltd.), a malic acid buffer containing mRNA encoding luciferase (mRNA concentration: 0.167 mg/mL, buffer concentration: 20 mM, pH: 3.0) was mixed at a flow rate of 750 µL/min and the lipid solution was mixed at a flow rate of 250 µL/min for 1 min to prepare an mRNA-encapsulating LNP suspension (1 mL). A similar operation was performed to obtain an mRNA-encapsulating LNP suspension (1 mL).

To each of the two obtained mRNA-encapsulating LNP suspensions (1 mL) was quickly added 1.0 mL of a 2-morpholinoethanesulfonic acid buffer (buffer concentration: 20 mM, pH: 6.0) while stirring with a vortex mixer, and the obtained two 2.0 mL mixtures were combined. To 4.0 mL of the obtained mixture was added 4 mL of a 2-morpholinoethanesulfonic acid buffer (buffer concentration: 20 mM, pH: 5.5), and the mixture was concentrated to 0.2 mL or less using Amicon Ultra-4 (MWCO: 100 kDa). After concentration by ultrafiltration, the obtained concentrate was diluted with a phosphate buffer (4 mL, buffer concentration: 9.6 mM, pH: 7.4), and the obtained diluted product was ultrafiltered. Malic acid (malic acid concentration: 20 mM, sodium chloride concentration: 30 mM, pH: 5.0) was added to make the volume of the mixture 4.0 mL, and the obtained mixture was again concentrated to 200 µL by ultrafiltration to prepare an mRNA-encapsulating LNP suspension with the dispersion medium exchanged.

A DBCO-NHS aqueous solution was added to an F(ab')₂ fragment aqueous solution such that DBCO-NHS was 2 equivalents per F(ab')₂ fragment. The mixture was incubated at room temperature for 2 hr and then at 4°C overnight. Ultrapure water was added to make the volume of the mixture 0.5 mL, and the mixture was ultrafiltered using Amicon Ultra-0.5 to remove unreacted DBCO-NHS to prepare the DBCO-bound F(ab')₂ fragment. The number of DBCOs introduced into the DBCO-bound F(ab')₂ fragment was calculated from the absorbances of the DBCO-bound F(ab')₂ fragment aqueous solution at 280 nm and 309 nm. The DBCO-bound F(ab')₂ fragment was added to the aforementioned mRNA-encapsulating LNP suspension such that the DBCO-bound F(ab')₂ fragment was in an equimolar amount with respect to the azide group of the aforementioned LNP encapsulating mRNA. The mixture was then incubated overnight at 4°C to prepare an mRNA-encapsulating and ligand-modified LNP suspension (amount of ligand with respect to total lipids: 0.8 mol%).

### [Example 1] Preparation of ligand-modified LNP encapsulating mRNA

A 10 mM SS-OP ethanol solution, a 2 mM cholesterol ethanol solution, and a 10 mM 1,2-dioleoyl-sn-glycero-3-phosphocholine ethanol solution were mixed in a molar ratio of 52.5/40/7.5. The obtained mixture and a 1 mM DMG-PEG2000 ethanol solution were mixed to prepare a lipid solution (DMG-PEG2000 amount: 1.5 mol% with respect to the total of SS-OP, cholesterol, and 1,2-dioleoyl-sn-glycero-3-phosphocholine).

The concentration of each lipid and the concentration of the total lipids in the lipid solution obtained as described above were as follows.
SS-OP concentration: 4.2 mM
cholesterol concentration: 3.2 mM
1,2-dioleoyl-sn-glycero-3-phosphocholine concentration: 0.6 mM
DMG-PEG2000 concentration: 0.12 mM
total lipids concentration: 8.12 mM

Using the nanoparticle production device "iLiNP" (manufactured by Lilac Pharma Co., Ltd.), a malic acid buffer (buffer concentration: 20 mM, pH: 3.0) was mixed at a flow rate of 875 µL/min and the lipid solution was mixed at a flow rate of 125 µL/min at 25°C for 2 min to obtain 2 mL of an empty LNP suspension (Step a)). A similar operation was performed to obtain 2 mL of an empty LNP suspension (Step a)).

To each of the two obtained empty LNP suspensions (2 mL) was quickly added 2.5 mL of a 2-morpholinoethanesulfonic acid buffer (buffer concentration: 20 mM, pH: 6.0) while stirring with a vortex mixer, and the obtained two 4.5 mL mixtures were combined. To 9.0 mL of the obtained mixture was added 4 mL of a 2-morpholinoethanesulfonic acid buffer (buffer concentration: 20 mM, pH: 6.0), and the obtained mixture was ultrafiltered using Amicon Ultra-15 (MWCO: 100 kDa). After concentration by ultrafiltration, the obtained concentrate was diluted with 14 mL of a 2-morpholinoethanesulfonic acid buffer (buffer concentration: 20 mM, pH: 6.0), and the obtained diluted product was ultrafiltered. To the obtained concentrate (100 µL) was added a 320 mg/mL sucrose solution (100 µL) to prepare an empty LNP suspension with the dispersion medium exchanged. The LNP suspension was stored at 4°C under an argon atmosphere until use.

An aqueous solution of mRNA encoding luciferase (2.5 µL, mRNA amount: 3 µg) was mixed with a 2-morpholinoethanesulfonic acid buffer (132.5 µL, buffer concentration: 20 mM, pH: 6.0) to prepare an mRNA solution. The entire amount (135 µL) of the obtained mRNA solution was mixed using a vortex mixer with 15 µL of the empty LNP suspension with the dispersion medium exchanged, and the obtained mixture was incubated at 37°C for 5 min. After incubation, a phosphate buffer (150 µL, buffer concentration: 9.6 mM, pH: 7.4) was added to the mixture to prepare an mRNA-encapsulating LNP suspension (N/P ratio: 36, mRNA concentration: 10 µg/mL) (Step b)).

The mRNA-encapsulating LNP suspension (150 µL) and the entire amount of the aqueous solution containing the ligand-bound PEG lipid obtained in Preparation Example 1 were mixed by pipetting and incubated at 4°C for 30 min to prepare an mRNA-encapsulating and ligand-modified LNP suspension (amount of ligand with respect to total lipids: 0.8 mol% (Step c)).

In addition, mRNA-encapsulating and ligand-modified LNP suspensions were prepared in the same manner as above, except that the incubation temperature during preparation of the mRNA-encapsulating and ligand-modified LNP suspension (hereinafter referred to as the "incubation temperature during ligand modification") was changed to 37°C or 50°C. In this way, three suspensions were prepared, each with a different incubation temperature during ligand modification.

### [Experimental Example 1] Evaluation of LNP particle size, PdI, and mRNA encapsulation rate

The particle size, polydispersity Index (PdI), and mRNA encapsulation rate of the ligand-modified LNP encapsulating mRNA obtained in Example 1 or Comparative Example 2 were analyzed. The particle size and PdI were measured by the dynamic light scattering method using a Zetasizer (registered trademark). The mRNA encapsulation rate was measured by the Ribogreen (registered trademark) assay. The results are shown in the following Table 2. As LNP of Example 1, one having an incubation temperature during ligand modification of 4°C was used.

**[Table 2]**

| | ligand-modified | particle size (nm) | PdI | mRNA encapsulation rate (%) |
|---|---|---|---|---|
| Example 1 | present | 136 | 0.1805 | 79 |
| Comparative Example 2 | present | 120 | 0.22 | 22 |

As shown in Table 2, the ligand-modified LNP encapsulating mRNA of Example 1 showed a higher mRNA encapsulation rate compared to the ligand-modified LNP encapsulating mRNA of Comparative Example 2.

### [Comparative Example 3] Preparation of LNP encapsulating mRNA but not modified with ligand

An mRNA-encapsulating and Azide-PEG-DSPE-modified LNP suspension was prepared in the same manner as in Comparative Example 1 except that
(i) a 10 mM SS-OP ethanol solution, a 20 mM cholesterol ethanol solution, a 10 mM 1,2-dioleoyl-sn-glycero-3-phosphocholine ethanol solution, a 1 mM DMG-PEG2000 ethanol solution, and a 0.5 mM 1,1'-dioctadecyl-3,3,3',3'-tetramethylindocarbocyanine perchlorate (fluorescent dye) ethanol solution were mixed in a molar ratio of 52.5/40/7.5/1.5/0.5 to prepare a lipid solution, and
(ii) mRNA encoding luciferase was used instead of the mRNA encoding NanoLuc^{®} luciferase.

The concentration of each lipid and the concentration of the total lipids in the lipid solution obtained as described above were as follows.
SS-OP concentration: 4.2 mM
cholesterol concentration: 3.2 mM
1,2-dioleoyl-sn-glycero-3-phosphocholine concentration: 0.6 mM
DMG-PEG2000 concentration: 0.12 mM
total lipids concentration: 8.12 mM

Furthermore, mRNA-encapsulating and Azide-PEG-DSPE-modified LNP suspensions were prepared in the same manner as above except that the incubation temperature during preparation of the mRNA-encapsulating and Azide-PEG-DSPE-modified LNP suspension (hereinafter referred to as the "incubation temperature during Azide-PEG-DSPE modification") was changed to 37°C or 50°C. In this way, three suspensions were prepared, each with a different incubation temperature during Azide-PEG-DSPE modification.

### [Example 2] Preparation of ligand-modified LNP encapsulating mRNA

A 10 mM SS-OP ethanol solution, a 20 mM cholesterol ethanol solution, a 10 mM 1,2-dioleoyl-sn-glycero-3-phosphocholine ethanol solution, a 1 mM DMG-PEG2000 ethanol solution, and a 0.5 mM 1,1'-dioctadecyl-3,3,3',3'-tetramethylindocarbocyanine perchlorate (fluorescent dye) ethanol solution were mixed in a molar ratio of 52.5/40/7.5/1.5/0.5 to prepare a lipid solution. mRNA-encapsulating and ligand-modified LNP suspensions were prepared in the same manner as in Example 1 except that this lipid solution was used and that incubation during ligand modification was performed at 4°C, 37°C, or 50°C.

The concentration of each lipid and the concentration of the total lipids in the lipid solution obtained as described above were as follows.
SS-OP concentration: 4.2 mM
cholesterol concentration: 3.2 mM
1,2-dioleoyl-sn-glycero-3-phosphocholine concentration: 0.6 mM
DMG-PEG2000 concentration: 0.12 mM
total lipids concentration: 8.12 mM

### [Experimental Example 2] Evaluation of LNP particle size, PdI, mRNA encapsulation rate, etc.

The particle size, PdI, and mRNA encapsulation rate of the ligand-modified LNP encapsulating mRNA obtained in Example 2 were measured in the same manner as in Experimental Example 1. The results are shown in the following Table 3. The "temperature" in the following Table 3 is an "incubation temperature during ligand modification".

**[Table 3]**

| | temperature (°C) | particle size (nm) | PdI | mRNA encapsulation rate (%) |
|---|---|---|---|---|
| Example 2 | 4 | 153 | 0.14 | 86 |
| Example 2 | 37 | 157 | 0.13 | 83 |
| Example 2 | 50 | 162 | 0.12 | 74 |

### [Experimental Example 3] In vitro evaluation of LNP uptake into mouse breast cancer-derived 4T1 cells (FACS analysis)

Mouse breast cancer-derived 4T1 cells were seeded onto a 12-well plate at 7.5×10⁴ cells/well. After 24 hr, either the mRNA-encapsulating and ligand-modified LNP suspension obtained in Example 2 or the mRNA-encapsulating but ligand-unmodified LNP suspension obtained in Comparative Example 3 was added to the 12-well plate to achieve a lipid concentration of 10 µM per well. The mixture in the wells was incubated for 2 hr. After incubation, the mixture in the wells was washed twice with a phosphate buffer (pH 7.4) to remove LNP not incorporated into the 4T1 cells, and the 4T1 cells were then detached using trypsin. The 4T1 cells were centrifuged under the conditions of 500×g and 4°C for 5 min, and the obtained cell pellet was suspended in 1,000 µL of an FACS buffer (PBS containing 0.05 wt% sodium azide and 0.5 wt% fetal bovine serum albumin) to prepare a suspension. The obtained suspension was then centrifuged under the conditions of 500×g and 4°C for 5 min, and the obtained cell pellet was used as a sample. The fluorescence intensity of LNP within 4T1 cells was measured using NovoCyte. The results are shown in the following Table 4. The "temperature" in the following Table 4 is an "incubation temperature during ligand modification" in Example 2, and an "incubation temperature during Azide-PEG-DSPE modification" in Comparative Example 3.

**[Table 4]**

| | temperature (°C) | ligand-modification | fluorescence intensity |
|---|---|---|---|
| Example 2 | 4 | present | 58795 |
| Comparative Example 3 | 4 | absent | 5452 |
| Example 2 | 37 | present | 7817.5 |
| Comparative Example 3 | 37 | absent | 884.5 |
| Example 2 | 50 | present | 1336.5 |
| Comparative Example 3 | 50 | absent | 506.5 |

As shown in Table 4, regardless of the incubation temperature during ligand modification or azide-PEG-DSPE modification, the ligand-modified LNPs encapsulating mRNA of Example 2 exhibited improved fluorescence intensity compared to the LNPs encapsulating mRNA but not modified with ligand of Comparative Example 3, and a significant improvement in the amount of uptake into 4T1 cells was found.

### [Example 3]

In the same manner as in Example 1 except that mRNA encoding NanoLuc^{®} luciferase was used instead of the mRNA encoding luciferase, and the incubation temperature during ligand modification was set to 4°C, an mRNA-encapsulating and ligand-modified LNP suspension was prepared.

### [Experimental Example 4] Evaluation of LNP gene expression activity in vivo

The mRNA-encapsulating and ligand-modified LNP suspension of Example 3, or mRNA-encapsulating but ligand-unmodified LNP suspension of Comparative Example 1, or PBS was intravenously administered to C57BL/6 mice from the tail vein (RNA amount: 1.0 µg).

After 21 hr from the administration, the mice were euthanized, and the whole brains were removed. To the whole brain obtained was added 800 µL of a homogenization buffer (pH: 7.4, 100 mM Tris·HCl, 2 mM EDTA, 0.1 w/v% Triton X-100), and the brain was disrupted using zirconia beads. 500 µL of the supernatant of the obtained suspension was centrifuged at 13000 rpm at 4°C for 10 min, and 20 µL of the supernatant was mixed with 50 µL of an aqueous solution of luciferase substrate, and the luminescence derived from NanoLuc^{®} luciferase was measured using GloMax. In addition, the supernatant was diluted 100-fold with ultrapure water, and the protein concentration of the solution was measured using the BCA method, and the luminescence intensity was normalized by the protein amount. The results are shown in the following Table 5.

**[Table 5]**

| | luminescence intensity (×10⁴ RLU/mg protein) |
|---|---|
| Example 3 | 103 |
| Comparative Example 1 | 3 |
| PBS | 0 |

As shown in Table 5, the ligand-modified LNP encapsulating mRNA of Example 3 exhibited improved luminescence intensity compared to the LNP encapsulating mRNA but not modified with ligand of Comparative Example 1, and a significant improvement in gene expression activity was found in the mouse brain.

### [Example 4] Preparation of ligand-modified LNP encapsulating mRNA

In the same manner as in Example 1 except that a 10 mM SM-102 ethanol solution, a 10 mM MC3 ethanol solution, a 10 mM ALC-0315 ethanol solution, a 10 mM compound 3 ethanol solution, a 10 mM compound 4 ethanol solution, a 10 mM compound 5 ethanol solution, or a 10 mM compound 6 ethanol solution was used instead of the 10 mM SS-OP ethanol solution, an mRNA-encapsulating and ligand-modified LNP suspension was prepared.

### [Experimental Example 5] Evaluation of LNP particle size, PdI, and mRNA encapsulation rate

The particle size, PdI, and mRNA encapsulation rate of the ligand-modified LNPs encapsulating mRNA of Example 4 were measured in the same manner as in Experimental Example 1. The results are shown in the following Table 6.

**[Table 6]**

| ionic lipid used in Example 4 | particle size (nm) | PdI | mRNA encapsulation rate (%) |
|---|---|---|---|
| SM-102 | 162 | 0.45 | 91 |
| MC3 | 96 | 0.3 | 74 |
| ALC-0315 | 103 | 0.31 | 97 |
| compound 3 | 114 | 0.2 | 78 |
| compound 4 | 114 | 0.21 | 74 |
| compound 5 | 112 | 0.14 | 77 |
| compound 6 | 108 | 0.1 | 60 |

### [Preparation Example 2] Preparation of aqueous solution containing ligand-bound PEG lipid and ligand-unbound PEG lipid

Using Amicon Ultra-4 (MWCO: 50 kDa), the solvent of an anti-CD3 antibody (manufactured by Invitrogen) aqueous solution was substituted with a 100 mM NaHCO₃ aqueous solution (pH: 9.0). The antibody aqueous solution with the substituted buffer solvent was mixed with 2 equivalents of DBCO-NHS, and the obtained mixture was incubated at room temperature for 2 hr. The mixture was further incubated overnight at 4°C. Using Amicon Ultra-4 (3 kDa), the solvent of the mixture was substituted with ultrapure water to prepare a DBCO-anti-CD3 antibody aqueous solution.

The 12 µM DBCO-anti-CD3 antibody aqueous solution (20 µL) obtained as mentioned above, a 0.02 mM Azide-PEG-DSPE aqueous solution (12.0 µL), a 0.1 mM Methoxy-PEG aqueous solution (14.4 µL), and a 2-morpholinoethanesulfonic acid buffer (pH: 6.0) (103.6 µL) were mixed, and the obtained mixture was incubated overnight at 4°C to prepare an aqueous solution containing a ligand-bound PEG lipid (ligand: anti-CD3 antibody) and a ligand-unbound PEG lipid (Methoxy-PEG) (ligand-bound PEG lipid concentration: 0.0016 mM, ligand-unbound PEG lipid concentration: 0.0096 mM).

### [Example 5] Preparation of ligand-modified LNP encapsulating mRNA

A 10 mM SS-OP ethanol solution, a 10 mM cholesterol ethanol solution, and a 10 mM 1,2-dioleoyl-sn-glycero-3-phosphocholine ethanol solution were mixed in a molar ratio of 52.5/40/7.5. The obtained mixture and a 1 mM DMG-PEG2000 ethanol solution were mixed to prepare a lipid solution (DMG-PEG2000 amount: 1.0 mol% with respect to the total of SS-OP, cholesterol, and 1,2-dioleoyl-sn-glycero-3-phosphocholine).

The concentration of each lipid and the concentration of the total lipids in the lipid solution obtained as described above were as follows.
SS-OP concentration: 10.5 mM
cholesterol concentration: 1.5 mM
1,2-dioleoyl-sn-glycero-3-phosphocholine concentration: 8 mM
DMG-PEG2000 concentration: 0.3 mM
total lipids concentration: 20.3 mM

Using the nanoparticle production device "iLiNP" (manufactured by Lilac Pharma Co., Ltd.), a malic acid buffer (buffer concentration: 20 mM, pH: 3.0) was mixed at a flow rate of 875 µL/min and the lipid solution was mixed at a flow rate of 125 µL/min at 25°C for 2 min to obtain 2 mL of an empty LNP suspension (Step a)). A similar operation was performed to obtain 2 mL of an empty LNP suspension (Step a)).

To each of the two obtained empty LNP suspensions (2 mL) was quickly added 2.5 mL of a 2-morpholinoethanesulfonic acid buffer (buffer concentration: 20 mM, pH: 6.0) while stirring with a vortex mixer, and the obtained two 4.5 mL mixtures were combined. To 9.0 mL of the obtained mixture was added 4 mL of a 2-morpholinoethanesulfonic acid buffer (buffer concentration: 20 mM, pH: 6.0), and the obtained mixture was ultrafiltered using Amicon Ultra-15 (MWCO: 100 kDa). After concentration by ultrafiltration, the obtained concentrate was diluted with 14 mL of a 2-morpholinoethanesulfonic acid buffer (buffer concentration: 20 mM, pH: 6.0), and the obtained diluted product was ultrafiltered. To the obtained concentrate (15 µL, lipid amount: 300 nmol) was added 60 µL of a sucrose solution dissolved in a 2-morpholinoethanesulfonic acid buffer (buffer concentration: 20 mM, pH: 6.0) to 320 mg/mL to prepare an empty LNP suspension with the dispersion medium exchanged. The LNP suspension was stored frozen at -20°C under an argon atmosphere and thawed at room temperature before use.

An aqueous solution of mRNA encoding luciferase (mRNA amount: 2.5 µg) was mixed with a 2-morpholinoethanesulfonic acid buffer (75 µL, buffer concentration: 40 mM, pH: 6.0) to prepare an mRNA solution. The entire amount (75 µL) of the obtained mRNA solution was mixed using a vortex mixer with 75 µL of the empty LNP suspension with the dispersion medium exchanged, and the obtained mixture was incubated at 75°C for 5 min. After incubation, a phosphate buffer (150 µL, buffer concentration: 9.6 mM, pH 7.4) was added to the mixture to prepare an mRNA-encapsulating LNP suspension (N/P ratio: 36, mRNA concentration: 13.3 µg/mL) (Step b)).

The mRNA-encapsulating LNP suspension (150 µL) and an aqueous solution (150 µL) containing the ligand-bound PEG lipid and the ligand-unbound PEG lipid obtained by the method described in Preparation Example 2 were mixed using a vortex mixer and incubated at 4°C for 30 min. A phosphate buffer (300 µL, buffer concentration: 9.6 mM, pH 7.4) was then added to prepare an mRNA-encapsulating and ligand-modified LNP suspension (amount of ligand with respect to total lipids: 0.08 mol%, amount of ligand-unbound PEG lipid with respect to total lipids: 0.48 mol%) (Step c)).

### [Comparative Example 4] Preparation of LNP encapsulating mRNA but not modified with ligand

In the same manner as in Example 5, Step a), exchange of dispersion medium, cryopreservation, thawing, and Step b) were performed to prepare an mRNA-encapsulating LNP suspension. The mRNA-encapsulating LNP suspension (150 µL) and a 2-morpholinoethanesulfonic acid buffer (150 µL, buffer concentration: 20 mM, pH: 6.0) were mixed using a vortex mixer, and a phosphate buffer (300 µL, buffer concentration: 9.6 mM, pH 7.4) was added to prepare an mRNA-encapsulating LNP suspension (N/P ratio: 36).

### [Experimental Example 6] Evaluation of LNP particle size, PdI, and mRNA encapsulation rate

The particle size, PdI, and mRNA encapsulation rate of the ligand-modified LNP encapsulating mRNA of Example 5, and the LNP encapsulating mRNA but not modified with ligand of Comparative Example 4 were measured in the same manner as in Experimental Example 1. The results are shown in the following Table 7.

**[Table 7]**

| | ligand-modification | particle size (nm) | PdI | mRNA encapsulation rate (%) |
|---|---|---|---|---|
| Example 5 | present | 136 | 0.1 | 89 |
| Comparative Example 4 | absent | 136 | 0.1 | 91 |

As shown in Table 7, even though the ligand-modified LNP encapsulating mRNA of Example 5 was ligand-modified, it showed a favorable mRNA encapsulation rate similar to the mRNA-encapsulating LNP suspension of Comparative Example 4.

### [Preparation Example 3] Preparation of lyophilize LNP

A 10 mM SS-OP ethanol solution, a 10 mM cholesterol ethanol solution, and a 10 mM 1,2-dioleoyl-sn-glycero-3-phosphocholine ethanol solution were mixed in a molar ratio of 52.5/40/7.5. The obtained mixture and a 1 mM DMG-PEG2000 ethanol solution were mixed in a molar ratio of 52.5/40/7.5/1.5 to prepare a lipid solution.

The concentration of each lipid and the concentration of the total lipids in the lipid solution obtained as described above were as follows.
SS-OP concentration: 4.2 mM
cholesterol concentration: 3.2 mM
1,2-dioleoyl-sn-glycero-3-phosphocholine concentration: 0.6 mM
DMG-PEG2000 concentration: 0.12 mM
total lipids concentration: 8.12 mM

Using the nanoparticle production device "NanoAssemblr Ignite" (manufactured by Precision NanoSystems), a malic acid buffer (buffer concentration: 20 mM, pH: 3.0) was mixed at a flow rate of 875 µL/min and the lipid solution was mixed at a flow rate of 125 µL/min at 25°C for 2 min to obtain 2 mL of an empty LNP suspension (Step a)). A similar operation was performed to obtain 2 mL of an empty LNP suspension (Step a)).

To each of the two obtained empty LNP suspensions (2 mL) was quickly added 2.5 mL of a 2-morpholinoethanesulfonic acid buffer (buffer concentration: 20 mM, pH: 6.0) while stirring with a vortex mixer, and the obtained two 4.5 mL mixtures were combined. To 9.0 mL of the obtained mixture was added 4 mL of a 2-morpholinoethanesulfonic acid buffer (buffer concentration: 20 mM, pH: 6.0), and the obtained mixture was ultrafiltered using Amicon Ultra-15 (MWCO: 100 kDa). After concentration by ultrafiltration, the obtained concentrate was diluted with 14 mL of a 2-morpholinoethanesulfonic acid buffer (buffer concentration: 20 mM, pH: 6.0), and the obtained diluted product was ultrafiltered. To the obtained concentrate (10 µL, lipid amount: 400 nmol) was added 100 µL of a sucrose solution dissolved in a 2-morpholinoethanesulfonic acid buffer (buffer concentration: 20 mM, pH: 6.0) to 320 mg/mL to prepare an empty LNP suspension with the dispersion medium exchanged.

For lyophilization, the empty LNP suspension with the dispersion medium exchanged was first frozen by cooling the suspension at normal pressure until the temperature thereof reached -40°C. The pressure was then reduced to 200 mTorr, and the temperature was then increased as described below. Specifically, the frozen product was left standing at -40°C for 1 hr under 200 mTorr pressure, followed by temperature rising, then left standing at -30°C for 6 hr, followed by temperature rising, then left standing at -20°C for 6 hr, followed by temperature rising, then left standing at -10°C for 6 hr, followed by temperature rising, then left standing at 0°C for 6 hr, followed by temperature rising, then left standing at 10°C for 3 hr, followed by temperature rising, then left standing at 20°C for 3 hr, followed by temperature rising, then maintaining at 30°C for 3 hr, and then the pressure was returned to normal pressure and the lyophilized LNP was recovered.

The obtained lyophilize LNP was stored at -80°C under an argon atmosphere until use.

### [Example 6] Preparation of ligand-modified LNP encapsulating mRNA

An aqueous solution of mRNA encoding NanoLuc^{®} luciferase (mRNA amount: 2.0 µg) was mixed with Nuclease free water (200 µL) to prepare an mRNA solution. The entire amount (200 µL) of the obtained mRNA solution and the lyophilize LNP obtained in Preparation Example 3 were mixed by pipetting and the obtained mixture was incubated at 37°C or 75°C for 5 min. After incubation, a phosphate buffer (200 µL, buffer concentration: 9.6 mM, pH: 7.4) was added to the mixture to prepare an mRNA-encapsulating LNP suspension (N/P ratio: 69, mRNA concentration: 5 µg/mL) (Step b)).

The mRNA-encapsulating LNP suspension (100 µL) and the entire amount of an aqueous solution containing the ligand-bound PEG lipid and the ligand-unbound PEG lipid obtained by the method described in Preparation Example 2 were mixed by pipetting and incubated at 4°C for 30 min. To the mixture after incubation was added a phosphate buffer (200 µL, buffer concentration: 9.6 mM, pH 7.4) to prepare an mRNA-encapsulating and ligand-modified LNP suspension (ligand amount with respect to total lipids: 0.08 mol%) (Step c)).

### [Example 7] Preparation of ligand-modified LNP encapsulating mRNA

The lyophilized LNP obtained in Preparation Example 3 and the entire amount of an aqueous solution containing the ligand-bound PEG lipid and the ligand-unbound PEG lipid obtained by the method described in Preparation Example 2 were mixed by pipetting and incubated at 4°C for 30 min to prepare a suspension of a ligand-modified LNP free of mRNA (ligand amount with respect to total lipids : 0.8 mol%) (Step c')).

An aqueous solution of mRNA encoding NanoLuc^{®} luciferase (mRNA amount: 2.5 µg) was mixed with Nuclease free water (200 µL) to prepare an mRNA solution. The entire amount (200 µL) of the obtained mRNA solution and the suspension (100 µL) of the ligand-modified LNP free of mRNA were mixed using a vortex mixer, and the obtained mixture was incubated at 75°C for 5 min. After incubation, a phosphate buffer (200 µL, buffer concentration: 9.6 mM, pH: 7.4) was added to the mixture to prepare an mRNA-encapsulating and ligand-modified LNP suspension (N/P ratio: 69, mRNA concentration: 4 µg/mL) (Step b')).

### [Experimental Example 7] Evaluation of LNP particle size, PdI, and mRNA encapsulation rate

The particle size, PdI, and mRNA encapsulation rate of the ligand-modified LNPs encapsulating mRNA of Examples 6 and 7 were measured in the same manner as in Experimental Example 1. The results are shown in the following Table 8. The "temperature" in the following Table 8 is an "incubation temperature during nucleic acid encapsulation".

**[Table 8]**

| | temperature (°C) | particle size (nm) | PdI | mRNA encapsulation rate (%) |
|---|---|---|---|---|
| Example 6 | 37 | 220 | 0.12 | 62 |
| Example 6 | 75 | 222 | 0.06 | 93 |
| Example 7 | 75 | 226 | 0.15 | 86 |

As shown in Table 8, the ligand-modified LNPs encapsulating mRNA of Examples 6 and 7 both showed a favorable mRNA encapsulation rate.

### [Preparation Example 4] Preparation of aqueous solution containing ligand-bound PEG lipid

In the same manner as in Preparation Example 1 except that the ligand to be used was changed to cRGD, and the lipid to be used was changed to GS-020TS, DSPE-034GS, or DSPE-050GS, an aqueous solution containing a ligand-bound PEG lipid was prepared. The ligand-bound PEG lipids obtained using GS-020TS, DSPE-034GS, and DSPE-050GS are hereinafter referred to as "DSPE-034-RGD", "DSPE-050-RGD", and "GS-020-RGD", respectively.

### [Preparation Example 5] Preparation of empty LNP suspension

A 10 mM SS-OP ethanol solution, a 2 mM cholesterol ethanol solution, and a 10 mM 1,2-dioleoyl-sn-glycero-3-phosphocholine ethanol solution were mixed in a molar ratio of 52.5/40/7.5. The obtained mixture and a 1 mM DMG-PEG2000 ethanol solution were mixed to prepare a lipid solution (DMG-PEG2000 amount: 1.5 mol% with respect to the total of SS-OP, cholesterol, and 1,2-dioleoyl-sn-glycero-3-phosphocholine).

The concentration of each lipid and the concentration of the total lipids in the lipid solution obtained as described above were as follows.
SS-OP concentration: 10.5 mM
cholesterol concentration: 1.5 mM
1,2-dioleoyl-sn-glycero-3-phosphocholine concentration: 8 mM
DMG-PEG2000 concentration: 0.3 mM
total lipids concentration: 20.3 mM

Using the nanoparticle production device "iLiNP" (manufactured by Lilac Pharma Co., Ltd.), a malic acid buffer (buffer concentration: 20 mM, pH: 3.0) was mixed at a flow rate of 875 µL/min and the lipid solution was mixed at a flow rate of 125 µL/min at 25°C for 2 min to obtain 2 mL of an empty LNP suspension (Step a)). A similar operation was performed to obtain 2 mL of an empty LNP suspension (Step a)).

To each of the two obtained empty LNP suspensions (2 mL) was quickly added 2.5 mL of a 2-morpholinoethanesulfonic acid buffer (buffer concentration: 20 mM, pH: 6.0) while stirring with a vortex mixer, and the obtained two 4.5 mL mixtures were combined. To 9.0 mL of the obtained mixture was added 4 mL of a 2-morpholinoethanesulfonic acid buffer (buffer concentration: 20 mM, pH: 6.0), and the obtained mixture was ultrafiltered using Amicon Ultra-15 (MWCO: 100 kDa). After concentration by ultrafiltration, the obtained concentrate was diluted with 14 mL of a 2-morpholinoethanesulfonic acid buffer (buffer concentration: 20 mM, pH: 6.0), and the obtained diluted product was ultrafiltered. To the obtained concentrate (100 µL) was added a 320 mg/mL sucrose solution (100 µL) to prepare an empty LNP suspension with the dispersion medium exchanged. The LNP suspension was stored at 4°C under an argon atmosphere until use.

### [Example 8] Preparation of ligand-modified LNP encapsulating mRNA

An mRNA-encapsulating LNP suspension was prepared in the same manner as in Example 5, except that the empty LNP suspension with the dispersion medium exchanged obtained in Preparation Example 5 was used and that incubation during nucleic acid encapsulation was performed at 37°C (N/P ratio: 36, mRNA concentration: 8.3 µg/mL) (Step b).

The mRNA-encapsulating LNP suspension (75 µL) and the aqueous solution containing the ligand-bound PEG lipid (i.e., DSPE-034-RGD, DSPE-050-RGD, or GS-020-RGD)) obtained in Preparation Example 4, or an aqueous solution (75 µL) containing 0.032 mg/mL DSPE-PEG-Mannose were mixed by pipetting and incubated at 4°C for 30 min to prepare an mRNA-encapsulating and ligand-modified LNP suspension (ligand amount with respect to total lipids: 0.8 mol%) (Step c)).

### [Example 9] Preparation of ligand-modified LNP encapsulating mRNA

The empty LNP suspension (15 µL) with the dispersion medium exchanged obtained in Preparation Example 5 and the aqueous solution (150 µL) containing DSPE-034-RGD or obtained in Preparation Example 4 were mixed by pipetting and incubated at 4°C for 30 min to prepare a suspension of a ligand-modified LNP free of mRNA (ligand amount with respect to total lipids: 0.8 mol%) (Step c')).

An aqueous solution of mRNA encoding luciferase (mRNA amount: 3 µg) was mixed with a 2-morpholinoethanesulfonic acid buffer (132 µL, buffer concentration: 20 mM, pH: 6.0) to prepare an mRNA solution. The entire amount (135 µL) of the obtained mRNA solution and the suspension (165 µL) of the ligand-modified LNP free of mRNA were mixed using a vortex mixer, and the obtained mixture was incubated at 37°C or 75°C for 5 min. After incubation, a phosphate buffer (300 µL, buffer concentration: 9.6 mM, pH: 7.4) was added to the mixture to prepare an mRNA-encapsulating LNP suspension (N/P ratio: 69, mRNA concentration: 15 µg/mL) (Step b')).

### [Example 10] Preparation of ligand-modified LNP encapsulating mRNA

The aqueous solution containing the ligand-bound PEG lipid (i.e., DSPE-034-RGD, DSPE-050-RGD, or GS-020-RGD) obtained in Preparation Example 4 or an aqueous solution containing DSPE-PEG-Mannose (150 µL), and an mRNA encoding luciferase solution (RNA amount: 3 µg, 2-morpholinoethanesulfonic acid buffer: 132 µL, buffer concentration: 20 mM, pH: 6.0) were mixed by pipetting to obtain an aqueous solution containing ligand-bound PEG lipid and mRNA.

The entire amount (285 µL) of the obtained aqueous solution containing the ligand PEG lipid and mRNA and the empty LNP suspension (15 µL) with the dispersion medium exchanged obtained in Preparation Example 5 were mixed using a vortex mixer, and the obtained mixture was incubated at 37°C for 5 min, after which incubated at 4°C for 30 min. After incubation, a phosphate buffer (300 µL, buffer concentration: 9.6 mM, pH: 7.4) was added to the mixture to prepare an mRNA-encapsulating and ligand-modified LNP suspension (N/P ratio: 69) (Step d)).

### [Experimental Example 8] Evaluation of LNP particle size, PdI, and mRNA encapsulation rate

The particle size, PdI, and mRNA encapsulation rate of the ligand-modified LNPs encapsulating mRNA of Examples 8 to 10 were measured in the same manner as in Experimental Example 1. The results are shown in the following Table 9. The following Table 9 also shows the ligand-bound PEG lipids used in Examples 8 to 10.

**[Table 9]**

| ligand-bound PEG lipid used | | particle size (nm) | PdI | mRNA encapsulation rate (%) |
|---|---|---|---|---|
| DSPE-034-RGD | Example 8 | 136 | 0.12 | 87 |
| | Example 9 | 127 | 0.18 | 65 |
| | Example 10 | 129 | 0.12 | 92 |
| DSPE-050-RGD | Example 8 | 139 | 0.11 | 89 |
| | Example 10 | 136 | 0.09 | 82 |
| GS-020-RGD | Example 8 | 132 | 0.12 | 100 |
| | Example 10 | 133 | 0.12 | 87 |
| DSPE-PEG-Mannose | Example 8 | 140 | 0.1 | 100 |
| | Example 9 | 133 | 0.17 | 75 |
| | Example 10 | 140 | 0.19 | 96 |

As shown in Table 9, the ligand-modified LNPs encapsulating mRNA of Examples 8 to 10 showed favorable mRNA encapsulation rates regardless of the type of ligand PEG-bound lipid.

### [Example 11] Preparation of ligand-modified LNP encapsulating mRNA

A 10 mM SS-OP ethanol solution, a 20 mM cholesterol ethanol solution, a 10 mM 1,2-dioleoyl-sn-glycero-3-phosphocholine ethanol solution, and a 1 mM DMG-PEG2000 ethanol solution were mixed in a molar ratio of 52.5/40/7.5/1.5 to prepare a lipid solution. mRNA-encapsulating and ligand-modified LNP suspensions were prepared in the same manner as in Example 1 except that this lipid solution was used, the incubation temperature during nucleic acid encapsulation was set to 4°C, 37°C, 60°C, or 95°C, and the incubation time during nucleic acid encapsulation was set to 1 min, 5 min, 30 min, 60 min, or 120 min.

The concentration of each lipid and the concentration of the total lipids in the lipid solution obtained as described above were as follows.
SS-OP concentration: 10.5 mM
cholesterol concentration: 1.5 mM
1,2-dioleoyl-sn-glycero-3-phosphocholine concentration: 8 mM
DMG-PEG2000 concentration: 0.3 mM
total lipids concentration: 20.3 mM

### [Experimental Example 9] Evaluation of LNP particle size, PdI, mRNA encapsulation rate, etc.

The particle size, PdI, and mRNA encapsulation rate of the ligand-modified LNP encapsulating mRNA of Example 11 were measured in the same manner as in Experimental Example 1. The results are shown in the following Table 10. The "temperature" in the following Table 10 is an "incubation temperature during nucleic acid encapsulation", and the "time" is an "incubation time during nucleic acid encapsulation".

**[Table 10]**

| temperature (°C) | time (min) | mRNA encapsulation rate (%) |
|---|---|---|
| 4 | 1 | 85 |
| | 5 | 77 |
| | 30 | 76 |
| | 60 | 78 |
| | 120 | 74 |
| 37 | 1 | 86 |
| | 5 | 79 |
| | 30 | 79 |
| | 60 | 82 |
| | 120 | 81 |
| 60 | 1 | 88 |
| | 5 | 84 |
| | 30 | 83 |
| | 60 | 81 |
| | 120 | 80 |
| 95 | 1 | 91 |
| | 5 | 77 |

### [Example 12] Preparation of ligand-modified LNP encapsulating mRNA

A 10 mM SS-OP ethanol solution, a 20 mM cholesterol ethanol solution, a 10 mM 1,2-dioleoyl-sn-glycero-3-phosphocholine ethanol solution, and a 1 mM DMG-PEG2000 ethanol solution were mixed in a molar ratio of 52.5/40/7.5/1.5 to prepare a lipid solution. mRNA-encapsulating and ligand-modified LNP suspensions were prepared in the same manner as in Example 1 except that this lipid solution was used, and the incubation during ligand modification in Step c) was performed at the temperature and time shown in the following Table 11.

The concentration of each lipid and the concentration of the total lipids in the lipid solution obtained as described above were as follows.
SS-OP concentration: 10.5 mM
cholesterol concentration: 1.5 mM
1,2-dioleoyl-sn-glycero-3-phosphocholine concentration: 8 mM
DMG-PEG2000 concentration: 0.3 mM
total lipids concentration: 20.3 mM

### [Experimental Example 10] Evaluation of LNP particle size, PdI, mRNA encapsulation rate, etc.

The particle size, PdI, and mRNA encapsulation rate of the ligand-modified LNP encapsulating mRNA of Example 12 were measured in the same manner as in Experimental Example 1. The results are shown in the following Table 11. The "temperature" in the following Table 11 is an "incubation temperature during ligand modification", and the "time" is an "incubation time during ligand modification".

**[Table 11]**

| temperature (°C) | time (min) | mRNA encapsulation rate (%) |
|---|---|---|
| 4 | 1 | 93 |
| | 5 | 91 |
| | 30 | 84 |
| | 60 | 82 |
| | 180 | 82 |
| | 300 | 83 |
| 75 | 1 | 93 |
| | 5 | 74 |
| | 30 | 65 |
| | 60 | 64 |
| 95 | 1 | 81 |

### [Preparation Example 6] Preparation of aqueous solution containing ligand-bound PEG lipid and aqueous solution containing ligand-bound PEG lipid and ligand-unbound PEG lipid

In the same manner as in Preparation Example 2 except that the concentrations of the ligand PEG-bound lipid (ligand: anti-CD3 antibody) and the ligand-unbound PEG lipid (Methoxy-PEG) in the aqueous solution were changed as shown in Table 12, an aqueous solution containing the ligand-bound PEG lipid, and an aqueous solution containing the ligand-bound PEG lipid and the ligand-unbound PEG lipid (hereinafter sometimes referred to as "aqueous solution 6-1" to "aqueous solution 6-7")were prepared.

**[Table 12]**

| aqueous solution of Preparation Example 6 | concentration of ligand-bound PEG lipid (mM) | concentration of ligand-unbound PEG lipid (mM) |
|---|---|---|
| aqueous solution 6-1 | 0.008 | 0 |
| aqueous solution 6-2 | 0.004 | 0.004 |
| aqueous solution 6-3 | 0.002 | 0.006 |
| aqueous solution 6-4 | 0.016 | 0.0032 |
| aqueous solution 6-5 | 0.016 | 0.0064 |
| aqueous solution 6-6 | 0.016 | 0.0128 |
| aqueous solution 6-7 | 0.004 | 0.0076 |

### [Example 13]

In the same manner as in Preparation Example 2 except that
a 0.5 mM 1,1'-dioctadecyl-3,3,3',3'-tetramethylindocarbocyanine perchlorate (fluorescent dye) ethanol solution was added to the lipid solution of Example 1 to prepare a lipid solution containing a fluorescent dye in which the molar ratio of SS-OP/cholesterol/1,2-dioleoyl-sn-glycero-3-phosphocholine/DMG-PEG2000/1,1'-dioctadecyl-3,3,3',3'-tetramethylindocarbocyanine perchlorate was 52.5/40/7.5/1.5/0.5,
a lipid solution containing the aforementioned fluorescent dye was used instead of the lipid solution of Example 1, and
any of aqueous solution 6-1 to aqueous solution 6-7 obtained in Preparation Example 6 was used in Step c) instead of the aqueous solution containing the ligand-bound PEG lipid obtained in Preparation Example 1, mRNA-encapsulating and ligand-modified LNP suspensions (hereinafter sometimes referred to as "suspension 13-1" to "suspension 13-7") were prepared.

### [Experimental Example 11] Evaluation of LNP particle size, PdI, mRNA encapsulation rate, etc.

The particle size, PdI, and mRNA encapsulation rate of the ligand-modified LNP encapsulating mRNA of Example 13 were measured in the same manner as in Experimental Example 1. The results are shown in the following Table 13. In addition, the following Table 13 shows the mRNA-encapsulating and ligand-modified LNP suspensions (suspension 13-1 to suspension 13-7) in Example 13 and the aqueous solutions (aqueous solution 6-1 to aqueous solution 6-7) of Preparation Example 6 used. Also, the following Table 13 shows "amount of ligand (anti-CD3 antibody) with respect to total lipid" and "amount of ligand-unbound PEG lipid (Methoxy-PEG) used in Step c) with respect to total lipids " under the columns of "ligand amount" and "amount of ligand-unbound PEG lipid", respectively.

**[Table 13]**

| suspension of Example 13 | aqueous solution of Preparation Example 6 | ligand amount (mol%) | amount of ligand-unbound PEG lipid (mol%) | particle size (nm) | PdI | mRNA encapsulation rate (%) |
|---|---|---|---|---|---|---|
| suspension 13-1 | aqueous solution 6-1 | 0.4 | 0 | 153 | 0.11 | 94 |
| suspension 13-2 | aqueous solution 6-2 | 0.2 | 0.2 | 152 | 0.05 | 95 |
| suspension 13-3 | aqueous solution 6-3 | 0.1 | 0.3 | 149 | 0.11 | 96 |
| suspension 13-4 | aqueous solution 6-4 | 0.08 | 0.16 | 149 | 0.17 | 92 |
| suspension 13-5 | aqueous solution 6-5 | 0.08 | 0.32 | 150 | 0.12 | 91 |
| suspension 13-6 | aqueous solution 6-6 | 0.08 | 0.64 | 157 | 0.13 | 84 |
| suspension 13-7 | aqueous solution 6-7 | 0.02 | 0.38 | 148 | 0.15 | 98 |

### [Experimental Example 12] Evaluation of LNP gene expression activity in vivo

The mRNA-encapsulating and ligand-modified LNP suspension No. 13-1 to No. 13-3 obtained in Example 13, or mRNA-encapsulating but ligand-unmodified LNP suspension of Comparative Example 3 was intravenously administered to C57BL/6 mice from the tail vein (RNA amount: 1.0 µg).

After 21 hr from the administration, the mice were euthanized, and the spleen was removed. The obtained spleen was collected in a Petri dish containing 3 mL of RPMI-1640 with L-Gln liquid (manufactured by NACALAI TESQUE, INC.) (1 wt% fetal bovine serum (manufactured by SIGMA)). The spleen cells were strained using tweezers and suspended in spleen cell medium by using a 5 mL syringe. The obtained cell suspension was passed through a 70 µL cell strainer and the cells were collected in a 15 mL tube. The collected cells were centrifuged at 4°C and 500 g for 4 min, the supernatant was removed, and a cell pellet was obtained. The cell pellet was suspended in 1 mL of Red Blood Cell Lysing Buffer and incubated at room temperature for 5 min to remove red blood cells. The cell suspension after removal of red blood cells was diluted 5-fold with an FACS buffer (PBS containing 0.05 wt% sodium azide aqueous solution and 0.5 wt% fetal bovine serum albumin). The obtained diluted suspension was centrifuged, and the supernatant was removed. To the cell pellet was added another 5 mL of an FACS buffer, and the mixture was centrifuged and the supernatant was removed. The obtained cell pellet was suspended in 3 mL of an FACS buffer. The obtained cell suspension was diluted 10-fold with an FACS buffer and 18 µL of the diluted cell suspension was mixed with 2 µL of acridine orange. The obtained mixture was used for cell counting using LUNA-FL (manufactured by Logos Biosystems). An FACS buffer was added to the cell suspension to a cell concentration of 1×10⁶ cells/mL. The cell suspension was then centrifuged at 4°C and 500 g for 4 min, and the supernatant was removed to obtain a cell pellet. To prevent nonspecific staining, a blocking solution was prepared by mixing 2 µL of CD16/32 and 98 µL of an FACS buffer. 50 µL of the blocking solution was added to the cell pellet and tapped to obtain a cell suspension. The obtained cell suspension was left standing for 10 min at 4°C in shading. BV605-labeled anti-CD45 antibody, BV421-labeled anti-CD3ε antibody, BV510-labeled anti-CD19 antibody, PE-labeled anti-CD4 antibody, and AF488-labeled anti-CD8a antibody were diluted with the blocking solution to the concentrations recommended by the manufacturer to obtain an antibody cocktail for staining. 54 µL of the staining antibody cocktail was added to 50 µL of the cell suspension, and the obtained mixture was left standing at 4°C in shading for 30 min. Five minutes before observation, 5 µL of 7-AAD Viability Staining Solution (manufactured by BioLegend) was added to the mixture to perform dead cell staining. 1 mL of an FACS buffer was added to the cell suspension containing the antibody cocktail and 7-AAD, and the centrifugation and supernatant removal procedures were repeated twice. The obtained cell pellet was suspended in 500 µL of an FACS buffer, the obtained suspension (sample) was analyzed using Novocyte to calculate DiD-positive percentage (%) in T cells (=100 x number of T cells exhibiting DiD fluorescence/total number of T cells). Here, T cells exhibiting DiD fluorescence were T cells that had taken up LNP. That is, the percentage of T cells exhibiting DiD fluorescence (i.e., T cells that had taken up LNP) to all T cells was calculated, wherein T cells refer to a group of living cells stained with BV421-labeled anti-CD3ε antibody among the living cells in the cell suspension. The results are shown in the following Table 1 4.

**[Table 14]**

| | | DiD positive cell percentage in T cells (%) |
|---|---|---|
| suspension of Comparative Example 3 | | 0 |
| suspension of Example 13 | suspension 13-3 | 13 |
| | suspension 13-2 | 8 |
| | suspension 13-1 | 4 |

As shown in Table 14, the ligand-modified LNPs encapsulating mRNA of Example 13 showed an improved DiD-positive percentage in T cells (%) compared to the LNP encapsulating mRNA but not modified with ligand of Comparative Example 3.

### [Example 14] Preparation of ligand-modified LNP encapsulating mRNA

In the same manner as in Preparation Example 5 except that
a 1 mM 1,1'-dioctadecyl-3,3,3',3'-tetramethylindocarbocyanine perchlorate (fluorescent dye) was added to the lipid solution of Preparation Example 5 to prepare a lipid solution containing a fluorescent dye with a molar ratio of SS-OP/cholesterol/1,2-dioleoyl-sn-glycero-3-phosphocholine/DMG-PEG2000/1,1'-dioctadecyl-3,3,3',3'-tetramethylindocarbocyanine perchlorate of 52.5/40/7.5/1.5/0.5, and
the lipid solution containing the aforementioned fluorescent dye was used instead of the lipid solution of Preparation Example 5,
Step a) and the exchange of the dispersion medium were performed and an empty LNP suspension with the dispersion medium exchanged was prepared.

Using a nanoparticle production device "NanoAssemblr Ignite" (manufactured by Precision NanoSystems), the empty LNP suspension with the dispersion medium exchanged was mixed at a flow rate of 5 mL/min, and a solution of mRNA encoding luciferase was mixed at a flow rate of 5 mL/min at 25°C for 55 sec to obtain 9.2 mL of a suspension (9200 µL, RNA amount: 184 µg). This solution was divided by 4.6 mL, and each was either left unincubated or incubated at 37°C for 5 min to prepare mRNA-encapsulating LNP suspensions (N/P ratio: 36, mRNA concentration: 20 µg/mL) (Step b)).

Using the nanoparticles production device "NanoAssemblr Ignite" (manufactured by Precision NanoSystems), the obtained mRNA-encapsulating LNP suspension was mixed at a flow rate of 0.5 mL/min, and the aqueous solution containing the ligand-bound lipid PEG and the ligand-unbound PEG lipid obtained in Preparation Example 2 was mixed at a flow rate of 0.5 mL/min at 25°C for 60 sec, or the obtained mRNA-encapsulating LNP suspension was mixed at a flow rate of 5 mL/min, and the aqueous solution containing the ligand-bound lipid PEG and the ligand-unbound PEG lipid obtained in Preparation Example 2 was mixed at a flow rate of 5 mL/min at 25°C for 6 sec to obtain 1 mL of a suspension. This solution was divided by 0.5 mL, and each was not incubated, or incubated at 4°C for 30 min. Thereto was added a phosphate buffer (0.5 mL, buffer concentration: 9.6 mM, pH 7.4) to prepare an mRNA-encapsulating and ligand-modified LNP suspension (ligand amount with respect to total lipids: 0.1 mol%, 0.2 mol%, or 0.4 mol%) (Step c)).

### [Experimental Example 13] Evaluation of LNP particle size, PdI, mRNA encapsulation rate, etc.

The particle size, PdI, and mRNA encapsulation rate of the ligand-modified LNPs encapsulating mRNA of Example 14 were measured in the same manner as in Experimental Example 1. The results are shown in the following Table 15. In the following Table 15, the incubation treatment in Step b) and Step c), the flow rate of the mRNA-encapsulating LNP suspension in Step c), and the flow rate of the aqueous solution containing the ligand-bound lipid PEG and the ligand-unbound PEG lipid in Step c) are shown. The aforementioned two flow rates are collectively shown in the following Table 15, the column of "flow rate in Step c)". In addition, the "amount of ligand (anti-CD3 antibody) with respect to total lipids" is also shown in the following Table 15, the column of "ligand amount".

**[Table 15]**

| Step b) | Step c) | flow rate (mL/min) in Step c) | ligand amount (mol%) | particle size (nm) | mRNA encapsulation rate (%) |
|---|---|---|---|---|---|
| incubation 37°C, 5 min | incubation 4°C, 30 min | 0.5 | 0.1 | 145 | 96 |
| | | | 0.2 | 147 | 95 |
| | | | 0.4 | 153 | 95 |
| | | 5 | 0.1 | 144 | 96 |
| | | | 0.2 | 149 | 96 |
| | | | 0.4 | 154 | 95 |
| | no incubation | 0.5 | 0.1 | 150 | 96 |
| | | | 0.2 | 148 | 95 |
| | | | 0.4 | 151 | 96 |
| | | 5 | 0.1 | 146 | 96 |
| | | | 0.2 | 151 | 95 |
| | | | 0.4 | 151 | 95 |
| no incubation | incubation 4°C, 30 min | 0.5 | 0.1 | 145 | 97 |
| | | | 0.2 | 148 | 97 |
| | | | 0.4 | 150 | 96 |
| | | 5 | 0.1 | 145 | 97 |
| | | | 0.2 | 151 | 97 |
| | | | 0.4 | 156 | 96 |
| | no incubation | 0.5 | 0.1 | 145 | 97 |
| | | | 0.2 | 148 | 97 |
| | | | 0.4 | 152 | 97 |
| | | 5 | 0.1 | 151 | 97 |
| | | | 0.2 | 148 | 97 |
| | | | 0.4 | 152 | 95 |

### [Industrial Applicability]

Lipid nanoparticles encapsulating nucleic acids obtained by the production method of the present invention are useful for nucleic acid medicament, gene therapy, biochemical experiment, and the like.

This application is based on a patent application No. 2023-159114 filed in Japan, the contents of which are encompassed in full herein.

## Claims

1. A method for producing a ligand-modified lipid nanoparticle encapsulating a nucleic acid, comprising
Step a) of mixing an alcohol solution comprising an ionic lipid, a sterol, and a PEG lipid with an acidic buffer having a pH of 1 to 6.5 to obtain a suspension of a lipid nanoparticle free of nucleic acids; and
Step b) of mixing the lipid nanoparticle free of nucleic acids and a nucleic acid solution to obtain a suspension of a lipid nanoparticle encapsulating a nucleic acid and Step c) of mixing the suspension of the lipid nanoparticle encapsulating the nucleic acid and a ligand-bound lipid to obtain a suspension of a ligand-modified lipid nanoparticle encapsulating the nucleic acid, or
Step c') of mixing the lipid nanoparticle free of nucleic acids, water, and a ligand-bound lipid to obtain a suspension of a ligand-modified lipid nanoparticle free of nucleic acids and Step b') of mixing the ligand-modified lipid nanoparticle free of nucleic acids and a nucleic acid solution to obtain a suspension of a ligand-modified lipid nanoparticle encapsulating the nucleic acid, or
Step d) of mixing the lipid nanoparticle free of nucleic acids, a nucleic acid solution, and a ligand-bound lipid to obtain a suspension of a ligand-modified lipid nanoparticle encapsulating the nucleic acid.

2. The method according to claim 1, wherein, in Step b), a suspension of the lipid nanoparticle free of nucleic acids and a nucleic acid solution are mixed to obtain a suspension of a lipid nanoparticle encapsulating the nucleic acid, or
in Step c'), a suspension of the lipid nanoparticle free of nucleic acids and a ligand-bound lipid are mixed to obtain a suspension of a ligand-modified lipid nanoparticle free of nucleic acids and in Step b'), the suspension of the ligand-modified lipid nanoparticle free of nucleic acids and a nucleic acid solution are mixed to obtain a suspension of a ligand-modified lipid nanoparticle encapsulating a nucleic acid, or
in Step d), a suspension of the lipid nanoparticle free of nucleic acids, a nucleic acid solution, and a ligand-bound lipid are mixed to obtain a suspension of a ligand-modified lipid nanoparticle encapsulating the nucleic acid.

3. The method according to claim 2, wherein the method comprises Step a), Step b), and Step c), or Step a), Step c'), and Step b').

4. The method according to any one of claims 1 to 3, wherein a mixture of the lipid nanoparticle free of nucleic acids and the nucleic acid solution in Step b), or a mixture of the ligand-modified lipid nanoparticle free of nucleic acids and the nucleic acid solution in Step b') is maintained at 0 to 95°C for 1 min to 2 hr.

5. The method according to any one of claims 1 to 3, wherein a mixture of the ligand-bound lipid and the suspension of the lipid nanoparticle encapsulating the nucleic acid in Step c), or a mixture of the lipid nanoparticle free of nucleic acids, water, and the ligand-bound lipid in Step c') is maintained at 0 to 95°C for 1 min to 5 hr.

6. The method according to claim 1 or 2, wherein a mixture of the lipid nanoparticle free of nucleic acids, the nucleic acid solution, and the ligand-bound lipid in Step d) is maintained at 0 to 95°C for 1 min to 2 hr.

7. The method according to claim 1 or 2, wherein
in Step c), the suspension of the lipid nanoparticle encapsulating the nucleic acid, a ligand-bound lipid, and a ligand-unbound lipid are mixed to obtain a suspension of a ligand-modified lipid nanoparticle encapsulating the nucleic acid, or
in Step c'), the lipid nanoparticle free of nucleic acids, water, a ligand-bound lipid, and a ligand-unbound lipid are mixed to obtain a suspension of a ligand-modified lipid nanoparticle free of nucleic acids, or
in Step d), the lipid nanoparticle free of nucleic acids, a nucleic acid solution, a ligand-bound lipid, and a ligand-unbound lipid are mixed to obtain a suspension of a ligand-modified lipid nanoparticle encapsulating the nucleic acid.

8. The method according to any one of claims 1 to 3, wherein the alcohol solution in Step a) further comprises a phospholipid.

9. The method according to any one of claims 1 to 3, wherein the ionic lipid comprises at least one selected from the group consisting of a compound represented by the formula (1): 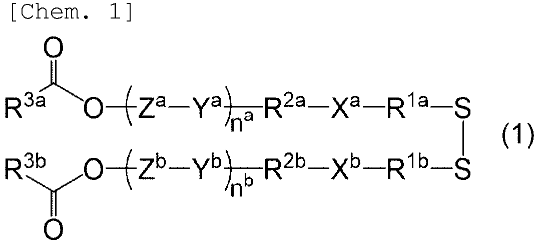 wherein, in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group, each having 8 or less carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1,
R^{3a} and R^{3b} are each independently
a residue derived from a reaction product of a liposoluble vitamin having a hydroxyl group, and succinic anhydride or glutaric anhydride,
a residue derived from a reaction product of a sterol derivative having a hydroxyl group, and succinic anhydride or glutaric anhydride,
an aliphatic hydrocarbon group having 1 to 40 carbon atoms,
an alkyl group having a cyclopropane ring and having 3 to 40 carbon atoms,
a group represented by the formula (3):
R⁹-O-CO-(CH₂)ₐ-* (3)
wherein, in the formula (3),
* is a bonding position,
R⁹ is an aliphatic hydrocarbon group having 2 to 20 carbon atoms,
a is an integer of 2 to 10,
a group having 50 or less carbon atoms and represented by the formula (4): 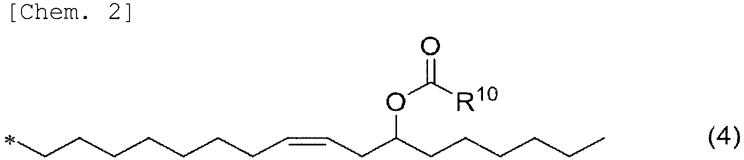 wherein, in the formula (4),
* is a bonding position,
R¹⁰ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and R¹⁰ is optionally substituted by a substituent selected from the group consisting of a 3- to 14-membered heterocyclic group and a hydrocarbon ring group having 3 to 12 carbon atoms, or
a group having 50 or less carbon atoms and represented by the following formula (5): 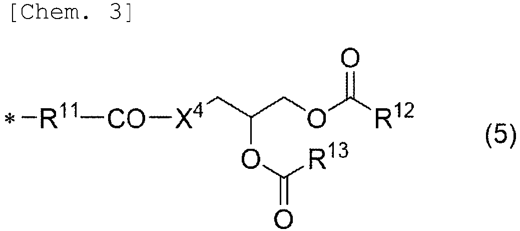 wherein, in the formula (5),
* is a bonding position,
R¹¹ is an alkylene group having 2 to 9 carbon atoms, an alkenediyl group having 2 to 9 carbon atoms, or an alkynediyl group having 2 to 9 carbon atoms, and R¹¹ is optionally substituted by a substituent selected from the group consisting of a halogen atom, a hydroxy group, and a hydrocarbon ring group having 3 to 12 carbon atoms, and
R¹² and R¹³ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in R¹² is optionally replaced by at least one bond selected from the group consisting of an ester bond, an amide bond, a carbamate bond, and a carbonate bond, at least one ethylene group or at least one trimethylene group in R¹³ is optionally replaced by at least one bond selected from the group consisting of an ester bond, an amide bond, a carbamate bond, and a carbonate bond, and R¹² and R¹³ are each independently optionally substituted by a substituent selected from the group consisting of a halogen atom, a hydroxy group, and a hydrocarbon ring group having 3 to 12 carbon atoms, and
X⁴ is an oxygen atom, NH, or a sulfur atom, [(4-hydroxybutyl)azanediyl]bis(hexane-6,1-diyl)bis(2-hexyldecanoate), heptadecan-9-yl 8-((2-hydroxyethyl)(6-oxo-6-(undecyloxy)hexyl)amino)octanoate, and (6Z,9Z,28Z,31Z)-heptatriaconta-6,9,28,31-tetraen-19-yl 4-(dimethylamino)butanoate.

10. The method according to claim 9, wherein the ionic lipid comprises a compound represented by the formula (1).

11. The method according to any one of claims 1 to 3, wherein the ligand in the ligand-bound lipid is a ligand having brain cell directivity or a ligand having T cell directivity.

12. A method for introducing a nucleic acid into a cell, comprising a step of contacting the ligand-modified lipid nanoparticle encapsulating the nucleic acid obtained by the method according to any one of claims 1 to 3 and the cell in vitro.

13. A method for introducing a nucleic acid into a target cell in a living body, comprising a step of administering the ligand-modified lipid nanoparticle encapsulating the nucleic acid obtained by the method according to any one of claims 1 to 3 to the living body.

14. A method for producing a pharmaceutical composition, comprising the method according to any one of claims 1 to 3.
